(19) Europäisches Patentamt / European Patent Office / Office européen des brevets

(11) **EP 4 299 594 A1**

(12) **EUROPEAN PATENT APPLICATION**
published in accordance with Art. 153(4) EPC


(43) Date of publication:
**03.01.2024 Bulletin 2024/01**

(21) Application number: **22784988.2**

(22) Date of filing: **07.04.2022**

(51) International Patent Classification (IPC):
***C07K 16/40*** (2006.01) ***C12N 15/85*** (2006.01)
***C12N 15/62*** (2006.01) ***G01N 33/68*** (2006.01)
***G01N 33/574*** (2006.01) ***A61P 35/00*** (2006.01)
***A61K 38/00*** (2006.01)

(52) Cooperative Patent Classification (CPC):
**A61K 38/00; A61K 39/00; A61P 35/00; C07K 14/705; C07K 16/40; C12N 5/06; C12N 15/62; C12N 15/85; G01N 33/574; G01N 33/68**

(86) International application number:
**PCT/KR2022/005030**

(87) International publication number:
**WO 2022/216079 (13.10.2022 Gazette 2022/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.04.2021 KR 20210045510**



(71) Applicant: **Lg Chem, Ltd.**
**Seoul 07336 (KR)**

(72) Inventors:
- **KIM, Youngkyun**
  **Daejeon 34122 (KR)**
- **SHIN, Jung Youn**
  **Daejeon 34122 (KR)**
- **KO, Yeongrim**
  **Daejeon 34122 (KR)**
- **YANG, Soyeon**
  **Daejeon 34122 (KR)**
- **HONG, Beom Ju**
  **Daejeon 34122 (KR)**
- **CHOI, Eunhye**
  **Daejeon 34122 (KR)**
- **LEE, Nakyoung**
  **Daejeon 34122 (KR)**

(74) Representative: **Hoffmann Eitle**
**Patent- und Rechtsanwälte PartmbB**
**Arabellastraße 30**
**81925 München (DE)**


Remarks:
The complete document including Reference Table(s) and the Sequence Listing(s) can be downloaded from the EPO website

# (54) GUCY2C BINDING POLYPEPTIDE AND USES THEREOF


(57) The present disclosure pertains to a GUCY2C-binding polypeptide and uses thereof and, specifically, to a GUCY2C-binding polypeptide, a fusion protein including same, a chimeric antigen receptor, an immune cell expressing the chimeric antigen receptor, and a use thereof for treatment and/or diagnosis of cancer.


【FIG. 6b】

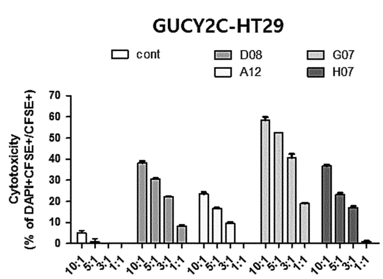


EP 4 299 594 A1

## Description

[TECHNICAL FIELD]

Cross-reference to related applications

The present application claims the benefit of priority based on Korean Patent Application No. 10-2021-0045510 filed on April 7, 2021, and all the contents disclosed in the document of the corresponding Korean patent application are incorporated as a part in the present description.

The present application relates to a GUVY2C binding polypeptide and uses thereof, and specifically, relates to a GUCY2C binding polypeptide, a fusion protein comprising the same, a chimeric antigen receptor, an immunocyte expressing the chimeric antigen receptor, and a use of cancer treatment and/or diagnosis thereof.

[BACKGROUND ART]

GUCY2C (Guanylate cyclase 2C); guanylyl cyclase C; GC-C or GCC) is a transmembrane cell surface receptor which acts on intestinal fluid, electrolyte homeostasis and maintenance of cell proliferation, and the like. In normal adult mammals, GUCY2C is expressed in mucosal cells covering the inside wall of small intestine, large intestine and rectum. These cells undergo cycles of proliferation, migration, differentiation and apoptosis, and imbalance between proliferation and apoptosis may induce formation of tumors in the gastrointestinal tract.

[DISCLOSURE]

[TECHNICAL PROBLEM]

Accordingly, in the present description, a novel GUCY2C binding polypeptide, a fusion protein comprising the same, and uses of cancer treatment and/or diagnosis thereof are provided.

One embodiment provides a GUCY2C binding polypeptide which binds to GUCY2C.

The GUCY2C binding polypeptide may comprise

a heavy chain variable region comprising heavy chain CDR1 (hereinafter, CDR-H1) represented by the amino acid sequence selected from the group consisting of SEQ ID NOs: 38 to 44, CDR-H2 represented by the amino acid sequence selected from the group consisting of SEQ ID NOs: 45 to 53, and CDR-H3 represented by the amino acid sequence selected from the group consisting of SEQ ID NOs: 54 to 65;
a light chain variable region comprising light chain CDR1 (hereinafter, CDR-L1) represented by the amino acid sequence selected from the group consisting of SEQ ID NOs: 66 to 78, CDR-L2 represented by the amino acid sequence selected from the group consisting of SEQ ID NOs: 79 to 88, and CDR-L3 represented by the amino acid sequence selected from the group consisting of SEQ ID NOs: 89 to 106;
or a combination thereof.

In one embodiment, the GUCY2C binding polypeptide may be a single chain variable fragment (scFv) comprising the heavy chain variable region and light chain variable region regardless of the order. The scFv may comprise the heavy chain variable region and light chain variable region in order of the heavy chain variable region and light chain variable region or order of the light chain variable region and heavy chain variable region, in a direction from the N-terminus to C-terminus, and for example, it may comprise them in order of the heavy chain variable region and light chain variable region in a direction from the N-terminus to C-terminus. Then, the heavy chain variable region and light chain variable region may be linked through a peptide linker or directly linked without a linker. In one specific embodiment, the polypeptide may be a scFv represented by the amino acid sequence selected from the group consisting of SEQ ID NOs: 1 to 18.

Another embodiment provides an antibody specifically binding to GUCY2C or an antigen binding fragment thereof, comprising the heavy chain variable region and light chain variable region.

Another embodiment provides a fusion protein comprising the GUCY2C binding polypeptide and a Fc domain of an immunoglobulin.

Another embodiment provides a conjugate comprising the GUCY2C binding polypeptide, antibody or antigen binding fragment thereof or fusion protein, and a drug. The drug may be one or more kinds selected from anticancer agents, contrast media, and the like.

Another embodiment provides a chimeric antigen receptor comprising the GUCY2C binding polypeptide. The chimeric antigen receptor may be specific to GUCY2C.

Another embodiment provides an immunocyte comprising the chimeric antigen receptor. The immunocyte may be an immunocyte expressing the chimeric antigen receptor on a cell surface. The immunocyte may be a GUCY2C specific immunocyte.

Another embodiment provides a polynucleotide encoding the GUCY2C binding polypeptide, an antibody binding specifically to GUCY2C or an antigen binding fragment thereof, a fusion protein, or a chimeric antigen receptor. Another embodiment provides a recombinant vector comprising the polynucleotide. Another embodiment provides a recombinant cell comprising the polynucleotide or recombinant vector.

Another embodiment provides a pharmaceutical composition for prevention and/or treatment of cancer comprising one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor as an active ingredient.

Another embodiment provides a method for preventing and/or treating cancer, comprising administering a pharmaceutically effective dose of one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor into a subject in need of prevention and/or treatment of cancer.

Another embodiment provides a use for using in prevention and/or treatment of cancer or a use for using in preparation of a pharmaceutical composition for prevention and/or treatment of cancer, of one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor.

Another embodiment provides a composition for diagnosis of cancer comprising one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor as an active ingredient.

Another embodiment provides a method for diagnosis of cancer or a method for providing information for diagnosis of cancer, comprising contacting one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor into a biological sample obtained from a subject. The subject may be a patient in need of diagnosis of cancer, and the biological sample may be one or more kinds selected from the group consisting of a cell, tissue, body fluid and culture thereof.

Another embodiment provides a use for using in diagnosis of cancer or a use for using in preparation of a composition for diagnosis of cancer, of one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor.

Another embodiment provides a composition for detecting GUCY2C comprising one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor as an active ingredient.

Another embodiment provides a method for detecting GUCY2C, comprising contacting one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor to a biological sample. The biological sample may be one or more kinds selected from the group consisting of a separated cell, tissue, body fluid and culture thereof.

Another embodiment provides a use for using in detection of GUCY2C of one or more kinds selected from the group consisting of the GUCY2C binding polypeptide, an antibody specifically binding to GUCY2C or an antigen binding fragment thereof, a fusion protein, a conjugate, a chimeric antigen receptor, a polynucleotide encoding them, a recombinant vector comprising the polynucleotide, a recombinant cell comprising the recombinant vector, and an immunocyte expressing the chimeric antigen receptor.

[TECHNICAL SOLUTION]

Definition of terms

In the present description, GUCY2C (Guanylate cyclase 2C; guanylyl cyclase C; GC-C or GCC), intestinal guanylate cyclase, guanylate cyclase-C receptor, or the heat-stable enterotoxin receptor (hSTAR) is a transmembrane cell surface receptor acting on intestinal fluid, electrolyte homeostasis, maintenance of cell proliferation, and the like. In one embodiment, GUCY2C may be GUCY2C derived from a mammal, and for example, it may be human GUCY2C (protein: GenBank Accession No. NP_004954.2, etc.; gene: GenBank Accession No. NM_004963.4, etc.), mouse GUCY2C (protein: GenBank Accession No. NP_001120790.1, NP_659504.2, etc.; gene: GenBank Accession No. NM_001127318.1, NM_145067.3, etc.), but not limited thereto. In one embodiment, GUCY2C may be expressed specifically in colorectal cancer of mammals (for example, primates such as humans, monkeys, and the like, rodents such as mice, rats, and the like, etc.), but it may be expressed even in the mesentery and the like such as large intestine and esophagus, stomach, pancreas and the like, but no special limitation is applied.

In the present description, that a polynucleotide (can be used interchangeably with "gene") or polypeptide (can be used interchangeably with "protein") "comprises a specific nucleic acid sequence or amino acid sequence" or "consists of or is represented by a specific nucleic acid sequence or amino acid sequence" may mean that the polynucleotide or polypeptide essentially comprises the specific nucleic acid sequence or amino acid sequence, and it may be interpreted as including "substantially equivalent sequences" in which a mutation (deletion, substitution, modification and/or addition) is added to the specific nucleic acid sequence or amino acid sequence within a range of maintaining the original function and/or desired function of the polynucleotide or polypeptide (or not excluding the mutation).

In one embodiment, that a polynucleotide or polypeptide "comprises a specific nucleic acid sequence or amino acid sequence" or "consists of or is represented by a specific nucleic acid sequence or amino acid sequence" may mean that the polynucleotide or polypeptide (i) may essentially comprise the specific nucleic acid sequence or amino acid sequence, or (ii) may consist of a nucleic acid sequence or amino acid sequence having identity of 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more to the specific nucleic acid sequence or amino acid sequence or essentially comprise this and maintain the original function and/or desired function. In the present description, that a polypeptide, an antibody or antigen binding fragment thereof (for example, CDR, variable region or heavy chain/light chain), a fusion protein and a chimeric antigen receptor "comprise a specific amino acid sequence or is represented by or consist of a specific amino acid sequence" may mean all of the case of essentially comprising the amino acid sequence, and the case of introducing an insignificant mutation which does not affect the original activity and/or desired activity (for example, GUCY2C binding activity, etc.) into the amino acid sequence (for example, substitution, deletion and/or addition of an amino acid residue).

In the present description, the term "identity" means a degree of correspondence with a given nucleic acid sequence or amino acid sequence and may be represented by a percentage (%). The identity to a nucleic acid sequence may be determined by using algorithm BLAST by a document (See: Karlin and Altschul, Pro. Natl. Acad. Sci. USA, 90, 5873, 1993) or FAST by Pearson (See: Methods Enzymol., 183, 63, 1990). Based on this algorithm BLAST, programs called BLASTN or BLASTX have been developed (See: http://www.ncbi.nlm.nih.gov).

Herein, the term "antibody" is used in the broadest sense as a generic term for proteins that specifically bind to a specific antigen, and may be a protein made by stimulation of an antigen in the immune system or a protein produced by chemical synthesis or recombinantly, and the type thereof is not particularly limited. Specifically, a monoclonal antibody (including a full-length monoclonal antibody), a polyclonal antibody, a multispecific antibody (e.g., bispecific antibody), a synthetic antibody (or also referred to as an antibody mimic), a chimeric antibody, a humanized antibody, a human antibody or an antibody fusion protein (or also referred to as an antibody conjugate) are encompassed, as long as exhibiting the desired biological activity.

A complete antibody (for example, IgG type) has a structure having two full length light chains and 2 full length heavy chains, and each light chain is linked to the heavy chain by a disulfide bond. The constant region of the antibody is divided into a heavy chain constant region and a light chain constant region, and the heavy chain constant region has a gamma ($\gamma$), mu ($\mu$), alpha ($\alpha$), delta ($\delta$) or epsilon ($\varepsilon$) type, and has gamma 1 ($\gamma$1), gamma 2 ($\gamma$2), gamma 3 ($\gamma$3), gamma 4 ($\gamma$4), alpha 1 ($\alpha$1) or alpha 2 ($\alpha$2) as a subclass. The constant region of the light chain has kappa ($\kappa$) and lambda ($\lambda$) types.

The term "antigen binding fragment" refers to a portion of an antibody that lacks at least some of amino acids present in its full length chain but is still capable of specifically binding to an antigen. Such fragment is biologically active in that it binds to a target antigen and is able to compete with other antigen binding molecules, including intact antibodies, for binding to a given epitope. The antigen binding fragment may not comprise a constant heavy chain domain of a Fc region of an intact antibody (i.e., depending on the antibody isotype, that is, CH2, CH3 and CH4). The example of the antigen binding fragment includes a scFv (single chain variable fragment) (for example, scFv, $(scFv)_2$, etc.), Fab (fragment antigen binding) (for example, Fab, Fab', $F(ab')_2$, etc.), a domain antibody, a peptibody, a minibody, an intrabody, a diabody, a triabody or a single-chain antibody, and the like, but not limited thereto. In addition, the antigen binding fragment may be a scFv, a fusion polypeptide in which a scFv is fused with a Fc region of an immunoglobulin (for example, IgA, IgD, IgE, IgG (IgG1, IgG2, IgG3, IgG4), IgM, etc.) (scFv-Fc) or a fusion polypeptide in which it is fused with a constant region of a light chain (for example, kappa or lambda) (scFv-Cκ (kappa constant region) or scFv-Cλ (lambda constant region)), but not limited thereto.

The term, "heavy chain" is interpreted to include all of a full length heavy chain comprising a variable domain $V_H$ comprising an amino acid sequence having a variable region sufficient for giving specificity to an antigen and 3 constant region domains $C_{H1}$, $C_{H2}$ and $C_{H3}$ and a hinge and fragments thereof. In addition, the term "light chain" is interpreted as a meaning of including all of a full length light chain comprising a variable region domain $V_L$ comprising an amino acid sequence having a variable region sequence sufficient for giving specificity to an antigen and a constant region domain $C_L$ and fragments thereof.

The term "complementarity-determining regions (CDR)" refers to a region giving binding specificity or binding affinity to an antigen among variable regions of an antibody. In general, 3 CDRs (CDR-H1, CDR-H2, CDR-H3) are present in the heavy chain variable region and 3 CDRs (CDR-L1, CDR-L2, CDR-L3) are present in the light chain variable region. The CDR may provide a key contact residue for binding an antibody or fragment thereof to an antigen or epitope. "Framework region (FR)" refers to a non-CDR part of variable regions of a heavy chain and a light chain, and generally, 4 FRs (FR-H1, FR-H2, FR-H3 and FR-H4) are present in the heavy chain variable region and 4 FRs (FR-L1, FR-L2, FR-L3 and FR-L4) are present in the light chain variable region. The exact amino acid sequence boundary of the given CDR or FR may be easily determined by using any one of a number of well-known systems such as Kabat numbering system, Chothia numbering system, Contact numbering system, IMGT numbering system, Abo numbering system, AbM numbering system and the like.

The term "variable region" refers to a domain of a heavy chain or light chain of an antibody which is involved in binding an antibody to an antigen. The heavy chain variable (VH) region and light chain variable (VL) region generally have a similar structure, and each domain includes 4 conserved framework regions (FR) and 3 CDRs.

<u>GUCY2C binding polypeptide, antibody, or antigen binding fragment thereof, fusion protein</u>

The GUCY2C binding polypeptide, antibody or antigen binding fragment thereof provided in the present description may comprise the following:

a heavy chain variable region comprising heavy chain CDR1 represented by an amino acid sequence selected from the group consisting of SEQ ID NOs: 38 to 44 (or less, CDR-H1), CDR-H2 represented by an amino acid sequence selected from the group consisting of SEQ ID NOs: 45 to 53, and CDR-H3 represented by an amino acid sequence selected from the group consisting of SEQ ID NOs: 54 to 65;
a light chain variable region comprising light chain CDR1 represented by an amino acid sequence selected from the group consisting of SEQ ID NOs: 66 to 78 (or less, CDR-L1), CDR-L2 represented by an amino acid sequence selected from the group consisting of SEQ ID NOs: 79 to 88 and CDR-L3 represented by an amino acid sequence selected from the group consisting of SEQ ID NOs: 89 to 106;
or a combination of the heavy chain variable region and light chain variable region.

The amino acid sequence and combination of each CDR comprised in the GUCY2C binding polypeptide, antibody or antigen binding fragment thereof provided in the present description are exemplified in Table 1 and Table 2 below: Table 1 shows the amino acid sequence of each CDR of the heavy chain variable region and Table 2 shows that of the light chain variable region.

[Table 1]

| clone ID | CDR-H1 | SEQ ID NO | CDR-H2 | SEQ ID NO | CDR-H3 | SEQ ID NO |
|---|---|---|---|---|---|---|
| A01 | GYTFTSYY | 38 | INPSGGST | 46 | DGQWLQFDY | 54 |
| A02 | GGTLSSYA | 39 | IIPILGIT | 47 | DQRPASMDV | 55 |
| A03 | GGTFSSYT | 40 | IIPILGIA | 48 | DYSSSWNSMDV | 56 |
| A04 | GGTLSSYA | 39 | IIPILGIT | 47 | DQRPASMDV | 55 |
| A05 | GGTFSSYT | 40 | IIPVLGIA | 49 | DYSSSWNSMDV | 56 |
| A06 | GGTFGSYT | 41 | IIPILGIA | 48 | DYSSSWNSMDV | 56 |
| A07 | GGSISSYY | 42 | IYYSGST | 50 | DVWGSGQSFDS | 57 |

(continued)

| clone ID | CDR-H1 | SEQ ID NO | CDR-H2 | SEQ ID NO | CDR-H3 | SEQ ID NO |
|---|---|---|---|---|---|---|
| A08 | GFTFSSYW | 43 | IKQDGSEK | 51 | APWYSSSPTPYGMDV | 58 |
| A10 | GGTFSSYA | 44 | IIPIFGTA | 52 | TRYIWGSYRAYGMDV | 59 |
| A12 | GGTLSSYA | 39 | IIPILGIT | 47 | DQRPASMDV | 55 |
| B01 | GGTLSSYA | 39 | IIPILGIT | 47 | DQRPASMDV | 55 |
| B07 | GYTFTSYY | 38 | INPSGGST | 46 | GTYSSGWTIDY | 60 |
| B08 | GGTFSSYA | 44 | IIPIFGTA | 52 | GHYYYMDV | 61 |
| B10 | GGTFSSYA | 44 | IIPIFGTA | 52 | GHYYYMDV | 61 |
| B11 | GGTFSSYA | 44 | IIPIFGTA | 52 | GIQPLRYYGMDV | 62 |
| B12 | GFTFSSYS | 45 | IYSGGST | 53 | GAGTLNAFDI | 63 |
| C01 | GGTFSSYA | 44 | IIPIFGTA | 52 | GYSSIYYYYGMDV | 64 |
| C02 | GGTFSSYT | 40 | IIPILGIA | 48 | DRSYNWLDP | 65 |
| C07 | GGSFSGYY | 107 | INHRGNT | 108 | ERGYTYGNFDH | 109 |

[Table 2]

| clone ID | CDR-L1 | SEQ ID NO | CDR-L2 | SEQ ID NO | CDR-L3 | SEQ ID NO |
|---|---|---|---|---|---|---|
| A01 | QSLLKKSDGNTY | 66 | KVS | 79 | MQGSHWPPT | 89 |
| A02 | SSDVGGYIY | 67 | DVS | 80 | SSYAGSNNYV | 90 |
| A03 | SSDIGYYHY | 68 | EDS | 81 | SSFTSRSTWV | 91 |
| A04 | SSDVGGYIY | 67 | DVS | 80 | SSYTSSNNYY | 92 |
| A05 | SSDVGGYNY | 69 | DVS | 80 | SSYAGSNNFV | 93 |
| A06 | SSDVGAYNY | 70 | EVS | 82 | SSYAGSNNWV | 94 |
| A07 | SGSIASNY | 71 | EHS | 83 | QSYDVSNRV | 95 |
| A08 | QDISNY | 72 | GAS | 84 | QQSYSTPLT | 96 |
| A10 | QSISSH | 73 | YAS | 85 | QQSISLPYT | 97 |
| A12 | SSDVGGYIY | 67 | DVS | 80 | SSYTSSNNYV | 98 |
| B01 | SSDVGGYNY | 69 | EVS | 82 | STVTSLSTYV | 99 |
| B07 | QSLVYTDGNTY | 74 | KVS | 79 | MHSKQWPPT | 100 |
| B08 | QSVSSN | 75 | GAS | 84 | QQYNNWPS | 101 |
| B10 | QSVSSN | 75 | GAS | 84 | QQYNNWPT | 102 |
| B11 | SSNIGSNY | 76 | RNN | 86 | AAWDDSLSGRGV | 103 |
| B12 | SSDVGAYSY | 77 | AVT | 87 | SSFAGGSTLV | 104 |
| C01 | SSDVGGYNY | 69 | DVS | 80 | GSYTSDGTLV | 105 |
| C02 | ISDVGDYNY | 78 | DVN | 88 | SSYTSSSTLV | 106 |
| C07 | QSVSRN | 110 | GAS | 84 | QQYKTWPRT | 111 |

In one embodiment, the GUCY2C binding polypeptide may comprise a heavy chain variable region comprising the aforementioned heavy chain CDR and a light chain variable region comprising the light chain CDR. The heavy chain variable region may comprise a framework of the heavy chain CDR and an immunoglobulin (for example, IgA, IgD, IgE, IgG (IgG1, IgG2, IgG3, IgG4), IgM, etc.) described above (e.g., structure of FR1-(CDR-H1)-FR2-(CDR-H2)-FR3-(CDR-H3)-FR4). The light chain variable region may comprise a framework of the light chain CDR lambda or kappa subtype described above (e.g., structure of FR1-(CDR-L1)-FR2-(CDR-L2)-FR3-(CDR-L3)-FR4).

In one specific embodiment, the GUCY2C binding polypeptide may be a scFv (single chain variable fragment) that is a single chain polypeptide in which the aforementioned heavy chain variable region and light chain variable region are linked through or not through a peptide linker.

The peptide linker may be a polypeptide consisting of any amino acids of 1 to 100 or 2 to 50, and the type of the amino acid comprised is not limited. For example, the peptide linker may comprise Gly, Asn and/or Ser residues, and may comprise neutral amino acids such as Thr and/or Ala. The amino acid sequence suitable for a peptide linker is known in the art. On the other hand, for the linker, its length may be variously determined, within the limit that does not affect the GUCY2C binding function of the scFv. For example, the peptide linker may be composed of comprising a total of 1 to 200, 2 to 50, or 5 to 25 of one or more kinds selected from the group consisting of Gly, Asn, Ser, Thr and Ala. In one embodiment, the peptide linker is (G4S)n (n is a repeated number of (G4S)), and may be represented by an integer of 1 to 10, for example, an integer of 2 to 5.

In one specific embodiment, the GUCY2C binding polypeptide in the scFv form may be represented by the amino acid sequence selected from SEQ ID NOs: 1 to 18, and these amino acid sequences are illustrated in Table 3 below:

[Table 3]

| scF v ID | scFv Region (Protein; N→C) | SEQ ID NO |
|---|---|---|
| A01 | QVQLVQSGAEVKKPGASVKVSCKAS**GYTFTSYY**MHWVRQAPGQGLEWMGI**INPSGGST**SYAQEFQGRVTMTRDTSTSTVYMELSSLRSEDTAVYYCAR**DGQWLQFDY**WGQGTLVTVSSGGGGSGGGGSGGGASDIVMTQSPLSLPVTLGQPASISCRSS**QSLLKKSDGNTY**LSWYHQRPGQSPRRLIY**KVS**NRDSGVPDRFSGSGSDTDFTLKISRVETEDVGIYYC**MQGSHWPPT**FGQGTKVEIK | 1 |
| A02 | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSELTQPASVSGSPGQSITISCTGT**SSDVGGYIY**VSWYQQHPGKVPKLMIH**DVS**HRPSGVSNRFSGSRSGNTASLTISGLQAEDEADYFC**SSYAGSNNYV**FGTGTKVTVL | 2 |
| A03 | QVQLVQSGAEVKKPGSSVKVSCKAS**GGTFSSYT**ISWVRQAPGQELEWMGR**IIPILGIA**NYAQKFQGRVTITADKSTSTAYMELSSLRSEDTAVYYCAR**DYSSSWNSMDV**WGQGTLVTVSSGGGGSGGGGSGGGASQSGLTQPPSASGSPGQSVTISCTGT**SSDIGYYHY**VSWYQQHPGKAPKLMIY**EDS**KRPSGISNRFSGSKSGTTASLTVSGLQAEDEAHYYC**SSFTSRSTWV**FGGGTQLTVL | 3 |
| A04 | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSELTQPASVSGSPGQSITISCTGT**SSDVGGYIY**VSWYQQHPGKVPKLMIH**DVS**HRPSGVSNRFSGSRSGNTASLTISGLQAEDEADYFC**SSYTSSNNYY**FGTGTKVTVL | 4 |

(continued)

| scF v ID | scFv Region (Protein; N→C) | SEQ ID NO |
|---|---|---|
| A05 | QVQLVQSGAEVKKPGSSVKVSCKAS**GGTFSSYT**ITWVRQAPGQGLEWMGR**IIPVLGIA**NYAQKFQGRVTITADKSTSTAYMELSSLRSEDTAVYYCAR**DYSSSWNSMDV**WGQGTLVTVSSGGGGSGGGGSGGGASQSGLTQPRSVSGSPGQSVTISCTGT**SSDVGGYNY**VSWYQQHPGKAPKLMIY**DVS**KRPSGVPDRFSGSKSGNTASLTVSGLHAEDEADYYC**SSYAGSNNFV**FGTGTKVTVL | 5 |
| A06 | QVQLVQSGAEVKKPGSSVKVSCKAS**GGTFGSYT**ISWVRQAPGQGLEWMGR**IIPILGIA**NYAQKFQGRVTITADKSTSTAYMELSSLRSEDTAVYYCAR**DYSSSWNSMDV**WGQGTLVTVSSGGGGSGGGGSGGGASQSGLTQPRSVSGSPGQSVTISCTGT**SSDVGAYNY**VSWYQQHPGKAPKLMIY**EVS**KRPSGVPDRFSASKSGNTASLTVSGLQAEDEADYYC**SSYAGSNNWV**FGGGTKLTVL | 6 |
| A07 | QVQLQESGPGLVKPSETLSLTCTVS**GGSISSYY**WSWIRQPPGKGLEWIGS**IYYSGST**NYNPSLKSRVTISRDKSKNQLFLKLNSMTAADTAVYYCAR**DVWGSGQSFDS**WGQGTLVTVSSGGGGSGGGGSGGGASNFMLTQPHSVSESPGKTVTISCTRS**SGSIASNY**VQWYQQRLGSSPTTVIY**EHS**RRPSGVPDRFSASIDSSSNSASLTISGLKTEDEADYYC**QSYDVSNRV**FGGGTKLTVL | 7 |
| A08 | EVQLLESGGGLVQPGGSLRLSCAAS**GFTFSSYW**MSWVRQAPGKGLEWVAN**IKQDGSEK**YYVDSVKGRFTISRDNAKNSLYLQMNSLRAEDTAVYYCAK**APWYSSSPTPYGMDV**WGQGTLVTVSSGGGGSGGGGSGGGASDIQMTQSPSSLSASVGDRVTITCQAS**QDISNY**LNWYQQKPGKAPRRLIY**GAS**TLMSGVPSRFSGSGSGTDFTLTISSLQPEDFATYYC**QQSYSTPLT**FGGGTKVEIK | 8 |
| A10 | EVQLVQSGAEVKKPGSSVKVSCKAS**GGTFSSYA**ISWVRQAPGQGLEWMGG**IIPIFGTA**NYAQKFQGRVTITADESTSTAYMELSSLRSEDTAVYYCAR**TRYIWGSYRAYGMDV**WGQGTMVTVSSGGGGSGGGGSGGGASDIQMTQSPSSMSASVGDRVTITCRAS**QSISSH**LNWYQQLPGNAPTLLIY**YAS**NLQSGVPSRFSGSGSGTDFTLTISSLQPDDFATYYC**QQSISLPYT**FGQGTKVEIK | 9 |

(continued)

| scF v ID | scFv Region (Protein; N→C) | SEQ ID NO |
|---|---|---|
| A12 | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSELTQPASVSGSPGQSITISCTGT**SSDVGGYIY**VSWYQQHPGKVPKLMIH**DVS**HRPSGVSNRFSGSRSGNTASLTISGLQAEDEADYFC**SSYTSSNNYV**FGTGTKVTVL | 10 |
| B01 | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSGLTQPASVSGSPGQSITISCTGT**SSDVGGYNY**VSWYQQHPGKAPKLMIY**EVS**NRPSGVSNRFSGSKSGNTASLTISGLQAEDEADYYC**STVTSLSTYV**FGTGTKLTVL | 11 |
| B07 | EVQLVQSGAEVKRPGSSVKVSCKAS**GYTFTSYY**MHWVRQAPGQGLEWMGI**INPSGGST**SYAQKFQGRVTMTRDTSTSTVYMELSSLRSEDTAVYYCAA**GTYSSGWTIDY**WGQGTTVTVSSGGGGSGGGGSGGGASDIVMTQSPLSLPVTLGQPASISCRSS**QSLVYTDGNTY**LNWFQQRPGQSPRRLIY**KVS**NRDSGVPDRFSGSGSGTDFTLKISRVEAEDVGIYYC**MHSKQWPPT**FGGGTKVEIK | 12 |
| B08 | EVQLVQSGAEVKKPGSSVKVSCKAS**GGTFSSYA**ISWVRQAPGQGLEWMGG**IIPIFGTA**NYAQKFQGRVTITADKSTSTAYMELSSLRSEDTAVYYCAR**GHYYYMDV**WGQGTTVTVSSGGGGSGGGGSGGGASDIVMTQSPATLSVSPGEGATLSCRAS**QSVSSN**LAWYQQKPGRAPRLLIY**GAS**TRATGIPARFSGSGSGTEFTLTISSLQSEDFAVYYC**QQYNNWPS**FGGGTKLEIK | 13 |
| B10 | EVQLVQSGAEVKKPGSSVKVSCKAS**GGTFSSYA**ISWVRQAPGQGLEWMGG**IIPIFGTA**NYAQKFQGRVTITADKSTSTAYMELSSLRSEDTAVYYCAR**GHYYYMDV**WGQGTTVTVSSGGGGSGGGGSGGGASDIVMTQSPATLSVSPGEGATLSCRAS**QSVSSN**LAWYQQKPGRAPRLLIY**GAS**TRATGIPARFSGSGSGTEFTLTISSLQSEDFAVYYC**QQYNNWPT**FGGGTKLEIK | 14 |
| B11 | QVQLVESGAEVKKPGSSVKVSCKAS**GGTFSSYA**ISWVRQAPGQGLEWMGG**IIPIFGTA**NYAQKFQGRVTITADESTSTAYMELSGLRSEDTAVYYCAR**GIQPLRYYGMDV**WGQGTLVTVSSGGGGSGGGGSGGGASQSALTQPPSASGTPGQRVTISCSGS**SSNIGSNY**VYWYQQLPGTAPKLLIY**RNN**QRPSGVPDRFSGSKSGTSASLAISGLRSEDEADYYC**AAWDDSLSGRGV**FGGGTQLTVL | 15 |

(continued)

| scF v ID | scFv Region (Protein; N→C) | SEQ ID NO |
|---|---|---|
| B12 | QVQLVESGGGLVKPGGSLRLSCAAS**GFTFSSYS**MNWVRQAPGKGLEWVSV**IYSGGST**HYADSVKGRFTISRHNSKNTLYLQMNSLRAEDTAVYYCAR**GAGTLNAFDI**WGQGTTVTVSSGGGGSGGGGSGGGASQSGLTQPPSTSGSPGQSVTISCTGT**SSDVGAYSY**VSWYQQHPGKAPKLLIY**AVT**KRPSGVPDRFSGSKSGNTASLTVSGLQDEDEADYYC**SSFAGGSTLV**FGGGTKLTVL | 16 |
| C01 | QMQLVQSGAEVKKPGSSVKVSCKAS**GGTFSSYA**ISWVRQAPGQGLEWMGG**IIPIFGTA**NYAQKFQGRVTITADESTSTAYMELSSLRSEDTAVYYCVR**GYSSIYYYYGMDV**WGQGTMVTVSSGGGGSGGGGSGGGASQSGLTQPRSVSGSPGQSVTISCTGT**SSDVGGYNY**VSWYQQHPGKAPKLMIY**DVS**KRPSGVSDRFSGSKSGNTASLTISGLQAEDEADYYC**GSYTSDGTLV**FGGGTKLTVL | 17 |
| C02 | QVQLVQSGAEVKKPGSSVKVSCKAS**GGTFSSYT**ISWVRQAPGQGLEWMGR**IIPILGIA**NYAQKFQGRVTITADKSTSTAYMELSSLRSEDTAVYYCAR**DRSYNWLDP**WGRGTLVTVSSGGGGSGGGGSGGGASQSALTQPVSVSGSPGQSITISCTGT**ISDVGDYNY**VSWYQQHPGKAPKLMIY**DVN**NRPSGVSNRFSGSKSGNTASLTISGLQAEDEADYYC**SSYTSSSTLV**FGGGTKLTVL | 18 |

(In Table 3, regions in bold and underlined represent -, CDR-H2, and CDR-H3, CDR-L1, CDR-L2, and CDR-L3 in order)

In another specific embodiment, the antibody or antigen binding fragment thereof may comprise the aforementioned 6 CDRs, and be based on an immunoglobulin (for example, IgA, IgD, IgE, IgG (IgG1, IgG2, IgG3, IgG4), IgM, etc.) and lambda or kappa type. The antibody may be a monoclonal antibody, and may be an animal (for example, mouse, rabbit, etc.)-derived antibody, a chimeric antibody, a humanized antibody or a human antibody.

Another embodiment provides a fusion protein comprising the GUCY2C binding polypeptide and a Fc domain of an immunoglobulin.

The GUCY2C binding polypeptide is as described above. The Fc domain of the immunoglobulin may be a Fc domain of an immunoglobulin (for example, IgA, IgD, IgE, IgG (IgG1, IgG2, IgG3, IgG4), IgM, etc.) of a mammal (for example, primates such as humans, monkeys, rodents such as mice, rats, and the like). The Fc domain may comprise or not comprise a hinge region, and may comprise CH2, CH3 or both of them. In the fusion protein provided in the present description, the GUCY2C binding polypeptide and Fc domain of the immunoglobulin may be linked regardless of the order, and for example, the GUCY2C binding polypeptide may be linked to the C terminus or N terminus of the Fc domain of the immunoglobulin, or two or more of GUCY2C binding polypeptides may be linked to one or more of the C-terminus or N-terminus of the Fc domain of the immunoglobulin. The GUCY2C binding polypeptide and the Fc domain polypeptide of the immunoglobulin may be linked through a linker, or be directly linked without a linker.

The GUCY2C binding polypeptide, anti-GUCY2C antibody or antigen binding fragment thereof provided in the present description may have binding affinity ($K_D$) to GUCY2C (for example, human GUCY2C) of 10mM or less, 5 mM or less, 1mM or less, 0.5mM or less, 0.2mM, or 0.15mM or less, for example, based on the case measured by surface plasmon resonance (SPR), and for example, it may be 0.001nM to 10mM, 0.005nM to 10mM, 0.01nM to 10mM, 0.05nM to 10mM, 0.1nM to 10mM, 0.5nM to 10mM, 1nM to 10mM, 0.001nM to 5mM, 0.005nM to 5mM, 0.01nM to 5mM, 0.05nM to 5mM, 0.1nM to 5mM, 0.5nM to 5mM, 1 nM to 5mM, 0.001nM to 1 mM, 0.005nM to 1 mM, 0.01nM to 1 mM, 0.05nM to 1mM, 0.1nM to 1 mM, 0.5nM to 1mM, 1 nM to 1 mM, 0.001nM to 0.5mM, 0.005nM to 0.5mM, 0.01nM to 0.5mM, 0.05nM to 0.5mM, 0.1nM to 0.5mM, 0.5nM to 0.5mM, 1nM to 0.5mM, 0.001nM to 0.2mM, 0.005nM to 0.2mM, 0.01nM to 0.2mM, 0.05nM to 0.2mM, 0.1nM to 0.2mM, 0.5nM to 0.2mM, 1nM to 0.2mM, 0.001nM to 0.15mM, 0.005nM to 0.15mM, 0.01nM to 0.15mM, 0.05nM to 0.15mM, 0.1nM to 0.15mM, 0.5nM to 0.15mM, or 1nM to 0.15mM.

Another embodiment provides a conjugate in which the aforementioned antibody or antigen binding fragment thereof or fusion protein, and a drug. The drug may be one or more kinds selected from the group consisting of anti-cancer agents, constant media, and the like.

The anti-cancer agent may be one or more kinds selected from maytansine, auristatin-based drugs, calicheamicin-based drugs, pyrrolobenzodiazepine-based drugs, duocarmycin, Docetaxel, Doxorubicin, Carboplatin (paraplatin), Cisplatin, Cyclophosphamide, Ifosfamide, Nidran, Nitrogen mustard, Mechlorethamine HCL, Bleomycin, Mitomycin C, Cytarabine, Flurouracil, Gemcitabine, Trimetrexate, Methotrexate, Etoposide, Vinblastine, vinorelbine, Alimta, Altretamine, Procarbazine, Paclitaxel (Taxol), Taxotere, Topotecan, Irinotecan, and the like, but not limited thereto. The constant medium may be one or more kinds selected from MRI (magnetic resonance imaging) constant media such as iron oxide, gadolinium, radioactive isotopes (e.g., iodide, gold, thallium, palladium, cesium, yttrium, gallium, copper, dysprosium, rubidium, ruthenium, radium, fluorine, bismuth, etc.), and the like, and PET (Positron Emission Tomography) constant media, and the like, but not limited thereto.

Chimeric antigen receptor (CAR), chimeric antigen receptor expressing cell

Another embodiment provides a chimeric antigen receptor (CAR) comprising the GUCY2C binding polypeptide. The GUCY2C binding polypeptide may be suitable for using in a chimeric antigen receptor as it may be operated to be expressed as a part of a single chain with other CAR components. The chimeric antigen receptor may be GUCY2C specific.

The chimeric antigen receptor may typically comprise an extracellular domain (ectodomain) comprising the GUCY2C binding polypeptide; a transmembrane domain; and an intracellular signaling domain (or T cell activation domain; endodomain). In addition, the extracellular domain may further comprise a spacer region (or hinge region) between the polypeptide and transmembrane domain. Furthermore, the chimeric antigen receptor may further comprise one or more co-stimulatory domains, and preferably, the co-stimulatory domain may be positioned between the transmembrane domain and intracellular signaling domain.

Therefore, as one preferable embodiment, the chimeric antigen receptor may comprise an extracellular domain comprising the GUCY2C binding polypeptide; a transmembrane domain; one or more co-stimulatory domains; and an intracellular signaling domain. Each domain may be heterogeneous. In other words, it may be composed of a sequence derived from different protein chains. Each domain may be linked by a short oligo- or polypeptide linker, for example, a linker with a length of 2 to 10 amino acids. In addition, the chimeric antigen receptor may consist of one polypeptide chain in which each domain is linked.

The extracellular domain comprises the aforementioned GUCY2C binding polypeptide, and this recognizes GUCY2C expressed on the cancer cell surface.

The extracellular domain may further comprise a spacer region (or hinge region). The spacer region may be positioned between the GUCY2C binding polypeptide and transmembrane domain. The spacer region allows the GUCY2C binding polypeptide to recognize a target antigen more flexibly, spaced a certain distance from the cell membrane of immunocytes (hereinafter, also called 'CAR expressing immunocyte'; for example, including CAR-NK, CAR-T cells, etc.) expressing a chimeric antigen receptor. The spacer region may be typically a polypeptide, and it may have an amino acid length of 10 or more, for example, an amino acid length of 10 to 300, an amino acid length of 10 to 250, an amino acid length of 10 to 200, an amino acid length of 10 to 150, an amino acid length of 10 to 100, or an amino acid length of 10 to 50, but not limited thereto.

As the spacer region, a hinge region of CD8$\alpha$ or CD28, or a constant region of an immunoglobulin (IgG), or the like may be illustrated, and in order to remove the off-target effect thereby, a mutation may be introduced. For example, the immunoglobulin constant region may be derived from an IgG hinge alone, or all or a part of a CH2 and/or CH3 domain, and for example, it may be a Fc region. The IgG (IgG1, IgG2, IgG3, IgG4, etc.) may be IgG2 or IgG4. In some embodiments, the spacer may be a chimeric polypeptide containing one or more among hinges and CH2 and CH3 sequences, derived from IgG2, IgG4, and/or IgG2 and IgG4.

The transmembrane domain plays a role of linking a cell membrane domain and a signaling domain inside the cell membrane, and may be derived from a natural or synthetic source. When the source is natural, the domain may be any membrane-binding or membrane-traversing protein. For example, the transmembrane domain may be a transmembrane domain of alpha, beta or zeta chain, CD3 epsilon, CD4, CD5, CD8, CD9, CD16, CD22, CD28, CD33, CD37, CD45, CD64, CD80, CD86, CD134, CD137 or CD154 of a T cell receptor, but not limited thereto. As one preferable embodiment, the transmembrane domain may be a transmembrane domain of CD28 or CD8, but not limited thereto. When the source is synthetic, the synthetic transmembrane domain may comprise a hydrophobic residue such as leucine and valine, and may comprise phenylalanine, tryptophane and valine, and the like at each terminus, but not limited thereto.

The co-stimulatory domain is a site to which a co-stimulatory signal is transmitted, and is a site for transmitting a signal so that CAR expressing immunocytes generate an immune response and self-proliferate. This may be selectively introduced to improve proliferation, cytotoxicity, sustained response, lifespan extension of CAR expressing immunocytes. The co-stimulatory domain may be one or more kinds, for example, 1 kind, 2 kinds or 3 kinds, selected from signaling sites of CD28, OX-40 (CD134), 4-1 BB (CD137), CD2, CD7, CD27, CD30, CD40, PD-1, ICOS, LFA-1 (CD11a/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor protein, immunoglobulin protein, cytokine receptor, integrin, SLAM (signaling lymphocytic activation molecule), activated NK cell receptor, BTLA, toll ligand receptor, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8 alpha, CD8 beta, IL-2R beta, IL-2R gamma, IL-7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAMF1( CD150, IPO-3), BLAME (SLAMF8), SELPLG (CD162), LTBR, LAT, GADS, SLP-76, PAG/Cbp, or CD19a, but not limited thereto. As a preferable embodiment, it may be one or more kinds, for example, 1 kind, 2 kinds or 3 kinds selected from signaling sites of CD28, OX-40 (CD134), 4-1BB (CD137), CD27 or ICOS, but not limited thereto.

The intracellular signaling domain is a site which activates an immune response of an immunocyte for an antigen bound to the GUCY2C binding polypeptide. The signaling domain may contain a signaling motif known as a component of an immunocyte receptor (e.g., T cell receptor (TCR), etc.) or a tyrosine-based activation motif (immunoreceptor tyrosine-based activation motif, ITAM). As the embodiment, the signaling domain may be a signaling domain derived from TCR or CD3 zeta, FcR gamma, CD3 gamma, CD3 delta and CD3 epsilon, but not limited thereto. As a preferable embodiment, it may be a signaling domain of CD3 zeta, but not limited thereto. The intracellular signaling domain may activate a CAR expressing immunocyte, when the GUCY2C binding polypeptide site of an extracellular domain binds to a target. For example, the CAR may stimulate the function of immunocytes (for example, NK cells, T cells, etc.), for example, cell lysis activity or T-helper activity, and induce secretion of cytokine or other factors.

In some embodiments, the CAR may be expressed in a form comprising a signal sequence. In addition, the CAR may be expressed with an additional sequence useful for monitoring, for example, a ribosome skip sequence such as 2A peptide or truncated cell surface polypeptide (tHER2 or tEGFR or truncated PSMA, etc.).

In the chimeric antigen receptor, the external domain (antigen binding domain and spacer domain), transmembrane domain and internal domain (any one auxiliary stimulating factor or at least one or more auxiliary stimulating factors among two, and a signaling domain) may be a single strand polypeptide linked in order in a direction from the N-terminus to C-terminus or in a direction from the C-terminus to N-terminus.

Another embodiment provides an immunocyte comprising the chimeric antigen receptor described above. The immunocyte may be an immunocyte specific to GUCY2C expressing the GUCY2C specific chimeric antigen receptor on the cell surface. In one embodiment, the immunocyte may be a genetically engineered cell to express the chimeric antigen receptor, for example, an immunocyte in which an encoding polynucleotide of the chimeric antigen receptor or a recombinant vector (expression vector) comprising the same is introduced.

The immunocyte includes a T cell, a tumor infiltrating lymphocyte (TIL), a NK (natural killer) cell, a TCR (T cell antigen receptor)-expressing cell, a dendritic cell or a NK-T cell, but not limited thereto. In addition, the immunocyte may be derived from a human induced pluripotent stem cell (iPSC). The immunocyte may be derived from any known source. For example, the immunocyte may be differentiated in vitro from a hematopoietic stem cell group, or obtained from a patient. The immunocyte may be obtained from, for example, peripheral blood mononuclear cells (PBMC), marrow, lymphatic gland tissue, cord blood, thymus tissue, tissue from an infected site, ascites, pleural effusion, spleen tissue and tumor. In addition, the immunocyte may be derived from one or more kinds of immune cell lines available in the art. The immunocyte may be a cell derived from a mammal (for example, primates such as humans, monkeys, rodents such as mice, rats, and the like).

The immunocyte may be autologous or allogeneic. Autologous refers to one derived from a patient to be treated. Allogeneic refers to one derived from another individual of the same species as a patient to be treated.

Furthermore, the immunocyte may be derived from a human induced pluripotent stem cell (iPSC). The immunocyte derived from iPSC has an advantage in that self-proliferation is possible and mass proliferation is easy and it is possible to manufacture general cell therapeutic agents applicable to all people, compared to autologous or allogeneic immunocytes.

The immunocyte may be transfected or transduced by a vector using a method of microinjection, electroporation, sonication, biolistic (for example, gene gun), lipid transfection, polymer transfection, calcium phosphate precipitation, protoplast fusion, liposome-mediated transfection, nanoparticles or polyplexes, or the like, but not limited thereto.

In the present description, the term "natural killer cells" or "NK cells" are cytotoxic lymphocytes composing a major component of congenital immune system, and is defined as a large granular lymphocyte (LGL) and consists of the third cell differentiated from common lymphoid progenitor (CLP) producing B and T lymphocytes. The "natural killer cells" or "NK cells" may comprise natural killer cells without additional modification derived from any tissue source, and comprise not only mature natural killer cells but also natural killer precursors. The natural killer cells are activated by a reaction for interferon or macrophage-derived cytokine, and the natural killer cells comprise two types of surface receptors controlling cytotoxic activity of cells, labelled as "activated receptors" and "inhibitory receptors". The natural killer cells may be generated from a hematopoietic cell, for example, a hematopoietic stem or precursor from any source, for example, placental tissue, placental perfusate, cord blood, placental blood, peripheral blood, spleen, liver, and the like.

The term "receptor" refers to all molecules which bind to a specific substance to modify the activity of NK cells. The specific substance may include a chemical composition, body-derived or artificial specific protein, peptide, cholesterol and glycoprotein, and include cytokine or chemokine by other immunocytes or NK cells themselves, or a specific receptor or membrane protein of a target cell or NK cell itself. For example, the receptor includes not only a case of positioning on the cell surface of NK cells and binding to a specific substance to transmit a signal into cells and cause activity of NK cells, but also all receptors which are receptors that pass through the cell membrane and are present in the inner surface of the cell membrane or cytoplasm and can bind to an external specific stimulating substance to cause a signaling action. The example of the receptor may include CD16, CD25, CD69, CD117, NKG2D, CD94/NKG2A, 2B4 (CD244), DNAM-1 (CD226), CD2, CXCR3, NKp30, NKp44, NKp46 and NKp80, but not limited thereto. Among the receptors, when CD117 expression is low and CD94/NKG2D expression is high, it can be determined that NK cells exhibit a maturation phenotype. To the "receptor", a cytokine-related receptor, "cytokine receptor" is also included, and to the cytokine receptor, IL-15Ra, IL-18Ra, CD122, PD-1 (CD279) and ICAM-1 (CD54) are included, but not limited thereto.

The NK cell can kill cancer cells by mediating target cancer cell apoptosis directly through secretion of cytokine such as perforin (Prf1), granzyme B (GzmB), interferon-$\gamma$ (IFN-$\gamma$), tumor necrosis factor-$\alpha$ (TNF-$\alpha$) and the like. Accordingly, by confirming the degree of secretion of effector molecules including major factors in the apoptosis process, IFN-$\gamma$, granzyme B and perforin, the killing ability of NK cells can be confirmed.

Polynucleotide, recombinant vector, recombinant cell

In the present description, the aforementioned GUCY2C binding polypeptide, antibody specifically binding to GUCY2 or antigen binding fragment thereof, fusion protein and chimeric antigen receptor and polynucleotide encoding the same may be recombinantly or synthetically produced.

Another embodiment provides a polynucleotide encoding the GUCY2C binding polypeptide, antibody specifically binding to GUCY2 or antigen binding fragment thereof, fusion protein or chimeric antigen receptor. In one specific embodiment, the polynucleotide may be codon-optimized for expression in a human.

In one embodiment, the polynucleotide encoding the GUCY2C binding polypeptide represented by an amino acid sequence of SEQ ID NOs: 1 to 18 may be represented by a nucleic acid sequence of SEQ ID NOs: 20 to 37.

Another embodiment provides a recombinant vector comprising the polynucleotide. The recombinant vector may be an expression vector for expressing the polynucleotide in a host cell. Another embodiment provides a recombinant cell comprising the polynucleotide or recombinant vector. The recombinant cell may be one in which the polynucleotide or recombinant vector is introduced into a host cell.

Another embodiment provides a method for preparing of a GUCY2C binding polypeptide, antibody specifically binding to GUCY2 or antigen binding fragment thereof, fusion protein or chimeric antigen receptor, comprising expressing the polynucleotide in an appropriate host cell. The expressing may comprise culturing a recombinant cell comprising the polynucleotide.

The term "vector" means a means for expressing a target gene (DNA or RNA) in a host cell. For example, a plasmid vector, a cosmid vector and a bacteriophage vector, a virus vector, and the like may be exemplified. In one specific embodiment, the vector may be a virus vector selected from the group consisting of a lentivirus vector, an adenovirus vector, a retrovirus vector, an adeno-associated virus vector (AAV), a murine leukemia virus vector, a SFG vector, a baculovirus vector, an Epstein Barr virus vector, a papovavirus vector, a vaccinia virus vector, a herpes simplex virus vector, and the like, but not limited thereto. In one specific embodiment, the recombinant vector may be produced by engineering a plasmid (for example, pSC101, pGV1106, pACYC177, ColE1, pKT230, pME290, pBR322, pUC8/9, pUC6, pBD9, pHC79, pIJ61, pLAFR1, pHV14, pGEX series, pET series and pUC19, etc.), phage (for example, $\lambda$gt4$\lambda$B, $\lambda$-Charon, $\lambda\Delta$z1 and M13, etc.) or virus (for example, SV40, etc.) commonly used in the art.

In the recombinant vector, the nucleic acid molecule may be operatively linked to a promoter. The term "operatively linked" means functional binding between a nucleotide expression regulatory sequence (for example, promoter sequence) and other nucleotide sequences. The regulatory sequence may regulate transcription and/or translation of other nucleotide sequences by being "operatively linked".

The recombinant vector may be typically constructed as a vector for cloning or vector for expression. As the vector for expression, common one used for expressing foreign protein in a plant, animal or microorganism in the art may be used. The recombinant vector may be constructed by various methods known in the art.

The recombinant vector may be constructed by using a prokaryotic cell or eukaryotic cell as a host. For example, when the used vector is an expression vector and a prokaryotic cell is used as a host, it is common to comprise a strong promoter capable of progressing transcription (for example, $pL^{\lambda}$ promoter, CMV promoter, *trp* promoter, *lac* promoter, *tac* promoter, T7 promoter, etc.), a ribosome binding site for initiation of translation and a transcription/translation termination sequence. When a eukaryotic cell is used as a host, a replication origin operating in a eukaryotic cell comprised in a vector includes f1 replication origin, SV40 replication origin, pMB1 replication origin, adeno replication origin, AAV replication origin and BBV replication origin, and the like, but not limited thereto. In addition, a promoter derived from genome of a mammal cell (for example, metallothionein promoter) or a promoter derived from a mammal virus (for example, adenovirus late promoter, vaccinia virus 7.5K promoter, SV40 promoter, cytomegalovirus promoter, *tk* promoter of HSV, etc.) may be used, and as the transcription termination sequence, it generally has a polyadenylated sequence.

The recombinant cell may be obtained by introducing the recombinant vector into an appropriate host cell. As the host cell, any host cell known in the art may be used as a cell capable of cloning or expressing the recombinant vector stably and continuously, and the prokaryotic cell includes for example, E. coli such as *E. coli* JM109, *E. coli* BL21, *E. coli* RR1, *E. coli* LE392, *E. coli* B, *E. coli* X 1776, *E. coli* W3110, and the like, Bacillus sp. strains such as Bacillus subtilis and Bacillus thuringiensis, and Enterobacteriaceae strains such as Salmonella typhimurium, Serratia marcescens and various Pseudomonas species, and the like, and when transformed in an eukaryotic cell, as a host cell, a yeast (*Saccharomyces cerevisiae*), an insect cell, a plant cell and an animal cell, for example, Sp2/0, CHO (Chinese hamster ovary) K1, CHO DG44, CHO S, CHO DXB11, CHO GS-KO, PER.C6, W138, BHK, COS-7, 293, HepG2, Huh7, 3T3, RIN, MDCK cell line, and the like may be used, but not limited thereto.

Delivery (introduction) of the nucleic acid molecule and recombinant vector comprising thereof into a host cell may use a method for delivery widely known in the art. The delivery method, for example, may use $CaCl_2$ method or electroporation method, or the like when the host cell is a prokaryotic cell, and may use microinjection, calcium phosphate precipitation, electroporation, liposome-mediated transfection and gene bombardment and the like when the host cell is a eukaryotic cell, but not limited thereto.

The method for selecting the transformed host cell may be easily conducted according to a method widely used in the art, by using a phenotype expressed by a selective label. For example, when the selective label is a specific antibiotic-resistant gene, a transformant may be easily selected by culturing the transformant in a medium in which the antibiotic is contained.

Another embodiment provides a method for preparing an anti-GUCY2C antibody or antigen binding fragment thereof comprising expressing the nucleic acid molecule or recombinant vector comprising thereof in a host cell. The expressing may be performed by culturing the recombinant comprising the nucleic acid molecule (for example, comprised in the recombinant vector) under the condition of allowing expressing of the nucleic acid molecule. The method for preparing may comprise separating and/or purifying an antibody or antigen binding fragment from a culture medium, after the expressing or culturing.

## Medical use

Another embodiment provides a composition for detecting GUCY2C comprising one or more kinds selected from the group consisting of the GUCY2C binding polypeptide, antibody specifically binding to GUCY2C or antigen binding fragment thereof, fusion protein, chimeric antigen receptor, polynucleotide encoding them and immunocyte as an active ingredient.

Another embodiment provides a method for detecting GUCY2C, comprising contacting one or more kinds selected from the group consisting of the GUCY2C binding polypeptide, antibody specifically binding to GUCY2C or antigen binding fragment thereof, fusion protein, chimeric antigen receptor, polynucleotide encoding them and immunocyte to a biological sample. The biological sample may be one or more kinds selected from the group consisting of a separated cell, tissue, body fluid and culture thereof. In the method, GUCY2C detection in the sample may be performed by confirming the reaction of the a GUCY2C binding polypeptide, antibody specifically binding to GUCY2C or antigen binding fragment thereof, fusion protein, chimeric antigen receptor, polynucleotide encoding them and immunocyte with GUCY2C (for example, confirming complex formation).

Another embodiment provides a use for using in detection of GUCY2C, of one or more kinds selected from the group consisting of the GUCY2C binding polypeptide, antibody specifically binding to GUCY2C or antigen binding fragment thereof, fusion protein, chimeric antigen receptor, polynucleotide encoding them and immunocyte.

Another embodiment provides a composition for diagnosing cancer comprising one or more kinds selected from the group consisting of the GUCY2C binding polypeptide, antibody specifically binding to GUCY2C or antigen binding fragment thereof, fusion protein, chimeric antigen receptor, polynucleotide encoding them and immunocyte as an active ingredient.

Another embodiment provides a method for diagnosing cancer or a method for providing information for diagnosis of cancer, comprising contacting one or more kinds selected from the group consisting of the GUCY2C binding polypeptide, antibody specifically binding to GUCY2C or antigen binding fragment thereof, fusion protein, chimeric antigen receptor, polynucleotide encoding them and immunocyte to a biological sample obtained from a subject. The subject may be a patient in need of diagnosis of cancer, and the biological sample may be one or more kinds selected from the group consisting of a cell, tissue, body fluid and culture thereof. In the method, when GUCY2C is detected, more specifically, when a complex of the GUCY2C binding polypeptide, antibody specifically binding to GUCY2C or antigen binding fragment thereof, fusion protein, chimeric antigen receptor, polynucleotide encoding them and immunocyte and GUCY2C is detected, it can be confirmed (decided, determined) that the biological sample comprises a cancer cell, or the patient from which the biological sample is obtained is a cancer patient.

Another embodiment provides a use for using in diagnosis of cancer or a use for using in preparation of a composition for diagnosis of cancer, of one or more kinds selected from the group consisting of the GUCY2C binding polypeptide, antibody specifically binding to GUCY2C or antigen binding fragment thereof, fusion protein, chimeric antigen receptor, polynucleotide encoding them and immunocyte.

The GUCY2C detection and/or complex formation confirmation may be performed by a common means of confirming protein-protein interaction or complex formation between protein-protein, and for example, it may be measured by a method selected from the group consisting of immunochromatography, immunohistochemical staining, enzyme linked immunosorbent assay (ELISA), radioimmunoassay (RIA), enzyme immunoassay (EIA), florescence immunoassay (FIA), luminescence immunoassay (LIA), western blotting, microarray, and the like, but not limited thereto.

The biological sample may be a subject (for example, mammals including primates such as humans, monkeys, rodents such as mice, rats, and the like) or a cell, tissue, body fluid (for example, blood, serum, urine, saliva, etc.) separated or artificially cultured from the subject, or the like.

Another embodiment provides a pharmaceutical composition for prevention and/or treatment of cancer comprising one or more kinds selected from the group consisting of the GUCY2C binding polypeptide, antibody specifically binding to GUCY2C or antigen binding fragment thereof, fusion protein, conjugate, chimeric antigen receptor, a polynucleotide encoding them, a recombinant vector comprising the polynucleotide, a recombinant cell comprising the recombinant vector, and an immunocyte expressing the chimeric antigen receptor as an active ingredient.

Another embodiment provides a method for prevention and/or treatment of cancer, comprising administering a pharmaceutically effective dose of one or more kinds selected from the group consisting of the GUCY2C binding polypeptide, antibody specifically binding to GUCY2C or antigen binding fragment thereof, fusion protein, conjugate, chimeric antigen receptor, a polynucleotide encoding them, a recombinant vector comprising the polynucleotide, a recombinant cell comprising the recombinant vector, and an immunocyte expressing the chimeric antigen receptor into a subject in need of prevention and/or treatment of cancer.

Another embodiment provides a use for using in prevention and/or treatment of cancer or a use for using in preparation of a pharmaceutical composition for prevention and/or treatment of cancer of one or more kinds selected from the group consisting of the GUCY2C binding polypeptide, antibody specifically binding to GUCY2C or antigen binding fragment thereof, fusion protein, conjugate, chimeric antigen receptor, a polynucleotide encoding them, a recombinant vector comprising the polynucleotide, a recombinant cell comprising the recombinant vector, and an immunocyte expressing the chimeric antigen receptor.

The pharmaceutical composition provided in the present description may further comprise a pharmaceutically acceptable carrier, in addition to the active ingredient (GUCY2C binding polypeptide, antibody specifically binding to GUCY2C or antigen binding fragment thereof, fusion protein, chimeric antigen receptor, a polynucleotide encoding them, a recombinant vector comprising the polynucleotide, a recombinant cell comprising the recombinant vector, and an immunocyte expressing the chimeric antigen receptor). The pharmaceutically acceptable carrier is one commonly used in preparation of a drug, and may be one or more kinds selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, and the like, but not limited thereto. The pharmaceutical composition may further comprise one or more kinds selected from the group consisting of diluents, excipients, lubricants, wetting agents, sweeteners, flavoring agents, emulsifiers, suspending agents, preservatives, and the like commonly used in preparation of a pharmaceutical composition.

The effective dose of the pharmaceutical composition or the antibody or antigen binding fragment thereof may be administered orally or parenterally. In case of parenteral administration, it may be administered by intravenous injection, subcutaneous injection, intramuscular injection, intraperitoneal injection, endothelial administration, intrapulmonary administration, intrarectal administration or lesion site local administration, or the like. In case of oral administration, as a protein or peptide is digested, an oral composition may be formulated to coat an active drug or protect it from degradation in stomach. In addition, the composition may be administered by any device in which an active substance can move to a target cell (for example, cancer cell).

The anti-GUCY2C antibody or antigen binding fragment thereof may be comprised in the pharmaceutical composition or administered into a patient in a pharmaceutically effective dose. In the present description, "pharmaceutically effective dose" may mean an amount of an active ingredient capable of exhibiting the desired effect (for example, anticancer effect) of the active ingredient (GUCY2C binding polypeptide, antibody specifically binding to GUCY2C or antigen binding fragment thereof, fusion protein, chimeric antigen receptor and/or immunocyte). The pharmaceutically effective dose may be variously prescribed by factors such as patient's age, body weight, gender, morbid condition, food, excretion rate, reaction sensitivity, preparation method, administration time, administration interval, administration route, administration method, and the like. For example, a daily dose of the active ingredient may be in a range of 0.005 ug/kg to 1000mg/kg, 0.005 ug/kg to 500mg/kg, 0.005 ug/kg to 250mg/kg, 0.005 ug/kg to 100mg/kg, 0.005 ug/kg to 75mg/kg, 0.005 ug/kg to 50mg/kg, 0.01 ug/kg to 1000mg/kg, 0.01 ug/kg to 500mg/kg, 0.01 ug/kg to 250mg/kg, 0.01 ug/kg to 100mg/kg, 0.01 ug/kg to 75mg/kg, 0.01 ug/kg to 50mg/kg, 0.05 ug/kg to 1000mg/kg, 0.05 ug/kg to 500mg/kg, 0.05 ug/kg to 250mg/kg, 0.05 ug/kg to 100mg/kg, 0.05 ug/kg to 75mg/kg, or 0.05 ug/kg to 50mg/kg, but not limited thereto. The daily dose may be prepared by formulating into one preparation in a single dose form, formulating by appropriately distributing, or internalizing in a multi-dose container.

The pharmaceutical composition may be formulated in a form of solution in an oil or aqueous medium, suspension, syrup or emulsion, or in a form of extract, powder, powder, granules, tablets or capsules, or the like, and may additionally comprise a dispersant or stabilizing agent for formulation.

The application subject patient of the present invention may be a mammal including primates such as humans, monkeys, rodents such as mice, rats, and the like.

The subject cancer for diagnosis and/or treatment of the composition and/or method provided in the present description may be solid cancer or blood cancer, and it is not limited thereto, but it may be colorectal cancer, colon cancer, colorectal cancer, rectal cancer, breast cancer, lung cancer, prostate cancer, ovarian cancer, brain cancer, liver cancer, cervical cancer, endometrial cancer, uterine cancer, kidney cancer, nephroblastoma, skin cancer, oral squamous carcinoma, epidermal cancer, nasopharyngeal cancer, head and neck cancer, bone cancer, esophageal cancer, bladder cancer, lymphatic cancer (for example, Hodgkin's lymphoma), stomach cancer, pancreatic cancer, testicular cancer, thyroid cancer, thyroid follicular cancer, melanoma, myeloma, multiple myeloma, mesothelioma, osteosarcoma, myelodysplastic syndrome, tumor of mesenchymal origin, soft tissue sarcoma, liposarcoma, gastrointestinal stromal sarcoma, malignant peripheral nerve sheath tumor (MPNST), Ewing sarcoma, leiomyosarcoma, mesenchymal chondrosarcoma, lymphosarcoma, fibrosarcoma, rhabdomyosarcoma, teratocarcinoma, neuroblastoma, medulloblastoma, glioma, benign tumor of skin, or leukemia. The lung cancer, may be for example, small cell lung carcinoma (SCLC) or non-small cell lung carcinoma (NSCLC). The leukemia may be for example, acute myeloid leukemia (AML), chronic myeloid leukemia (CML), acute lymphocytic leukemia (ALL) or chronic lymphocytic leukemia (CLL). The cancer may be primary cancer or metastatic cancer. The cancer may be cancer expressing or overexpressing GUCY2C, for example, colorectal cancer expressing or overexpressing GUCY2C, or colorectal cancer-derived metastatic cancer, but not limited thereto.

Treatment of cancer in the present description may mean all anticancer actions which prevent or alleviate or improve degeneration of symptoms of cancer, or destroying cancer partially or completely, such as inhibition of proliferation of cancer cells, cancer cell death, metastasis inhibition and the like.

In one specific embodiment, the treatment subject patient may be a patient receiving secondary anti-hyperproliferative therapy. For example, the secondary anti-hyperproliferative therapy may be chemotherapy, radiation therapy, immunotherapy, phototherapy, cryotherapy, toxin therapy, hormone therapy or surgical operation.

In one embodiment, anticancer treatment using an immunocyte comprising a chimeric antigen receptor comprising the GUCY2C binding polypeptide of the present application may be achieved by a series of processes of extracting immunocytes (for example, NK cells, T cells, etc.) in blood of a healthy person or a patient to be treated, and then genetically engineering to express a chimeric antigen receptor comprising the GUCY2C binding polypeptide of the present application, and amplifying and culturing the engineered immunocytes and administering the cultured engineered immunocytes into a patient.

As a preferable embodiment, the extracting immunocytes in blood of a healthy person or a patient to be treated, for example, may extract immunocytes by passing through a process of separating leukocytes using leukapheresis (or aphresis) and then concentrating immunocytes. The immunocytes may be separated by using a specific antibody bead binder or marker at a level of CD4/CD8 configuration. Alternatively, it is possible to obtain a large amount of immunocytes stably through differentiation from stem cells.

The genetically engineering so that the extracted immunocytes express the chimeric antigen receptor provided in the present application, for example, may inject a nucleic acid molecule designed to express a chimeric antigen receptor (CAR) by using a vector, for example, a virus vector (lentivirus vector or retrovirus vector, etc.). The CAR may be introduced in a DNA form, and may be integrated into genome of immunocytes, after being introduced in an RNA form and then reverse transcribed into DNA with reverse transcriptase.

The amplifying and culturing the engineered immunocytes may culture, proliferate and amplify (expansion) immunocytes according to culture technology known in the art. Then, safety against use of viruses and technology for selecting well-produced CAR expressing immunocytes are required.

Finally, the administering the engineered immunocytes into a patient again, for example, may be achieved by infusion. As one embodiment, before infusion of CAR expressing immunocytes, in order to lower the leukocyte count, the patient may receive chemotherapy to control lymphocyte removal using cyclophosphamide or fludarabine, or the like. In addition, in order to improve persistence of CAR expressing immunocytes, cytokine such as IL-2, and the like may be administered together.

The engineered immunocyte administered into a patient may mediate an immune response for a tumor cell. This immune response includes activation of immunocytes, secretion of cytokine such as IL-2 and IFN-gamma by immunocytes, proliferation and extension of immunocytes recognizing a tumor antigen, and immunocyte-mediated specific death (tumor removal) of a target-positive cell. For example, when CAR specifically binds to GUCY2C in a CAR expressing immunocyte, the immunocyte may be activated through phosphorylation of a tyrosine-based activation motif (immunoreceptor tyrosine-based activation motif, ITAM) of CD3 zeta and then proliferation, cytotoxicity and/or secretion of cytokine of the immunocyte may be induced.

As described above, anticancer therapy using CAR expressing immunocytes fundamentally activates immune system of a patient to exhibit a continuous anticancer effect, and therefore, it has an advantage of no need to be administered continuously and enabling personalized treatment by using patient's own immunocytes.

Administration of the composition provided in the present description may inhibit or stop or delay occurrence or progression of disease condition, or cause or induce or promote a protective immune response.

[ADVANTAGEOUS EFFECTS]

Immunocytes expressing binding fragments (antibody, scFv) which binds to GUCY2C provided in the present description with high affinity and a chimeric antigen receptor (CAR) comprising the same on the surface can be usefully applied as an anticancer agent having an excellent anticancer effect against cancer, in particular, cancer expressing GUCY2C.

[BRIEF DESCRIPTION OF THE DRAWINGS]

FIG. 1a is a schematic diagram schematically showing the process of measuring the binding affinity to GUCY2C of scFv using ELISA.

FIG. 1b is a graph showing the binding affinity to 3 kinds of GUCY2C (human GUCY2C, monkey GUCY2C, and mouse GUCY2C) of scFv measured by ELISA.

FIG. 2a is a schematic diagram schematically showing the process of measuring affinity ranking to GUCY2C of scFv using ELISA.

FIGs. 2b and 2c shows the result of measuring affinity ranking to GUCY2C of scFV measured by ELISA, and 2b shows the result of scFv 5nM, and 2c shows the result of scFv 50nM.

FIG. 3a shows the result of cell binding assay for GUCY2C cells of scFv.

FIG. 3b is a graph showing MFI (mean of fluorescence intensity) obtained as the result of the cell binding assay for GUCY2C cells of scFv.

FIG. 4a is a graph showing the result of confirming expression of an NK cell surface marker of naive NK cells differentiated in iPSC by flow cytometry.

FIG. 4b is a graph showing the result of confirming expression of an NK cell surface marker of naive NK cells differentiated in iPSC.

FIG. 4c is a graph showing the result of confirming expression of an effector molecule of the apoptosis process of NK cells of naive NK cells differentiated in iPSC.

FIG. 5 is a graph showing the expression level of anti-GUCY2C CAR in NK cells in which anti-GUCY2C CAR is introduced.

FIG. 6a is a graph showing cytotoxicity of anti-GUCY2C-CAR expressing NK cells for target cells not expressing GUCY2C.

FIG. 6b is a graph showing cytotoxicity of anti-GUCY2C-CAR expressing NK cells for target cells expressing GUCY2C.

FIG. 7 is a graph showing CAR-dependent killing ability of anti-GUCY2C-CAR expressing NK cells for target cells not expressing GUCY2C and target cells expressing GUCY2C in vitro.

FIG. 8 is a graph showing CAR-dependent killing ability of anti-GUCY2C-CAR expressing NK cells and CD19 targeting CAR-NK cells in vivo.

FIG. 9a is a graph of confirming that the secreted IFN-$\gamma$ amount is significantly increased, when anti-GUCY2C-CAR expressing NK cells are co-cultured with target cells expressing GUCY2C. In the graph, "No NK" is an experimental group untreated with NK cells and corresponds to a value 0.

FIG. 9b is a graph of confirming that the IFN-$\gamma$ amount is significantly increased, when CAR-NK cells comprising GUCY2C binding scFV (5F9, D08, G07) are co-cultured with T84 cells, which are GUCY2C positive cancer cells.

In the graph, "No NK" is an experimental group untreated with NK cells and corresponds to a value 0.
FIG. 10 is a graph which compares the survival rate when anti-GUCY2C-CAR expressing NK cells are administered to the survival rate of the control group (vehicle administration group).

[MODE FOR INVENTION]

Hereinafter, the present invention will be described by examples in more detail, but they are illustrative only, and are not intended to limit the scope of the present invention. It is obvious to those skilled in the art that the examples described below can be modified within a range without departing from the essential gist of the invention.

**Example 1: Production of GUCY2C binding scFv**

A recombinant antigen was prepared by conjugating human GUCY2C (R&D Systems, Cat no. 2157-GC; SEQ ID NO: 112)) with CD4 (SEQ ID NO: 115), and clones secreting a scFv specific to the antigen were screened, to secure 18 scFvs specifically binding to GCUCY2C.
The scFv obtained as above and nucleic acid molecules encoding thereof were shown in Table 4 and Table 5 below, respectively:

[Table 4]

| scFv amino acid sequence | | | | |
|---|---|---|---|---|
| **scF v ID** | **Clone ID** | **Light chain subtyp e** | **scFv Region (Protein; N→C)** | **SEQ ID NO** |
| A01 | 2426_ 01_A0 2 | Kappa | QVQLVQSGAEVKKPGASVKVSCKAS**GYTFTSYY**MHWVRQAPGQGLEWMGI**INPSGGST**SYAQEFQGRVTMTRDTSTSTVYMELSSLRSEDTAVYYCAR**DGQWLQFDY**WGQGTLVTVSSGGGGSGGGGSGGGASDIVMTQSPLSLPVTLGQPASISCRSS**QSLLKKSDGNTY**LSWYHQRPGQSPRRLIY**KVS**NRDSGVPDRFSGSGSDTDFTLKISRVETEDVGIYYC**MQGSHWPPT**FGQGTKVEIK | 1 |
| A02 | 2427_ 01_A0 8 | Lambd a | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSELTQPASVSGSPGQSITISCTGT**SSDVGGYIY**VSWYQQHPGKVPKLMIH**DVS**HRPSGVSNRFSGSRSGNTASLTISGLQAEDEADYFC**SSYAGSNNYV**FGTGTKVTVL | 2 |

(continued)

| scFv amino acid sequence | | | | |
|---|---|---|---|---|
| scF v ID | Clone ID | Light chain subtype | scFv Region (Protein; N→C) | SEQ ID NO |
| A03 | 2427_01_A12 | Lambd a | QVQLVQSGAEVKKPGSSVKVSCKAS**GGTFSSYT**ISWVRQAPGQELEWMGR**IIPILGIA**NYAQKFQGRVTITADKSTSTAYMELSSLRSEDTAVYYCAR**DYSSSWNSMDV**WGQGTLVTVSSGGGGSGGGGSGGGASQSGLTQPPSASGSPGQSVTISCTGT**SSDIGYYHY**VSWYQQHPGKAPKLMIY**EDS**KRPSGISNRFSGSKSGTTASLTVSGLQAEDEAHYYC**SSFTSRSTWV**FGGGTQLTVL | 3 |
| A04 | 2427_01_B02 | Lambd a | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSELTQPASVSGSPGQSITISCTGT**SSDVGGYIY**VSWYQQHPGKVPKLMIH**DVS**HRPSGVSNRFSGSRSGNTASLTISGLQAEDEADYFC**SSYTSSNNYY**FGTGTKVTVL | 4 |
| A05 | 2427_01_B07 | Lambd a | QVQLVQSGAEVKKPGSSVKVSCKAS**GGTFSSYT**ITWVRQAPGQGLEWMGR**IIPVLGIA**NYAQKFQGRVTITADKSTSTAYMELSSLRSEDTAVYYCAR**DYSSSWNSMDV**WGQGTLVTVSSGGGGSGGGGSGGGASQSGLTQPRSVSGSPGQSVTISCTGT**SSDVGGYNY**VSWYQQHPGKAPKLMIY**DVS**KRPSGVPDRFSGSKSGNTASLTVSGLHAEDEADYYC**SSYAGSNNFV**FGTGTKVTVL | 5 |
| A06 | 2427_01_C01 | Lambd a | QVQLVQSGAEVKKPGSSVKVSCKAS**GGTFGSYT**ISWVRQAPGQGLEWMGR**IIPILGIA**NYAQKFQGRVTITADKSTSTAYMELSSLRSEDTAVYYCAR**DYSSSWNSMDV**WGQGTLVTVSSGGGGSGGGGSGGGASQSGLTQPRSVSGSPGQSVTISCTGT**SSDVGAYNY**VSWYQQHPGKAPKLMIY**EVS**KRPSGVPDRFSASKSGNTASLTVSGLQAEDEADYYC**SSYAGSNNWV**FGGGTKLTVL | 6 |

(continued)

| scFv amino acid sequence | | | | |
|---|---|---|---|---|
| scF v ID | Clone ID | Light chain subtype | scFv Region (Protein; N→C) | SEQ ID NO |
| A07 | 2427_01_C02 | Lambda | QVQLQESGPGLVKPSETLSLTCTVS**GGSISSYY**WSWIRQPPGKGLEWIGS**IYYSGST**NYNPSLKSRVTISRDKSKNQLFLKLNSMTAADTAVYYCAR**DVWGSGQSFDS**WGQGTLVTVSSGGGGSGGGGSGGGASNFMLTQPHSVSESPGKTVTISCTRS**SGSIASNY**VQWYQQRLGSSPTTVIY**EHS**RRPSGVPDRFSASIDSSSNSASLTISGLKTEDEADYYC**QSYDVSNRV**FGGGTKLTVL | 7 |
| A08 | 2432_01_D05 | Kappa | EVQLLESGGGLVQPGGSLRLSCAAS**GFTFSSYW**MSWVRQAPGKGLEWVAN**IKQDGSEK**YYVDSVKGRFTISRDNAKNSLYLQMNSLRAEDTAVYYCAK**APWYSSSPTPYGMDV**WGQGTLVTVSSGGGGSGGGGSGGGASDIQMTQSPSSLSASVGDRVTITCQAS**QDISNY**LNWYQQKPGKAPRRLIY**GAS**TLMSGVPSRFSGSGSGTDFTLTISSLQPEDFATYYC**QQSYSTPLT**FGGGTKVEIK | 8 |
| A10 | 2432_01_D08 | Kappa | EVQLVQSGAEVKKPGSSVKVSCKAS**GGTFSSYA**ISWVRQAPGQGLEWMGG**IIPIFGTA**NYAQKFQGRVTITADESTSTAYMELSSLRSEDTAVYYCAR**TRYIWGSYRAYGMDV**WGQGTMVTVSSGGGGSGGGGSGGGASDIQMTQSPSSMSASVGDRVTITCRAS**QSISSH**LNWYQQLPGNAPTLLIY**YAS**NLQSGVPSRFSGSGSGTDFTLTISSLQPDDFATYYC**QQSISLPYT**FGQGTKVEIK | 9 |
| A12 | 2433_01_G08 | Lambda | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSELTQPASVSGSPGQSITISCTGT**SSDVGGYIY**VSWYQQHPGKVPKLMIH**DVS**HRPSGVSNRFSGSRSGNTASLTISGLQAEDEADYFC**SSYTSSNNYV**FGTGTKVTVL | 10 |

(continued)

| scFv amino acid sequence | | | | |
|---|---|---|---|---|
| scF v ID | Clone ID | Light chain subtyp e | scFv Region (Protein; N→C) | SEQ ID NO |
| B01 | 2433_01_H07 | Lambd a | EVQLVQPGAEVKKPGSSVKVSCKAS**GGTLSSYA**ISWVRQAPGQGLEWMGR**IIPILGIT**NYAQKFQGRVTITADKSTSTAYMELSSLRSEDTAVYFCAR**DQRPASMDV**WGQGTLVTVSSGGGGSGGGGSGGGASQSGLTQPASVSGSPGQSITISCTGT**SSDVGGYNY**VSWYQQHPGKAPKLMIY**EVS**NRPSGVSNRFSGSKSGNTASLTISGLQAEDEADYYC**STVTSLSTYV**FGTGTKLTVL | 11 |
| B07 | 2436_02_F10 | Kappa | EVQLVQSGAEVKRPGSSVKVSCKAS**GYTFTSYY**MHWVRQAPGQGLEWMGI**INPSGGST**SYAQKFQGRVTMTRDTSTSTVYMELSSLRSEDTAVYYCAA**GTYSSGWTIDY**WGQGTTVTVSSGGGGSGGGGSGGGASDIVMTQSPLSLPVTLGQPASISCRSS**QSLVYTDGNTY**LNWFQQRPGQSPRRLIY**KVS**NRDSGVPDRFSGSGSGTDFTLKISRVEAEDVGIYYC**MHSKQWPPT**FGGGTKVEIK | 12 |
| B08 | 2436_02_F11 | Kappa | EVQLVQSGAEVKKPGSSVKVSCKAS**GGTFSSYA**ISWVRQAPGQGLEWMGG**IIPIFGTA**NYAQKFQGRVTITADKSTSTAYMELSSLRSEDTAVYYCAR**GHYYYMDV**WGQGTTVTVSSGGGGSGGGGSGGGASDIVMTQSPATLSVSPGEGATLSCRAS**QSVSSN**LAWYQQKPGRAPRLLIY**GAS**TRATGIPARFSGSGSGTEFTLTISSLQSEDFAVYYC**QQYNNWPS**FGGGTKLEIK | 13 |
| B10 | 2436_02_G01 | Kappa | EVQLVQSGAEVKKPGSSVKVSCKAS**GGTFSSYA**ISWVRQAPGQGLEWMGG**IIPIFGTA**NYAQKFQGRVTITADKSTSTAYMELSSLRSEDTAVYYCAR**GHYYYMDV**WGQGTTVTVSSGGGGSGGGGSGGGASDIVMTQSPATLSVSPGEGATLSCRAS**QSVSSN**LAWYQQKPGRAPRLLIY**GAS**TRATGIPARFSGSGSGTEFTLTISSLQSEDFAVYYC**QQYNNWPT**FGGGTKLEIK | 14 |

(continued)

| scFv amino acid sequence | | | | |
|---|---|---|---|---|
| scF v ID | Clone ID | Light chain subtyp e | scFv Region (Protein; N→C) | SEQ ID NO |
| B11 | 2437_ 02_G0 7 | Lambd a | QVQLVESGAEVKKPGSSVKVSCKAS**GGTFSSY A**ISWVRQAPGQGLEWMGG**IIPIFGTA**NYAQKFQ GRVTITADESTSTAYMELSGLRSEDTAVYYCAR **GIQPLRYYGMDV**WGQGTLVTVSSGGGGSGGG GSGGGASQSALTQPPSASGTPGQRVTISCSGS **SSNIGSNY**VYWYQQLPGTAPKLLIY**RNN**QRPSG VPDRFSGSKSGTSASLAISGLRSEDEADYYC**AA WDDSLSGRGV**FGGGTQLTVL | 15 |
| B12 | 2437_ 02_G1 0 | Lambd a | QVQLVESGGGLVKPGGSLRLSCAAS**GFTFSSY S**MNWVRQAPGKGLEWVSV**IYSGGST**HYADSV KGRFTISRHNSKNTLYLQMNSLRAEDTAVYYCA R**GAGTLNAFDI**WGQGTTVTVSSGGGGSGGGG SGGGASQSGLTQPPSTSGSPGQSVTISCTGT**S SDVGAYSY**VSWYQQHPGKAPKLLIY**AVT**KRPS GVPDRFSGSKSGNTASLTVSGLQDEDEADYYC **SSFAGGSTLV**FGGGTKLTVL | 16 |
| C01 | 2437_ 02_H0 5 | Lambd a | QMQLVQSGAEVKKPGSSVKVSCKAS**GGTFSSY A**ISWVRQAPGQGLEWMGG**IIPIFGTA**NYAQKFQ GRVTITADESTSTAYMELSSLRSEDTAVYYCVR **GYSSIYYYYGMDV**WGQGTMVTVSSGGGGSGG GGSGGGASQSGLTQPRSVSGSPGQSVTISCTG T**SSDVGGYNY**VSWYQQHPGKAPKLMIY**DVS**KR PSGVSDRFSGSKSGNTASLTISGLQAEDEADYY C**GSYTSDGTLV**FGGGTKLTVL | 17 |
| C02 | 2437_ 02_H0 8 | Lambd a | QVQLVQSGAEVKKPGSSVKVSCKAS**GGTFSSY T**ISWVRQAPGQGLEWMGR**IIPILGIA**NYAQKFQ GRVTITADKSTSTAYMELSSLRSEDTAVYYCAR **DRSYNWLDP**WGRGTLVTVSSGGGGSGGGGS GGGASQSALTQPVSVSGSPGQSITISCTGT**ISD VGDYNY**VSWYQQHPGKAPKLMIY**DVN**NRPSG VSNRFSGSKSGNTASLTISGLQAEDEADYYC**SS YTSSSTLV**FGGGTKLTVL | 18 |

(continued)

| scFv amino acid sequence | | | | |
|---|---|---|---|---|
| **scF v ID** | **Clone ID** | **Light chain subtyp e** | **scFv Region (Protein; N→C)** | **SEQ ID NO** |
| C07 (posi tive cont rol) | 5F9 scFv | Kappa | EIVMTQSPATLSVSPGERATLSCRASQSVSRNL AWYQQKPGQAPRLLIYGASTRATGIPARFSGSG SGTEFTLTIGSLQSEDFAVYYCQQYKTWPRTFG QGTNVEIKASGGGGSGGGGSGGGGSGGGGS ELQVQLQQWGAGLLKPSETLSLTCAVFGGSFS GYYWSWIRQPPGKGLEWIGEINHRGNTNDNPS LKSRVTISVDTSKNQFALKLSSVTAADTAVYYCA RERGYTYGNFDHWGQGTLVTVSS | 19 |

(In Table 4, regions in bold and underlined represent CDR-H1, CDR-H2, and CDR-H3, CDR-L1, CDR-L2, and CDR-L3 in order)

[Table 5]

| scFv encoding nucleic acid molecule | | |
|---|---|---|
| **scF v ID** | **scFv Region (Nucleotides; 5'→3')** | **SEQ ID NO** |
| A01 | CAGGTGCAGCTGGTGCAGTCTGGGGCTGAGGTGAAGAAGC CTGGGGCCTCAGTGAAGGTTTCCTGCAAGGCATCTGGATAC ACCTTCACCAGCTACTATATGCACTGGGTGCGACAGGCCCC TGGACAAGGGCTTGAGTGGATGGGAATAATCAACCCTAGTG GTGGTAGCACAAGCTACGCACAGGAGTTCCAGGGCAGAGTC ACCATGACCAGGGACACGTCCACGAGCACAGTCTACATGGA GCTGAGCAGCCTGAGATCTGAGGACACGGCCGTGTATTACT GTGCGAGAGATGGGCAGTGGCTTCAATTTGACTACTGGGGC CAAGGAACCCTGGTCACCGTCTCGAGTGGTGGAGGCGGTTC AGGCGGAGGTGGCTCTGGCGGTGGCGCTAGCGATATTGTG ATGACACAGTCTCCACTCTCCCTGCCCGTCACCCTTGGGCA GCCGGCCTCCATCTCCTGCAGGTCTAGTCAAAGCCTCCTAAA AAAGAGTGATGGGAACACCTACTTGAGTTGGTATCACCAGAG GCCAGGCCAATCTCCACGGCGCCTAATTTATAAGGTTTCTAA TCGGGACTCTGGGGTCCCAGACAGATTCAGCGGCAGTGGGT CAGACACTGATTTCACTCTGAAAATCAGCAGAGTGGAGACTG AGGATGTTGGAATTTATTACTGCATGCAAGGTTCACACTGGC CTCCGACGTTCGGCCAAGGGACCAAGGTGGAAATCAAA | 20 |

(continued)

| scFv encoding nucleic acid molecule | | |
|---|---|---|
| **scF v ID** | **scFv Region (Nucleotides; 5'→3')** | **SEQ ID NO** |
| A02 | GAGGTGCAGCTGGTGCAGCCTGGGGCTGAGGTGAAGAAGCCTGGGTCCTCGGTGAAGGTCTCCTGCAAGGCTTCTGGAGGCACCCTCAGCAGCTATGCTATCAGCTGGGTGCGACAGGCCCCTGGACAAGGGCTTGAGTGGATGGGAAGGATCATCCCTATCCTTGGTATAACAAACTACGCACAGAAGTTCCAGGGCAGAGTCACGATTACCGCGGACAAATCCACGAGCACAGCCTACATGGAGCTGAGCAGCCTGAGATCTGAGGACACGGCCGTGTATTTCTGTGCGAGAGATCAGCGGCCGGCGAGCATGGACGTCTGGGGCCAGGGCACCCTGGTCACCGTCTCGAGTGGTGGAGGCGGTTCAGGCGGAGGTGGCTCTGGCGGTGGCGCTAGCCAGTCTGAGCTGACTCAGCCTGCCTCCGTGTCTGGGTCTCCTGGACAGTCGATCACCATCTCCTGCACTGGAACCAGCAGTGACGTTGGTGGTTATATCTATGTCTCCTGGTACCAACAGCACCCAGGCAAAGTCCCCAAACTCATGATTCATGATGTCAGTCATCGGCCCTCAGGGGTTTCTAATCGCTTCTCTGGCTCCAGGTCTGGCAACACGGCCTCCCTGACCATCTCTGGGCTCCAGGCTGAGGACGAGGCTGACTATTTCTGCAGCTCATATACAAGCAGCAACAATTATGTCTTCGGAACTGGGACCAAGGTCACCGTCCTA | 21 |
| A03 | CAGGTCCAGCTGGTGCAATCTGGGGCTGAGGTGAAGAAGCCTGGGTCCTCGGTGAAGGTCTCCTGCAAGGCTTCTGGAGGCACCTTCAGCAGCTATACTATCAGCTGGGTGCGACAGGCCCCTGGACAAGAGCTTGAGTGGATGGGAAGGATCATCCCTATCCTTGGTATAGCAAACTACGCACAGAAGTTCCAGGGCAGAGTCACGATTACCGCGGACAAATCCACGAGCACAGCCTACATGGAGCTGAGCAGCCTGAGATCTGAGGACACGGCCGTGTATTACTGTGCGAGAGATTATAGCAGCAGCTGGAACTCTATGGACGTCTGGGGCCAGGGAACCCTGGTCACCGTCTCGAGTGGTGGAGGCGGTTCAGGCGGAGGTGGCTCTGGCGGTGGCGCTAGCCAGTCTGGGCTGACTCAGCCTCCCTCCGCGTCCGGGTCTCCTGGACAGTCAGTCACCATCTCCTGCACTGGAACCAGCAGTGACATTGGTTATTATCACTATGTCTCCTGGTACCAACAACACCCGGGCAAAGCCCCCAAACTCATGATTTATGAGGACAGTAAGAGGCCCTCAGGGATTTCTAATCGTTTCTCTGGCTCCAAGTCTGGCACCACGGCCTCCCTGACCGTCTCTGGGCTCCAGGCTGAGGACGAGGCTCATTATTACTGCAGTTCTTTTACAAGTAGAAGTACTTGGGTGTTCGGCGGAGGGACCCAGCTCACCGTCCTA | 22 |

(continued)

| scFv encoding nucleic acid molecule | | |
|---|---|---|
| scF v ID | scFv Region (Nucleotides; 5'→3') | SEQ ID NO |
| A04 | GAGGTGCAGCTGGTGCAGCCTGGGGCTGAGGTGAAGAAGC<br>CTGGGTCCTCGGTGAAGGTCTCCTGCAAGGCTTCTGGAGGC<br>ACCCTCAGCAGCTATGCTATCAGCTGGGTGCGACAGGCCCC<br>TGGACAAGGGCTTGAGTGGATGGGAAGGATCATCCCTATCC<br>TTGGTATAACAAACTACGCACAGAAGTTCCAGGGCAGAGTCA<br>CGATTACCGCGGACAAATCCACGAGCACAGCCTACATGGAG<br>CTGAGCAGCCTGAGATCTGAGGACACGGCCGTGTATTTCTG<br>TGCGAGAGATCAGCGGCCGGCGAGCATGGACGTCTGGGGC<br>CAGGGCACCCTGGTCACCGTCTCGAGTGGTGGAGGCGGTT<br>CAGGCGGAGGTGGCTCTGGCGGTGGCGCTAGCCAGTCTGA<br>GCTGACTCAGCCTGCCTCCGTGTCTGGGTCTCCTGGACAGT<br>CGATCACCATCTCCTGCACTGGAACCAGCAGTGACGTTGGT<br>GGTTATATCTATGTCTCCTGGTACCAACAGCACCCAGGCAAA<br>GTCCCCAAACTCATGATTCATGATGTCAGTCATCGGCCCTCA<br>GGGGTTTCTAATCGCTTCTCTGGCTCCAGGTCTGGCAACAC<br>GGCCTCCCTGACCATCTCTGGGCTCCAGGCTGAGGACGAGG<br>CTGACTATTTCTGCAGCTCATATACAAGCAGCAACAATTATGT<br>CTTCGGAACTGGGACCAAGGTCACCGTCCTA | 23 |
| A05 | CAGGTCCAGCTGGTGCAATCTGGGGCTGAGGTGAAGAAGCC<br>TGGGTCCTCGGTGAAGGTCTCCTGCAAGGCTTCTGGAGGCA<br>CCTTCAGCAGCTATACTATCACCTGGGTGCGACAGGCCCCT | 24 |
| | GGACAAGGGCTTGAGTGGATGGGAAGGATCATCCCTGTCCT<br>TGGTATAGCAAACTACGCACAGAAGTTCCAGGGCAGAGTCA<br>CGATTACCGCGGACAAATCCACGAGCACAGCCTACATGGAG<br>CTGAGCAGCCTGAGATCTGAGGACACGGCCGTGTATTACTG<br>TGCGAGAGATTATAGCAGCAGCTGGAACTCTATGGACGTCT<br>GGGGCCAGGGAACCCTGGTCACCGTCTCGAGTGGTGGAGG<br>CGGTTCAGGCGGAGGTGGCTCTGGCGGTGGCGCTAGCCAG<br>TCTGGGCTGACTCAGCCTCGCTCAGTGTCCGGGTCTCCTGG<br>ACAGTCAGTCACCATCTCCTGCACTGGAACCAGCAGTGATGT<br>TGGTGGTTATAACTATGTCTCCTGGTACCAACAGCACCCAGG<br>CAAAGCCCCCAAACTCATGATTTATGATGTCAGTAAGCGGCC<br>CTCAGGGGTCCCTGATCGCTTCTCCGGCTCCAAGTCTGGGA<br>ACACGGCCTCCCTGACCGTCTCTGGGCTCCACGCTGAGGAT<br>GAGGCTGATTATTACTGCAGCTCATATGCAGGCAGCAACAAT<br>TTTGTCTTCGGAACTGGGACCAAGGTCACCGTCCTA | |

(continued)

| scFv encoding nucleic acid molecule | | |
|---|---|---|
| scF v ID | scFv Region (Nucleotides; 5'→3') | SEQ ID NO |
| A06 | CAGGTGCAGCTGGTGCAATCTGGGGCTGAGGTGAAGAAGCC TGGGTCCTCGGTGAAGGTCTCCTGCAAGGCTTCTGGAGGCA CCTTCGGCAGCTATACTATCAGCTGGGTGCGACAGGCCCCT GGACAAGGGCTTGAGTGGATGGGAAGGATCATCCCTATCCT TGGTATAGCAAACTACGCACAGAAGTTCCAGGGCAGAGTCA CGATTACCGCGGACAAATCCACGAGCACAGCCTACATGGAG CTGAGCAGCCTGAGATCTGAGGACACGGCCGTGTATTACTG TGCGAGAGATTATAGCAGCAGCTGGAACTCTATGGACGTCT GGGGCCAAGGAACCCTGGTCACCGTCTCGAGTGGTGGAGG CGGTTCAGGCGGAGGTGGCTCTGGCGGTGGCGCTAGCCAG TCTGGGCTGACTCAGCCTCGCTCAGTGTCCGGGTCTCCTGG ACAGTCAGTCACCATCTCCTGCACTGGAACCAGCAGTGATGT TGGTGCTTATAACTATGTCTCCTGGTACCAACAGCACCCAGG CAAAGCCCCCAAACTCATGATTTATGAGGTCAGTAAGCGGCC CTCAGGGGTCCCTGATCGCTTCTCTGCCTCCAAGTCTGGCA ACACGGCCTCCCTGACCGTCTCTGGGCTCCAGGCTGAGGAT GAGGCTGATTATTACTGCAGCTCATATGCAGGCAGCAACAAT TGGGTGTTCGGCGGAGGGACCAAGCTGACCGTCCTA | 25 |
| A07 | CAGGTGCAGCTGCAGGAGTCGGGCCCAGGGCTTGTGAAGC CTTCGGAGACCCTGTCCCTCACTTGCACTGTCTCTGGTGGCT CCATCAGTAGTTACTACTGGAGCTGGATCCGGCAGCCCCCA GGGAAGGGACTGGAGTGGATTGGGTCTATCTATTACAGTGG GAGCACCAACTACAACCCCTCCCTCAAGAGTCGAGTCACCAT CTCAAGAGACAAGTCGAAGAACCAGTTGTTTCTGAAGTTGAA TTCTATGACCGCCGCGGACACGGCCGTCTATTATTGTGCGA GAGATGTTTGGGGCAGTGGCCAGTCATTTGACAGTTGGGGC CAGGGCACCCTGGTCACCGTCTCGAGTGGTGGAGGCGGTT CAGGCGGAGGTGGCTCTGGCGGTGGCGCTAGCAATTTTATG CTGACTCAGCCCCACTCTGTGTCGGAGTCTCCGGGGAAGAC GGTAACCATCTCCTGCACCCGCAGCAGTGGCAGCATTGCCA GCAACTATGTGCAGTGGTACCAGCAGCGCTTGGGCAGTTCC CCCACCACTGTGATCTATGAACATAGCCGAAGACCCTCTGG GGTCCCTGATCGGTTCTCTGCCTCCATCGACAGCTCCTCCAA CTCTGCCTCCCTCACCATCTCTGGACTGAAGACTGAGGACG AGGCTGACTACTACTGTCAGTCTTATGATGTCAGCAATCGAG TGTTCGGCGGAGGGACCAAGCTGACCGTCCTA | 26 |

(continued)

| scFv encoding nucleic acid molecule | | |
|---|---|---|
| **scF v ID** | **scFv Region (Nucleotides; 5'→3')** | **SEQ ID NO** |
| A08 | GAGGTGCAGCTGTTGGAGTCTGGGGGAGGCTTGGTCCAGCCTGGGGGGTCCCTGAGACTCTCCTGTGCAGCCTCTGGATTCACCTTTAGTAGCTATTGGATGAGCTGGGTCCGCCAGGCTCCAGGGAAGGGGCTGGAGTGGGTGGCCAACATAAAGCAAGATGGAAGTGAGAAATACTATGTGGACTCTGTGAAGGGCCGATTCACCATCTCCAGAGACAACGCCAAGAACTCGCTGTATCTGCAAATGAACAGCCTGAGAGCTGAGGACACGGCTGTGTATTATTGTGCGAAAGCCCCGTGGTATAGCAGCTCGCCGACACCCTACGGTATGGACGTCTGGGGCCAGGGCACCCTGGTCACCGTCTCGAGTGGTGGAGGCGGTTCAGGCGGAGGTGGCTCTGGCGGTGGCGCTAGCGACATCCAGATGACCCAGTCTCCATCCTCCCTGTCTGCATCTGTAGGAGACAGAGTCACCATCACTTGCCAGGCGAGTCAGGACATTAGCAACTATTTAAATTGGTATCAGCAGAAACCAGGGAAAGCCCCTAGGCGCCTGATCTATGGTGCATCCACTTTGATGAGTGGGGTCCCATCAAGGTTCAGCGGCAGTGGATCTGGGACAGATTTCACTCTCACCATCAGCAGTCTGCAACCTGAAGATTTTGCAACTTACTACTGTCAACAGAGTTACAGTACACCTCTCACTTTCGGCGGAGGGACCAAGGTGGAAATCAAA | 27 |
| A10 | GAGGTGCAGCTGGTGCAGTCTGGGGCTGAGGTGAAGAAGCCTGGGTCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGAGGGATCATCCCTATCTTTGGTACAGCAAACTACGCACAGAAGTTCCAGGGCAGAGTCACGATTACCGCGGACGAATCCACGAGCACAGCCTACATGGAGCTGAGCAGCCTGAGATCTGAGGACACGGCCGTGTATTACTGTGCGAGAACACGTTACATTTGGGGGAGTTATCGGGCATACGGTATGGACGTCTGGGGCCAAGGGACAATGGTCACCGTCTCGAGTGGTGGAGGCGGTTCAGGCGGAGGTGGCTCTGGCGGTGGCGCTAGCGACATCCAGATGACCCAGTCTCCATCCTCCATGTCTGCATCTGTAGGAGACAGAGTCACCATCACTTGCCGCGCGAGTCAGAGCATTAGCAGTCATTTAAATTGGTATCAGCAGCTGCCAGGCAATGCCCCTACTCTCCTGATCTATTATGCTTCCAATTTACAAAGTGGGGTCCCATCTAGGTTCAGTGGCAGTGGAT | 28 |
| | CTGGGACAGATTTCACTCTCACCATCAGCAGTCTGCAGCCTGATGATTTTGCAACTTACTACTGTCAACAGAGTATCAGTCTCCCGTACACTTTTGGCCAGGGGACCAAGGTGGAGATCAAA | |

(continued)

| scFv encoding nucleic acid molecule | | |
|---|---|---|
| scF v ID | scFv Region (Nucleotides; 5'→3') | SEQ ID NO |
| A12 | GAGGTGCAGCTGGTGCAGCCTGGGGCTGAGGTGAAGAAGC CTGGGTCCTCGGTGAAGGTCTCCTGCAAGGCTTCTGGAGGC ACCCTCAGCAGCTATGCTATCAGCTGGGTGCGACAGGCCCC TGGACAAGGGCTTGAGTGGATGGGAAGGATCATCCCTATCC TTGGTATAACAAACTACGCACAGAAGTTCCAGGGCAGAGTCA CGATTACCGCGGACAAATCCACGAGCACAGCCTACATGGAG CTGAGCAGCCTGAGATCTGAGGACACGGCCGTGTATTTCTG TGCGAGAGATCAGCGGCCGGCGAGCATGGACGTCTGGGGC CAGGGCACCCTGGTCACCGTCTCGAGTGGTGGAGGCGGTT CAGGCGGAGGTGGCTCTGGCGGTGGCGCTAGCCAGTCTGA GCTGACTCAGCCTGCCTCCGTGTCTGGGTCTCCTGGACAGT CGATCACCATCTCCTGCACTGGAACCAGCAGTGACGTTGGT GGTTATATCTATGTCTCCTGGTACCAACAGCACCCAGGCAAA GTCCCCAAACTCATGATTCATGATGTCAGTCATCGGCCCTCA GGGGTTTCTAATCGCTTCTCTGGCTCCAGGTCTGGCAACAC GGCCTCCCTGACCATCTCTGGGCTCCAGGCTGAGGACGAGG CTGACTATTTCTGCAGCTCATATACAAGCAGCAACAATTATGT CTTCGGAACTGGGACCAAGGTCACCGTCCTA | 29 |
| B01 | GAGGTGCAGCTGGTGCAGCCTGGGGCTGAGGTGAAGAAGC CTGGGTCCTCGGTGAAGGTCTCCTGCAAGGCTTCTGGAGGC ACCCTCAGCAGCTATGCTATCAGCTGGGTGCGACAGGCCCC TGGACAAGGGCTTGAGTGGATGGGAAGGATCATCCCTATCC TTGGTATAACAAACTACGCACAGAAGTTCCAGGGCAGAGTCA CGATTACCGCGGACAAATCCACGAGCACAGCCTACATGGAG CTGAGCAGCCTGAGATCTGAGGACACGGCCGTGTATTTCTG TGCGAGAGATCAGCGGCCGGCGAGCATGGACGTCTGGGGC CAGGGCACCCTGGTCACCGTCTCGAGTGGTGGAGGCGGTT CAGGCGGAGGTGGCTCTGGCGGTGGCGCTAGCCAGTCTGG GCTGACTCAGCCTGCCTCCGTGTCTGGGTCTCCTGGACAGT CGATCACCATCTCCTGCACTGGAACCAGCAGTGATGTTGGT GGTTATAACTATGTCTCCTGGTACCAACAGCACCCAGGCAAA GCCCCCAAACTCATGATTTATGAGGTCAGTAATCGGCCCTCA GGGGTTTCTAATCGCTTCTCTGGCTCCAAGTCTGGCAACACG GCCTCCCTGACCATCTCTGGGCTCCAGGCTGAGGACGAGGC TGATTACTACTGCAGCACAGTTACAAGCCTCAGCACTTATGT CTTCGGAACTGGGACCAAGCTGACCGTCCTA | 30 |

(continued)

| scFv encoding nucleic acid molecule | | |
|---|---|---|
| **scF v ID** | **scFv Region (Nucleotides; 5'→3')** | **SEQ ID NO** |
| B07 | GAGGTGCAGCTGGTGCAGTCTGGGGCTGAGGTGAAGAGGCCTGGGTCCTCGGTGAAGGTCTCCTGCAAGGCATCTGGATACACCTTCACCAGCTACTATATGCACTGGGTGCGACAGGCCCCTGGACAAGGGCTTGAGTGGATGGGAATAATCAACCCTAGTGGTGGTAGCACAAGCTACGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGGCAGGAACGTATAGCAGTGGCTGGACGATTGACTACTGGGGGCAAGGGACCACGGTCACCGTCTCGAGTGGTGGAGGCGGTTCAGGCGGAGGTGGCTCTGGCGGTGGCGCTAGCGATATTGTGATGACGCAGTCTCCACTCTCCCTGCCCGTCACCCTTGGACAGCCGGCCTCCATCTCCTGCAGGTCTAGTCAAAGCCTCGTATACACTGATGGAAACACCTACTTGAATTGGTTTCAGCAGAGGCCAGGCCAATCTCCAAGGCGCCTAATTTATAAGGTTTCTAACCGGGACTCTGGGGTCCCAGACAGATTCAGCGGCAGTGGGTCAGGCACTGATTTCACACTGAAAATCAGCAGGGTGGAGGCTGAGGATGTTGGGATTTATTACTGCATGCATAGTAAACAGTGGCCTCCCACTTTCGGCGGAGGGACCAAGGTGGAAATCAAA | 31 |
| B08 | GAGGTGCAGCTGGTGCAGTCTGGGGCTGAGGTGAAGAAGCCTGGGTCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGAGGGATCATCCCTATCTTTGGTACAGCAAACTACGCACAGAAGTTCCAGGGCAGAGTCACGATTACCGCGGACAAATCCACGAGCACAGCCTACATGGAGCTGAGCAGCCTGAGATCTGAGGACACGGCCGTGTATTACTGTGCGAGAGGACACTACTACTACATGGACGTCTGGGGGCAAGGGACCACGGTCACCGTCTCGAGTGGTGGAGGCGGTTCAGGCGGAGGTGGCTCTGGCGGTGGCGCTAGCGATATTGTGATGACTCAGTCTCCAGCCACCCTGTCTGTGTCTCCAGGGGAAGGAGCCACCCTCTCCTGCAGGGCCAGTCAGAGTGTTAGCAGCAACTTAGCCTGGTATCAGCAGAAACCTGGCCGGGCTCCCAGGCTCCTCATCTATGGTGCATCCACCAGGGCCACTGGTATCCCAGCCAGGTTCAGTGGCAGTGGGTCTGGGACAGAGTTCACTCTCACCATCAGCAGCCTGCAGTCTGAAGATTTTGCAGTTTATTACTGTCAGCAGTATAATAACTGGCCCACTTTCGGCGGAGGGACCAAGCTGGAGATCAAA | 32 |

(continued)

| scFv encoding nucleic acid molecule | | |
|---|---|---|
| **scF v ID** | **scFv Region (Nucleotides; 5'→3')** | **SEQ ID NO** |
| B10 | GAGGTGCAGCTGGTGCAGTCTGGGGCTGAGGTGAAGAAGC<br>CTGGGTCCTCGGTGAAGGTCTCCTGCAAGGCTTCTGGAGGC<br>ACCTTCAGCAGCTATGCTATCAGCTGGGTGCGACAGGCCCC<br>TGGACAAGGGCTTGAGTGGATGGGAGGGATCATCCCTATCT<br>TTGGTACAGCAAACTACGCACAGAAGTTCCAGGGCAGAGTC<br>ACGATTACCGCGGACAAATCCACGAGCACAGCCTACATGGA<br>GCTGAGCAGCCTGAGATCTGAGGACACGGCCGTGTATTACT<br>GTGCGAGAGGACACTACTACTACATGGACGTCTGGGGCAA<br>GGGACCACGGTCACCGTCTCGAGTGGTGGAGGCGGTTCAG<br>GCGGAGGTGGCTCTGGCGGTGGCGCTAGCGATATTGTGATG<br>ACTCAGTCTCCAGCCACCCTGTCTGTGTCTCCAGGGGAAGG<br>AGCCACCCTCTCCTGCAGGGCCAGTCAGAGTGTTAGCAGCA<br>ACTTAGCCTGGTATCAGCAGAAACCTGGCCGGGCTCCCAGG<br>CTCCTCATCTATGGTGCATCCACCAGGGCCACTGGTATCCCA<br>GCCAGGTTCAGTGGCAGTGGGTCTGGGACAGAGTTCACTCT<br>CACCATCAGCAGCCTGCAGTCTGAAGATTTTGCAGTTTATTA<br>CTGTCAGCAGTATAATAACTGGCCCACTTTCGGCGGAGGGA<br>CCAAGCTGGAGATCAAA | 33 |
| B11 | CAGGTGCAGCTGGTGGAGTCTGGGGCTGAGGTGAAGAAGC<br>CTGGGTCCTCGGTGAAGGTCTCCTGCAAGGCTTCTGGAGGC<br>ACCTTCAGCAGCTATGCTATCAGCTGGGTGCGACAGGCCCC<br>TGGACAAGGACTTGAGTGGATGGGAGGGATCATCCCTATCT<br>TTGGTACAGCAAACTACGCACAGAAGTTCCAGGGCAGAGTC<br>ACGATTACCGCGGACGAATCCACGAGCACAGCCTACATGGA<br>GCTGAGCGGCCTGAGATCTGAGGACACGGCCGTGTATTACT<br>GTGCGAGAGGTATACAGCCTCTTCGCTACTACGGTATGGAC<br>GTCTGGGGCCAAGGAACCCTGGTCACCGTCTCGAGTGGTGG<br>AGGCGGTTCAGGCGGAGGTGGCTCTGGCGGTGGCGCTAGC<br>CAGTCTGCGCTGACTCAGCCACCCTCAGCGTCTGGGACCCC<br>CGGGCAGAGGGTCACCATCTCTTGTTCTGGAAGCAGCTCCA<br>ACATCGGAAGTAATTATGTATACTGGTACCAGCAGCTCCCAG<br>GAACGGCCCCCAAACTCCTCATCTATAGGAATAATCAGCGGC<br>CCTCAGGGGTCCCTGACCGATTCTCTGGCTCCAAGTCTGGC<br>ACCTCAGCCTCCCTGGCCATCAGTGGGCTCCGGTCCGAGGA<br>TGAGGCTGATTATTACTGTGCAGCATGGGATGACAGCCTGA<br>GTGGTCGGGGAGTGTTCGGCGGAGGGACCCAGCTCACCGT<br>CCTA | 34 |

(continued)

| scFv encoding nucleic acid molecule | | |
|---|---|---|
| **scF v ID** | **scFv Region (Nucleotides; 5'→3')** | **SEQ ID NO** |
| B12 | CAGGTGCAGCTGGTGGAGTCTGGGGGAGGCCTGGTCAAGCCTGGGGGGTCCCTGAGACTCTCCTGTGCAGCCTCTGGATTCACCTTCAGTAGCTATAGCATGAACTGGGTCCGCCAGGCTCCAGGGAAGGGGCTGGAGTGGGTCTCAGTTATTTATAGCGGTGGTAGCACACACTACGCAGACTCCGTGAAGGGCCGATTCACCATCTCCAGACACAATTCCAAGAACACGCTGTATCTTCAAATGAACAGCCTGAGAGCTGAGGACACGGCCGTGTATTACTGTGCGAGGGGGGCTGGTACCTTAAATGCTTTTGATATCTGGGGGCAAGGGACCACGGTCACCGTCTCGAGTGGTGGAGGCGGTTCAGGCGGAGGTGGCTCTGGCGGTGGCGCTAGCCAGTCTGGGCTGACTCAGCCTCCCTCCACGTCCGGGTCTCCTGGACAGTCAGTCACCATCTCCTGCACTGGAACCAGCAGTGACGTTGGTGCTTATAGCTATGTCTCCTGGTATCAACAACACCCAGGCAAAGCCCCCAAACTTCTCATTTATGCGGTCACTAAGAGGCCCTCGGGGGTCCCTGATCGCTTCTCTGGCTCCAAGTCTGGCAACACGGCCTCCCTGACCGTCTCTGGACTCCAGGATGAGGATGAGGCTGATTATTACTGCAGCTCTTTTGCAGGCGGCAGCACTCTGGTGTTCGGCGGAGGGACCAAGCTGACCGTCCTA | 35 |
| C01 | CAAATGCAGCTGGTGCAGTCTGGGGCTGAGGTGAAGAAGCCTGGGTCCTCGGTGAAGGTCTCCTGCAAGGCTTCTGGAGGCACCTTCAGCAGCTATGCTATCAGCTGGGTGCGACAGGCCCCTGGACAAGGGCTTGAGTGGATGGGAGGGATCATCCCTATCTTTGGTACAGCAAACTATGCACAGAAGTTCCAGGGCAGAGTCACGATTACCGCGGACGAATCCACGAGCACAGCCTACATGGAGCTGAGCAGCCTGAGATCTGAGGACACGGCCGTGTATTACTGTGTGAGAGGATACAGTTCAATATACTACTACTACGGTATGGACGTCTGGGGCCAAGGGACAATGGTCACCGTCTCGAGTGGTGGAGGCGGTTCAGGCGGAGGTGGCTCTGGCGGTGGCGCTAGCCAGTCTGGGCTGACTCAGCCTCGCTCAGTGTCCGGGTCTCCTGGACAGTCAGTCACCATCTCCTGCACTGGAACCAGCAGTGATGTTGGTGGTTATAACTATGTCTCCTGGTACCAACAGCACCCAGGCAAAGCCCCCAAACTCATGATTTATGATGTCAGTAAGCGGCCCTCAGGGGTTTCTGATCGCTTCTCTGGCTCCAAGTCTGGCAACACGGCCTCCCTGACCATCTCTGGGCTCCAGGCTGAGGACGAGGCTGATTATTACTGCGGCTCATATACAAGCGACGGGACTCTAGTATTCGGCGGAGGGACCAAGCTGACCGTCCTA | 36 |

(continued)

| scFv encoding nucleic acid molecule | | |
|---|---|---|
| **scF v ID** | **scFv Region (Nucleotides; 5'→3')** | **SEQ ID NO** |
| C02 | CAGGTGCAGCTGGTGCAATCTGGGGCTGAGGTGAAGAAGCC TGGGTCCTCGGTGAAGGTCTCCTGCAAGGCTTCTGGAGGCA CCTTCAGCAGCTATACTATCAGCTGGGTGCGACAGGCCCCT GGACAAGGGCTTGAGTGGATGGGAAGGATCATCCCTATCCT TGGTATAGCAAACTACGCACAGAAGTTCCAGGGCAGAGTCA CGATTACCGCGGACAAATCCACGAGCACAGCCTACATGGAG CTGAGCAGCCTGAGATCTGAGGACACGGCCGTGTATTACTG TGCGAGAGATAGGTCTTACAACTGGCTCGACCCCTGGGGCC GTGGCACCCTGGTCACCGTCTCGAGTGGTGGAGGCGGTTCA GGCGGAGGTGGCTCTGGCGGTGGCGCTAGCCAGTCTGCGC TGACTCAGCCTGTCTCCGTGTCTGGGTCTCCTGGACAGTCG ATCACCATCTCCTGCACTGGAACCATCAGTGACGTTGGTGAT TATAACTATGTCTCCTGGTACCAACAGCACCCAGGCAAAGCC CCCAAACTCATGATTTATGACGTCAATAATCGGCCCTCAGGG GTTTCTAATCGCTTCTCTGGCTCCAAGTCTGGCAACACGGCC TCCCTGACCATCTCTGGGCTCCAGGCTGAGGACGAGGCTGA TTATTACTGCAGCTCATATACAAGCAGCAGCACTCTGGTATT CGGCGGAGGGACCAAGCTGACCGTCCTA | 37 |

**Example 2: Binding affinity to GUCY2C of selected scFv**

The binding affinity to the antigen GUCY2C of the scFv produced in Example 1 was measured by ELISA. As an antigen for measuring the binding affinity, together with the human GUCY2C recombinant antigen prepared in Example 1, using a monkey GUCY2C recombinant antigen and a murine GUCY2C recombinant antigen prepared by the same method, the affinity to each antigen was measured (See FIG. 1a).

More specifically, GUCY2C-rCD4 fusion protein, in which the prepared human GUCY2C (SEQ ID NO: 112), monkey GUCY2C (SEQ ID NO: 114), and mouse GUCY2C (SEQ ID NO: 113) were fused, respectively, was bound to a place coated with streptavidin in a Nunc MaxisorpTM 96 well plate. After removing all the synthetic proteins not combined with washing buffer, the anti-GUCY2C scFV obtained in the culture solution was incubated in each well. After that, the affinity was measured by reading signals DELFIA enhanced using Eu-anti-tag Ab.

The information of the used antigen is as follows:

| | Organism | Uniprot ID | NCBI Gene ID | NCBI Reference Sequence |
|---|---|---|---|---|
| Hu-GUCY2C | Homo sapiens (human) | P25092 | 2984 | NP_004954.2 |
| Mu-GUCY2C | Mus musculus (house mouse) | Q3UWA6 | 14917 | NP_001120790.1 |
| Cy-GUCY2C | Macaca fascicularis (crab-eating macaque) | G7PJX5 | 102130850 | XP_005570270.1 |
| rCD4 | Rattus norvegicus | P05540 | 24932 | NP_036837.1 |

In addition, the 18 kinds of scFvs produced in Example 1 were prepared at two concentrations (5nM, 50nM), and they were used as a primary antibody of ELISA, respectively, to detect the GUCY2C antigen, and thereby, the intensity of the binding affinity between each clone was measured, and using this, ranking was secured. More specifically, after coating an anti-FLAG antibody in a MaxiSorb plate and binding by adding scFvs obtained previously, the remaining scFvs were removed by a washing process. Here, a biotinylated human-GUCY2C-rCD4 protein was incubated and combined, and then the affinity to the antigen was measured by reading signals by DELFIA enhancement with color development through streptavidin-Europlum.

Furthermore, the affinity to the GUCY2C antigen of the 18 kinds of scFvs produced in Example 1 was measured by SPR (Surface plasmon resonance) analysis. More specifically,

protein-G was attached to an HCA chip at a concentration of 150ug/ml, and then 5 mM scFvs and the antigen were flowed at various concentrations from 800 nM to 12.5 nM at a rate of 40ul/sec. SPR assay was performed with association time 2 min and dissociation time 10 min under the condition of 25 degrees of Celcius using MASS2 (Sierra SPR-32; Bruker). The data were analyzed with Software R3.

The binding affinity to 3 kinds of antigens (human GUCY2C, monkey GUCY2C, and mouse GUCY2C) of the 18 kinds of scFvs (SEQ ID NOs: 1 to 18) and positive control group, scFv (5F9 scFv-Fc; SEQ ID NO: 19) measured as above and affinity ranking result were shown in Table 6 and FIGs. 1b (binding affinity, ELISA), 2b (affinity ranking, 5nM) and 2c (affinity ranking, 50nM):

[Table 6]

| scFv ID | Clone ID | Binding ELISA (Hu-GUCY2C -rCD4) | Binding ELISA (Cy-GUCY2C -rCD4) | Binding ELISA (Mu-GUCY2C -rCD4) | Affinity Ranking ELISA (5 nM) | Affinity Ranking ELISA (50 nM) | Affinity (SPR) |
|---|---|---|---|---|---|---|---|
| A01 | 2426_01_A02 | 30194 | 28056 | 352 | 13814 | 104387 | n.d. |
| A02 | 2427_01_A08 | 48333 | 60 | 74 | 33437 | 167867 | 743 nM |
| A03 | 2427_01_A12 | 59173 | 7871 | 77 | 36021 | 199194 | 219 nM |
| A04 | 2427_01_B02 | 50995 | 73 | 66 | 30425 | 173460 | 311 nM |
| A05 | 2427_01_B07 | 43016 | 89 | 62 | 18286 | 131086 | 4394 nM |
| A06 | 2427_01_C01 | 63094 | 1527 | 80 | 35080 | 185082 | 716 nM |
| A07 | 2427_01_C02 | 79025 | 51086 | 17087 | 4741 | 27729 | 3731 nM |
| A08 | 2432_01_D05 | 58055 | 2273 | 1276 | 8697 | 54750 | n.d. |
| A10 | 2432_01_D08 | 55377 | 37187 | 82 | 81236 | 242966 | 48 nM |
| A12 | 2433_01_G08 | 38220 | 56 | 53 | 28096 | 151338 | 297 nM |
| B01 | 2433_01_H07 | 39417 | 4486 | 70 | 26219 | 133243 | 335 nM |

(continued)

| scFv ID | Clone ID | Binding ELISA (Hu-GUCY2C -rCD4) | Binding ELISA (Cy-GUCY2C -rCD4) | Binding ELISA (Mu-GUCY2C -rCD4) | Affinity Ranking ELISA (5 nM) | Affinity Ranking ELISA (50 nM) | Affinity (SPR) |
|---|---|---|---|---|---|---|---|
| B07 | 2436_02_F10 | 68628 | 48862 | 66 | 25925 | 139097 | 293 nM |
| B08 | 2436_02_F11 | 61079 | 33693 | 56 | 23307 | 152983 | n.d. |
| B10 | 2436_02_G01 | 34355 | 21161 | 82 | 12521 | 104751 | 2856 nM |
| B11 | 2437_02_G07 | 37532 | 23563 | 44 | 14849 | 89646 | 1329 nM |
| B12 | 2437_02_G10 | 30920 | 22095 | 48 | 9592 | 77088 | 4539 nM |
| C01 | 2437_02_H05 | 37474 | 21616 | 51 | 3090 | 29476 | 1148 nM |
| C02 | 2437_02_H08 | 49474 | 32344 | 61 | 11013 | 86347 | 1481 nM |
| C07 (positive control) | 5F9 scFv | 83913 | 49221 | 41 | 133261 | 183619 | 15 nM |
| (n.d.: not detected) | | | | | | | |

As shown in Table 6 and FIG. 1b, it was shown that the binding affinity to human GUCY2C of all the 18 kinds of scFvs was significantly higher than the binding affinity to the monkey GUCY2C and mouse GUCY2C, and it was confirmed that it specifically bound to human GUCY2C. In addition, as shown in Table 6 and FIGs. 2b and 2c, it was confirmed that there was affinity to monkey GUCY2C in the scFv with relatively high affinity to human GUCY2C. When the SPR results were determined overall, it was confirmed that the scFV of A10 had relatively high binding ability and had affinity to human and monkey GUCY2C.

**Example 3: Cell binding assay**

Using cells naturally expressing GUCY2C, whether 18 kinds of scFvs produced in Example 1 could actually detect GUCY2C expression on the cell surface was confirmed. For this, a GUCY2C positive cancer cell, T84 colon carcinoma cell (ATCC® CCL-248™) was used. As a control group, GUCY2C negative breast cancer cell T-47D (ATCC® HTB-133™) was used.

After separating the two cell lines using trypsin-EDTA, they were placed in FACS buffer (1x PBS + 2 % BSA). 10 ug/mL of each of the 18 kinds of scFvs and positive control group, 5F9 scFv was cultured with T84 and T-47D cells on ice for 1 hour. The scFv combined to cells was detected using 5 ug/mL IgG-Fc-PE Ab (BioLegend; 409304). The cells were cultured with ToPro®3 (live/dead stain) and then red with a flow cytometer. The obtained data were floated using FlowJo 10.5 software (FlowJo LLC).

The obtained result was shown in FIG. 3a.

In addition, the MFI (mean of fluorescence intensity) obtained by the flow cytometry was shown in FIG. 3b.

As shown in FIGs. 3a and 3b, it was confirmed that A10 scFv (SEQ ID NO: 9) bound to GUCY2C expressing T84 cells at the equal level to the positive control group, 5F9, and B01 scFv (SEQ ID NO: 11), B11 scFv (SEQ ID NO: 15), and B12 scFv (SEQ ID NO: 16) also showed the binding possibility.

### Example 4: Preparation of immunocytes expressing anti-GUCY2C-CAR (NK cells)

#### 4.1. Lentivirus production

CAR-NK cells are a form in which a chimeric antigen receptor (CAR) is expressed on the NK cell surface, and the chimeric antigen receptor used in the present example is composed of an extracellular domain comprising a scFv polypeptide binding to GUCY2C (See Example 1 and Table 4), a transmembrane domain (CD28; encoded by GenBank Accession no. NM_006139.4), and an intracellular signaling domain (CD3zeta; encoded by GenBank Accession no. NM_001378516.1) (anti-GUCY2C-CAR). CAR gene was introduced into NK by Lenti virus.

In order to generate Lenti virus expressing CAR, Vrial plasmid transfection was conducted by treating LentiX-293T (#632180 Clonthech) with Lipofectamine 3000 transfection kit (#L3000015, Invitrogen) and a plasmid expressing anti-GUCY2C CAR (GUCY2C binding scFv-CD28-CD3zeta), and the supernatant was obtained within 2 days from the next day after transfection. A virus was concentrated using Lenti-X concentrator (#631232, Clonetech). The obtained precipitate was dissolved in CTS-PBS and stored at -80°C.

After extracting RNA from the virus using a viral RNA isolation kit (#740956, Macherey-Nagel), the virus titer was measured using Lenti-X qRT-PCR titration kit (#631235, Takara).

#### Example 4.2. Preparation of NK cells

iPSC (CMC-hiPSC-003, Korea Centers for Disease Control and Prevention) was cultured in mTeSR™ Plus (STEMCELL Technoology, 100-0276) for 2-3 days, and when aggregates with a diameter of about 500,um were formed, the culture solution was changed into Medium A of STEMdiff™ Hematopoietic Kit (STEMCELL Technoology, 05310), thereby occurring hematopoietic stem cell (HSC) differentiation. After culturing in Medium A for 3 days, they were additionally cultured in Medium B of the same kit for 9 days to obtain HSCs. Then, half of the culture solution was removed every 2-3 days, and the same amount of new culture solution was added to replace the culture solution.

The HSCs were transferred to a plate surface-treated with Lymphoid Differentiation Coating Material comprised in StemSpan™ NK Cell Generation Kit (STEMCELL Technoology, 09960) and cultured in Lymphoid Progenitor Expansion Medium comprised in the same kit product for 14 days, and the culture solution was replaced by half every 3-4 days. Then, in 14 days after culturing by replacing the culture solution by half in NK Cell Differentiation Medium every 3-4 days, NK cells were obtained.

#### Example 4.3. Confirmation of surface type characteristics of naive NK cells differentiated in iPSC

In order to confirm whether differentiation of the naive NK cells differentiated in iPSC obtained in Example 4.2 was normally performed and they had surface type traits and NK cell intrinsic function (perforin, granzyme B, IFN), expression of the NK cell surface marker was confirmed by flow cytometry and expression of an effector molecule of the apoptosis process of NK cells was confirmed.

Specifically, the naive NK cells differentiated in iPSC obtained in Example 4.2 were incubated with each antibody represented in FIG. 4a to FIG. 4b at 4°C for 1 hour and then they were confirmed with a flow cytometer (FACS). In addition, in order to confirm cytokine expressing inside the cells, a hole was made in the cell surface using a permeabilization kit and fixing was conducted using a fixing solution, and then they were dyed using antibodies (TNF-$\alpha$, IFN-$\gamma$, perforin, Granzyme B) represented in FIG. 4c, and they were analyzed with a flow cytometer.

The obtained result was shown in FIGs. 4a to 4c. As shown in FIGs. 4a and 4b, the NK cells expressing anti-GUCY2C CAR showed low CD117 expression and high CD94/NKG2D expression and showed a maturation phenotype of NK cells, and it was confirmed that expression of an NK activating receptor was high and a cytokine receptor was normally expressed. In addition, as shown in FIG. 4c, it was confirmed that expression of IFN-$\gamma$, granzyme B and perforin, which were major factors in the apoptosis process of NK cells was high in the NK cells expressing anti-GUCY2C CAR.

#### Example 4.4. Production of NK cells expressing anti-GUCY2C CAR

In the process of obtaining NK cells from iPSc as above, in an intermediate, HSC step, a lentivirus in which the anti-GUCY2C CAR (GUCY2C binding scFv-CD28-CD3zeta) prepared in Example 4.1 was loaded was treated with lentiboost (Sirion) by MOI 3000, and on the next day, with the NK cells, differentiation was progressed by the described method.

In order to confirm expression of CAR including clone ID A12 (SEQ ID NO: 3), D08 (SEQ ID NO: 9), H07 (SEQ ID NO: 11), G07 (SEQ ID NO: 15), or 5F9 (SEQ ID NO: 19; positive control) as GUCY2C binding scFV in the differentiated NK cells, a goat antihuman Fab antibody (#31628, Invitrogn) in which FITC was conjugated was used by 1:100, and it was incubated at 4 degrees of Celsius for 20 minutes. After that, it was washed with FACS buffer (2% FBS/PBS) and the expression level was confirmed by FACS analysis (LSR fortessa, BD).

The obtained result was shown in FIG. 5. As shown in FIG. 5, it was confirmed that all the differentiated NK cells expressed the GUCY2C binding scFV (D08, G07, A12, H07).

**Example 4.5. In vitro cytotoxicity test of anti-GUCY2C-CAR expressing NK cells**

The GUCY2C gene (NM_004963.4) was subcloned in pLV-EF1α-puroR plasmid, and a lentivirus was produced by referring to the method described in Example 4.1. The prepared lentivirus was transduced into HT29 cells (ATCC HTB38™) to express it on the cell surface, and after 2 days, only the cells expressing GUCY2C were selected by treating puromycin at a concentration of 2.5ug/ml. After 2 weeks of selection as such, the HT29 cell line expressing GUCY2C was finally obtained. The target cells prepared as such (HT29 (ATCC HTB38™) or HT29-GUCY2C) were labelled using CFSE proliferation kit (Thermo Fisher, C34554).

After washing by using PBS, an effector cell (anti-GUCY2C-CAR expressing NK cell) of each ratio was aliquoted in a 96well plate so as to be 100ul/well. Each target cell was fixed and the number of NK cells was adjusted according to the E (effector cells):T (target cells) ratio, thereby preparing so that the E:T ratio was 10:1, 3:1, 1:1, 0.5:1. Then, a sample having only target cells and a well having only effector cells were prepared for a negative control. These mixed cells were cultured in a 37°C incubator for 4 hours, and then washed with washing buffer for FACS (10% FBS/PBS) and then, finally, a sample was prepared with DAPI-FACS buffer (DAPI final working 5ug/ml) 70ul/well and then in 10 minutes, it was analyzed by FACS (Intellicyt® iQue Screener PLUS). Using the analyzed value, by the following equation, lysis activity was converted.

$$\% \text{ Cytotoxicity} = \frac{CFSE+DAPI+cell\ (dead\ target\ cell)}{CFSE\ positive\ cell\ (total\ target\ cell)}\ X\ 100\ (\%)$$

The obtained result was shown in FIG. 6a (result in case that the target cell was HT29 cell) and 6b (result in case that the target cell was GUCY2C expressing HT29 cell). As shown in FIGs. 6a and 6b, the cytotoxicity of NK cells expressing CAR including 4 kinds of GUCY2C binding scFVs (D08, G07, A12, H07) (CAR-NK) was confirmed. It was confirmed that when the target cell was a colorectal cancer cell line HT29 which did not express GUCY2C, CAR-NK showed the cytotoxicity of about 20% or less, while it showed the cytotoxicity of 60% or more at maximum in the GUCY2C overexpressing HT29-GUCY2C target cell line. It was confirmed that there was difference in the value of cytotoxicity depending on the type of scFv, but all the used clones showed dose-dependent cytotoxicity against GUCY2C.

**4.6 Confirmation of CAR dependent killing effect and NK intrinsic killing (CAR-independent) effect of anti-GUCY2C-CAR expressing NK cells in vitro**

In order to confirm the CAR dependent killing effect in vitro of anti-GUCY2C-CAR expressing NK cells, the following experiment was performed by using NK cells expressing CAR including a GUCY2C binding scFV (D08).

Specifically, by the same method as the method described in Example 4.4, target cells (HT29 (ATCC HTB38™) or HT29-GUCY2C) were secured, and they were analyzed by FACS (Intellicyt® iQue Screener PLUS). Using the analyzed value, by the following equation, lysis activity was converted.

$$\% \text{ Cytotoxicity} = \frac{CFSE+DAPI+cell\ (dead\ target\ cell)}{CFSE\ positive\ cell\ (total\ target\ cell)}\ X\ 100\ (\%)$$

Differentiation was conducted by making aggregates and seeding a certain amount and then securing hematopoietic stem cells by hematopoietic stem cell differentiation media and differentiating them into lymphoid progenitors. Cells were obtained through the process of differentiation into NK cells again.

The obtained result was shown in FIG. 7 (Clone No. D08 of GUCY2C targeting CAR-NK cells). As shown in FIG. 7, the CAR dependent killing effect and NK intrinsic killing effect (CAR-independent) were confirmed, when NK cells expressing CAR including the GUCY2C binding scFV (D08) (GUCY2C targeting CAR-NK cells) were co-cultured with the target cell line in vitro. In other words, it was confirmed that they had the CAR concentration-dependent killing effect, not random, by the result that the cytotoxicity was increased as the E:T ratio was increased (the effector cells were increased) in the GUCY2C-HT29 cells of FIG. 7, and it was confirmed that there was also the NK intrinsic killing effect (CAR-independent) by showing the cytotoxicity even in mock-HT29 cells.

### Example 4.7. Confirmation of CAR-dependent killing effect and NK intrinsic killing (CAR-independent) of anti-GUCY2C-CAR expressing NK cells in vivo

In order to confirm the CAR-dependent killing effect and NK intrinsic killing (CAR-independent) effect of NK expressing anti-GUCY2C-CAR in vivo, the following experiment was performed by using NK cells expressing CAR including a GUCY2C binding scFV (D08).

Specifically, in 1 hour after a CFSE-stained target cell line (HT29-GUCY2 cell) was intraperitoneally injected into mice, NK cells expressing CAR including the GUCY2C binding scFV (D08) were intraperitoneally injected with IL-2/15 to confirm the degree of reduction of the target cells in the mouse body. To observe this, the remaining cells in the peritoneal cavity were obtained by the method of waiting for 4 hours and injecting PBS into the peritoneal cavity and recovering it again, and the death of the target cells was observed by the method of measuring CFSE dye through flow cytometry.

AS a result, as shown in FIG. 8, the CAR dependent killing effect and NK intrinsic killing effect (CAR-independent) of NK cells expressing CAR including the GUCY2C binding scFV (D08) in vivo were confirmed.

### Example 4.8. Confirmation of IFN-γ secretion ability of NK cells expressing anti-GUCY2C-CAR

In order to confirm the IFN-γ secretion ability affecting the killing ability of NK cells expressing anti-GUCY2C-CAR, the following experiment was performed by using NK cells expressing CAR including a GUCY2C binding scFV (5F9, D08, G07).

Specifically, ELISA method was used to measure the IFN-γ secretion ability, and after co-culturing target cells and each NK cell for 24 hours, the supernatant was recovered. The recovered supernatant was incubated in a plate in which an antibody to recognize IFN-γ was coated, and the remaining solution was removed by washing operation. For this, by recognizing the IFN-γ antibody capable of developing color again, the degree of color development was measured to confirm the secreted IFN-γ.

As a result, as shown in FIG. 9a, it was confirmed that the amount of the secreted IFN-γ was significantly increased, when the anti-GUCY2C-CAR expressing NK cells were co-cultured with the HT29-GUCY2C targeting cell line overexpressing GUCY2C (HT29 GCC cells). As shown in FIG. 9b, there was no increase in the amount of IFN-γ by co-culturing with HT29-GUCY2C cells in the naive NK (Naive NK) cells not including CAR, and a relatively small amount of IFN-γ increase was confirmed by co-culturing with GUCY2C positive cancer cells, T84 cells. On the other hand, it was confirmed that the IFN-γ amount was significantly increased, compared to the Mock HT29 cells not expressing GUCY2C, when the NK cells expressing CAR including a GUCY2C binding scFV (5F9, D08, G07) were co-cultured with GUCY2C positive cancer cells, T84 cells.

### Example 4.9. Confirmation of survival rate when administering anti-GUCY2C-CAR expressing NK cells in animal model

In order to confirm the survival rate when administering anti-GUCY2C-CAR expressing NK cells in an animal model, the following experiment was performed.

Specifically, after transplanting HT29-GUCY2C-Luc cells i.p. ($2.5\times10^6$ cells) into NOG mice (DO), on day 3, groups were separated with mice expressing the same amount of HT29 cells (IVIS total flux), and CAR NK and cytokine were administered together. The GUCY2C-CAR NK cells were intraperitoneally administered in an amount of $1\times10^7$ cells, and the concentration of the cytokine was administered as hIL-2 (Novartis Proleukin)/10 ug (ip, 4 times/week), hIL-15 (Peprotech)/3 ug (ip, qd). Then, the survival rate and survival day of each experimental group were confirmed.

[Table 7]

| Group | No. | death day | | |
|---|---|---|---|---|
| G1 (HT29-Luc, ip, Vehicle + Test sample 2) | 1 | 2021-12-14 | 66 | |
| | 2 | 2021-12-14 | 66 | |
| | 3 | 2021-12-13 | 65 | |
| | 4 | 2021-12-14 | 66 | |
| | 5 | 2021-12-14 | 66 | |
| | 6 | 2021-12-14 | 66 | |
| G2 (HT29-Luc, ip, Test sample 1 + Test sample 2) | 1 | | | 95 |
| | 2 | 2021-12-31 | 83 | |
| | 3 | | | 95 |
| | 4 | 2021-12-27 | 79 | |
| | 5 | 2021-12-14 | 66 | |
| | 6 | 2021-12-30 | 82 | |

As a result, as shown in Table 7 and FIG. 10, it was confirmed that the survival rate was significantly high in the CAR-NK cell administration group compared to the control group, and the survival day of the CAR-NK cell administration group was longer than the control group, as the average survival day was 66 days for the control group and 82.5 days for the CAR-NK cell administration group.

## Claims

**1.** A GUCY2C binding polypeptide, comprising the following:

a heavy chain variable region comprising heavy chain CDR1 (hereinafter, CDR-H1) represented by the amino acid sequence selected from the group consisting of SEQ ID NOs: 38 to 44, CDR-H2 represented by the amino acid sequence selected from the group consisting of SEQ ID NOs: 45 to 53, and CDR-H3 represented by the amino acid sequence selected from the group consisting of SEQ ID NOs: 54 to 65;
a light chain variable region comprising light chain CDR1 (hereinafter, CDR-L1) represented by the amino acid sequence selected from the group consisting of SEQ ID NOs: 66 to 78, CDR-L2 represented by the amino acid sequence selected from the group consisting of SEQ ID NOs: 79 to 88, and CDR-L3 represented by the amino acid sequence selected from the group consisting of SEQ ID NOs: 89 to 106;
or a combination of the heavy chain variable region and light chain variable region.

**2.** The GUCY2C binding polypeptide according to claim 1, wherein the GUCY2C binding polypeptide is scFv (single chain variable fragment).

**3.** The GUCY2C binding polypeptide according to claim 2, represented by the amino acid sequence selected from the group consisting of SEQ ID NOs: 1 to 18.

**4.** A GUCY2C binding antibody comprising the GUCY2C binding polypeptide of claim 1 or antigen binding fragment thereof.

**5.** A fusion protein, comprising the GUCY2C binding polypeptide of claim 1 and a Fc domain of an immunoglobulin.

**6.** The fusion protein according to claim 5, wherein the GUCY2C binding polypeptide is represented by the amino acid sequence selected from the group consisting of SEQ ID NOs: 1 to 18.

**7.** A chimeric antigen receptor, which is a chimeric antigen receptor comprising an extracellular domain, a transmembrane domain and an intracellular signaling domain, wherein the extracellular domain comprises the GUCY2C binding scFv of claim 2.

8. The chimeric antigen receptor according to claim 7, wherein the GUCY2C binding scFv is represented by the amino acid sequence selected from the group consisting of SEQ ID NOs: 1 to 18.

9. An immunocyte expressing the chimeric antigen receptor of claim 7 or claim 8.

10. The immunocyte according to claim 9, wherein the immunocyte is a T cell, a tumor infiltrating lymphocyte, a NK (Natural killer) cell, a TCR-expressing cell, a dendritic cell, or an NK-T cell.

11. A polynucleotide, encoding the polypeptide of any one claim of claim 1 to claim 3, a fusion protein comprising the polypeptide, or a chimeric antigen receptor comprising the polypeptide.

12. The polynucleotide according to claim 11, represented by the nucleic acid sequence selected from the group consisting of SEQ ID NOs: 20 to 37.

13. A composition for detecting GUCY2C, comprising the polypeptide of any one claim of claim 1 to claim 3, a fusion protein comprising the polypeptide, or a chimeric antigen receptor comprising the polypeptide.

14. A composition for diagnosing cancer, comprising the polypeptide of any one claim of claim 1 to claim 3, a fusion protein comprising the polypeptide, or a chimeric antigen receptor comprising the polypeptide.

15. The composition for diagnosing cancer according to claim 14, wherein the cancer expresses GUCY2C.

16. A pharmaceutical composition for prevention or treatment of cancer, comprising

the GUCY2C binding polypeptide of any one claim of claim 1 to claim 3,
a polynucleotide encoding the GUCY2C binding polypeptide,
a recombinant vector comprising the polynucleotide encoding the GUCY2C binding polypeptide,
a chimeric antigen receptor comprising the GUCY2C binding polypeptide,
a polynucleotide encoding the chimeric antigen receptor;
a recombinant vector comprising the polynucleotide encoding the chimeric antigen receptor; and
an immunocyte comprising a polynucleotide encoding the chimeric antigen receptor or expressing the chimeric antigen receptor.

17. The pharmaceutical composition for prevention or treatment of cancer according to claim 16, wherein the cancer expresses GUCY2C.

【FIG. 1a】

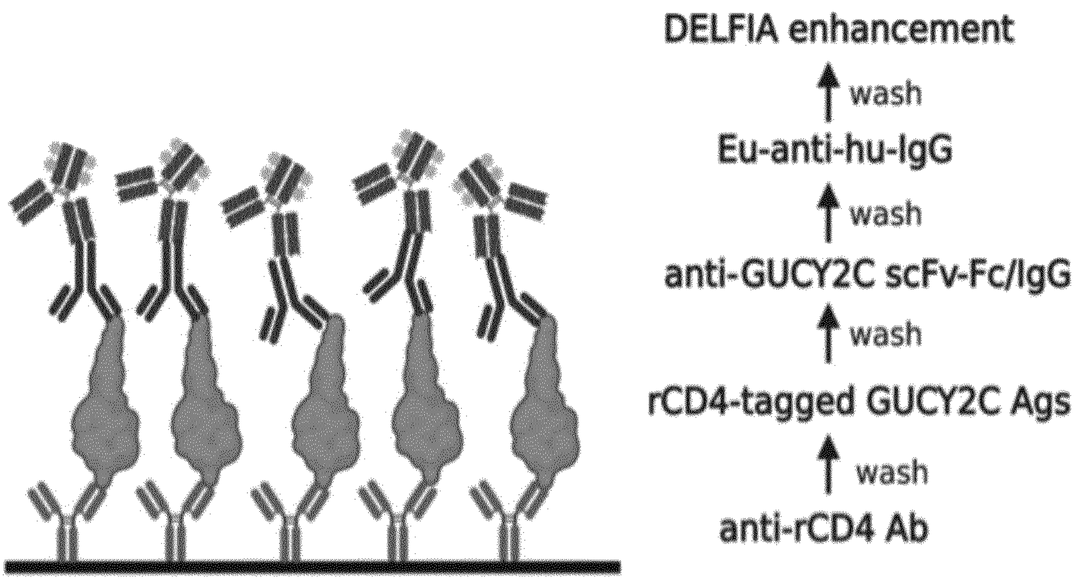
DELFIA enhancement
wash
Eu-anti-hu-IgG
wash
anti-GUCY2C scFv-Fc/IgG
wash
rCD4-tagged GUCY2C Ags
wash
anti-rCD4 Ab

【FIG. 1b】

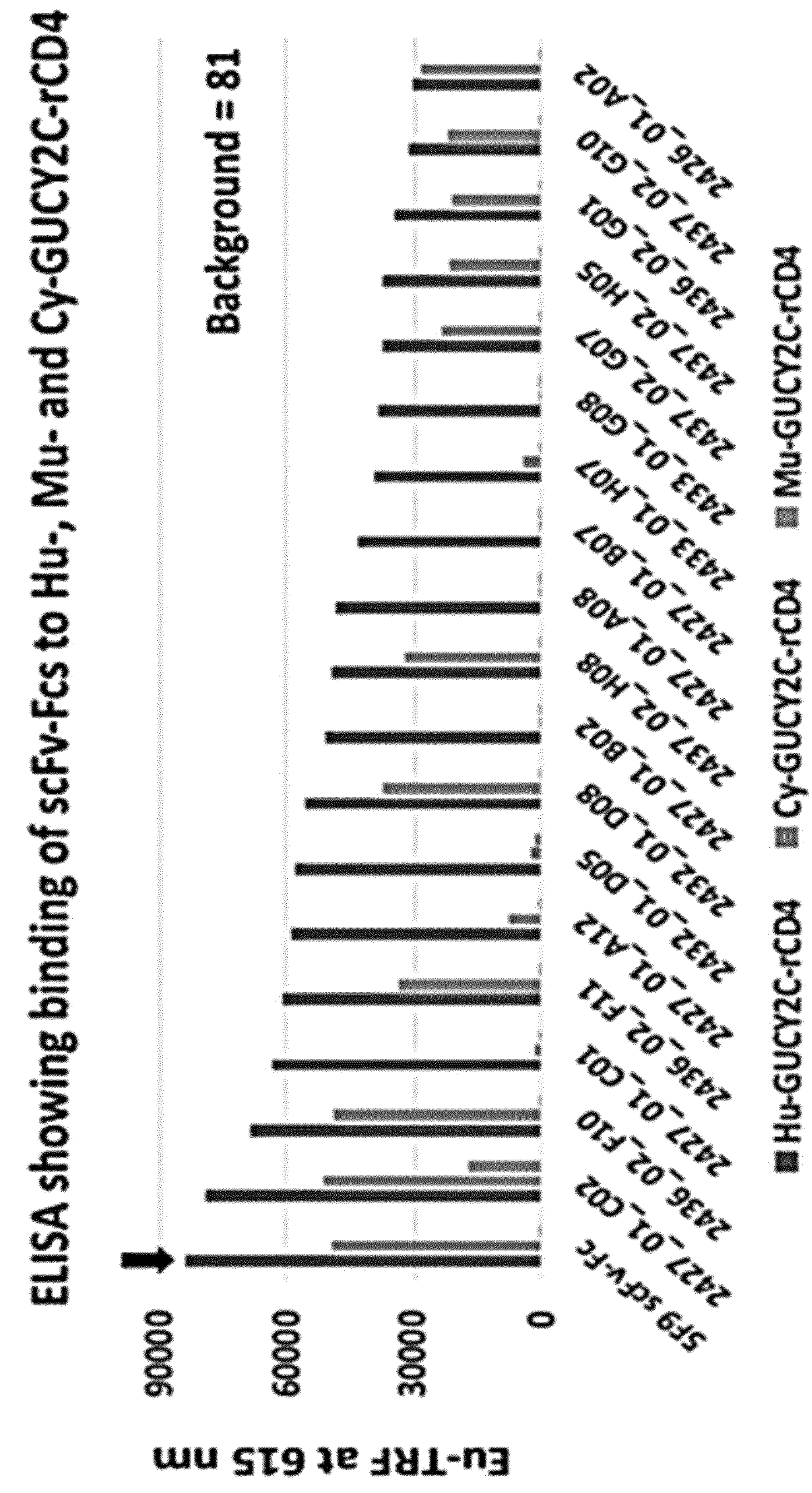
ELISA showing binding of scFv-Fcs to Hu-, Mu- and Cy-GUCY2C-rCD4
Background = 81
Eu-TRF at 615 nm
90000
60000
30000
0
5F9 scFv-Fc
2427_01_C02
2436_02_F10
2427_01_C01
2436_02_F11
2427_01_A12
2432_01_D05
2432_01_D08
2427_01_B02
2437_02_H08
2427_01_A08
2427_01_B07
2433_01_H07
2433_01_G08
2437_02_G07
2437_02_H05
2436_02_G01
2437_02_G10
2426_01_A02
Hu-GUCY2C-rCD4
Cy-GUCY2C-rCD4
Mu-GUCY2C-rCD4

【FIG. 2a】

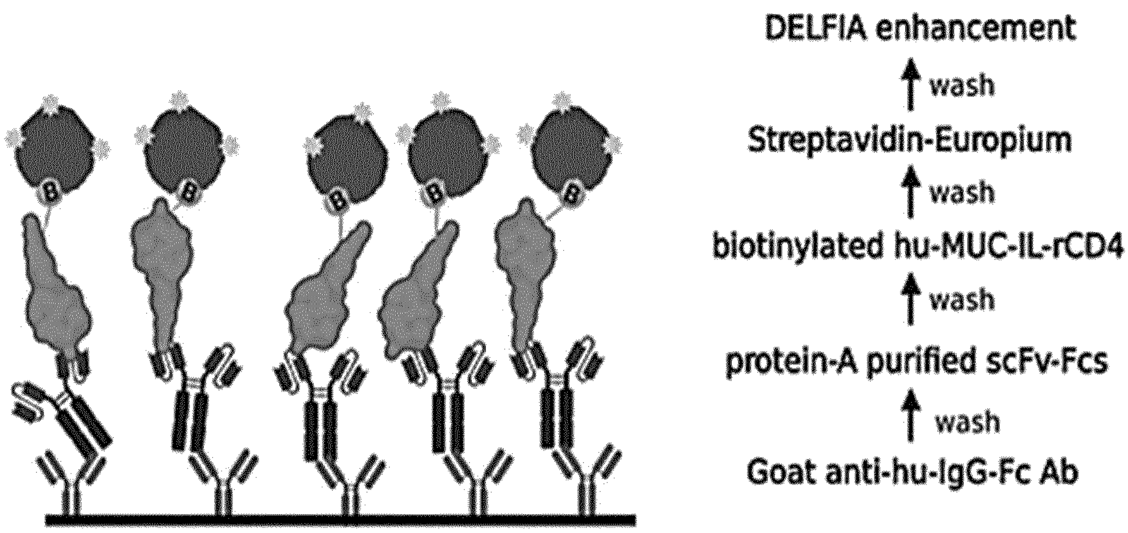
DELFIA enhancement
wash
Streptavidin-Europium
wash
biotinylated hu-MUC-IL-rCD4
wash
protein-A purified scFv-Fcs
wash
Goat anti-hu-IgG-Fc Ab

【FIG. 2b】

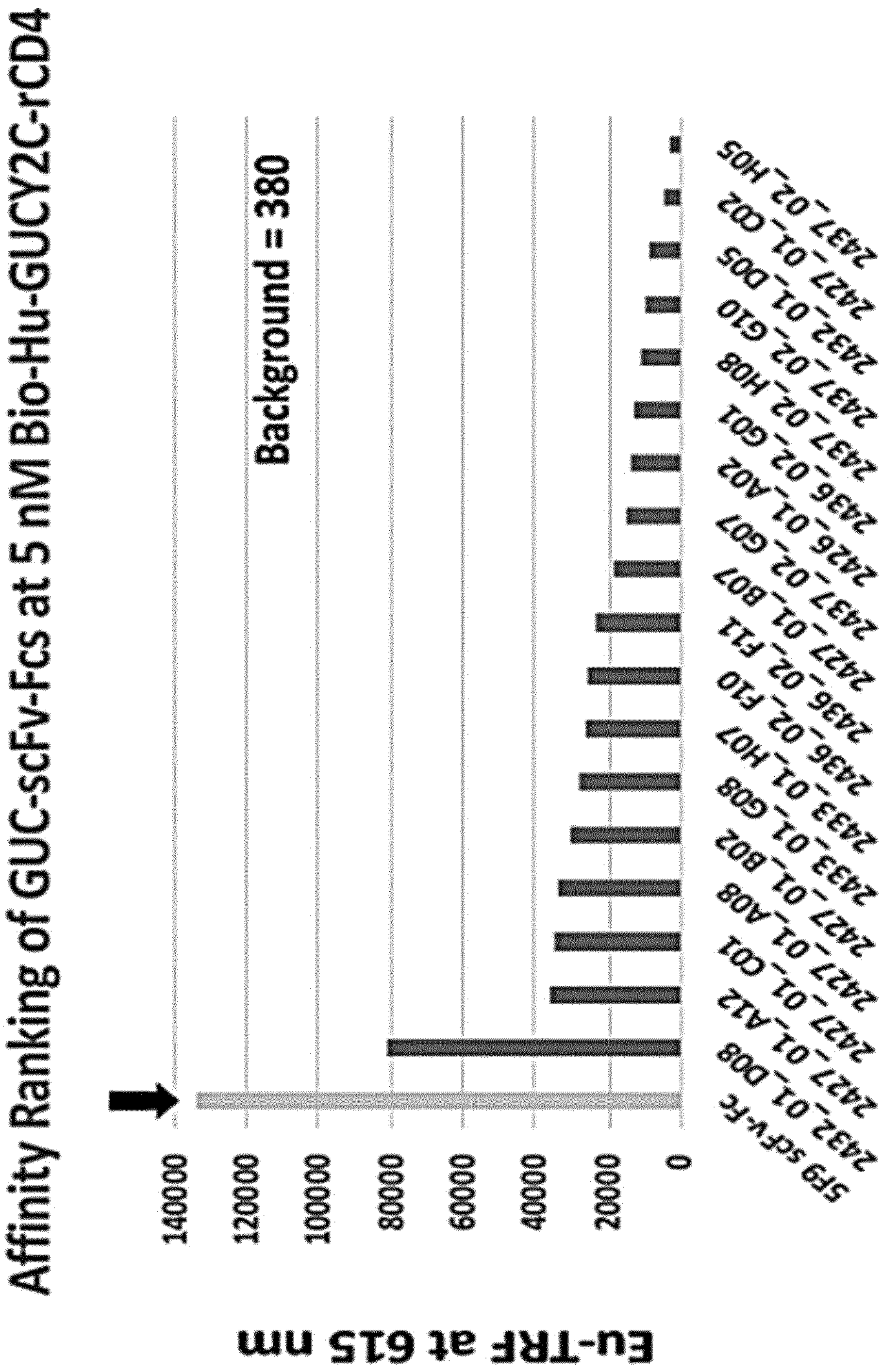
Affinity Ranking of GUC-scFv-Fcs at 5 nM Bio-Hu-GUCY2C-rCD4
Background = 380
Eu-TRF at 615 nm
140000
120000
100000
80000
60000
40000
20000
0
5F9 scFv-Fc
2432_01_D08
2427_01_A12
2427_01_C01
2427_01_A08
2427_01_B02
2433_01_G08
2433_01_H07
2436_02_F10
2436_02_F11
2427_01_B07
2437_02_G07
2426_01_A02
2436_02_G01
2437_02_H08
2437_02_G10
2432_01_D05
2427_01_C02
2437_02_H05

【FIG. 2c】

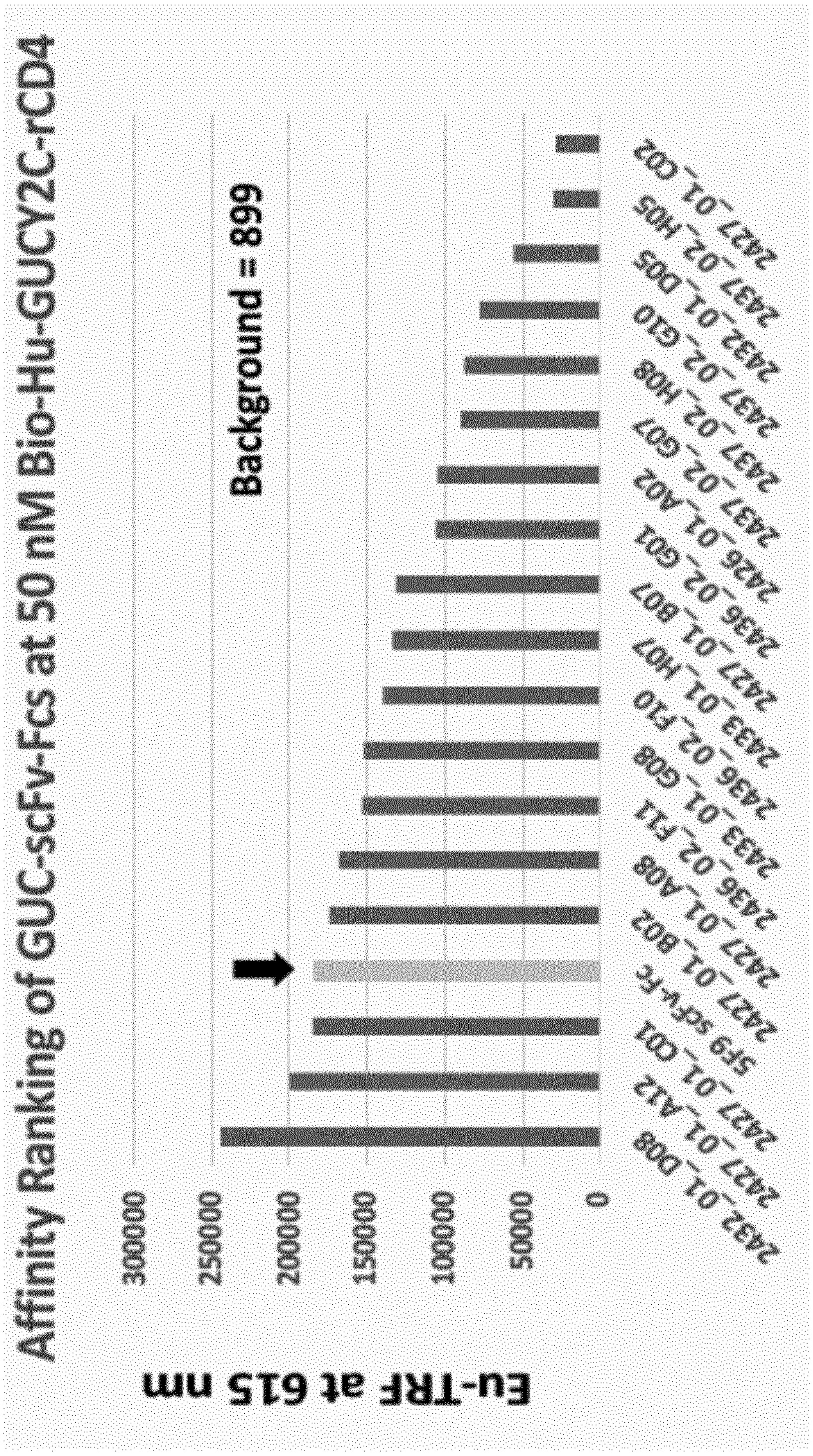
Affinity Ranking of GUC-scFv-Fcs at 50 nM Bio-Hu-GUCY2C-rCD4
Background = 899
Eu-TRF at 615 nm
300000
250000
200000
150000
100000
50000
0
2432_01_D08
2427_01_A12
2427_01_C01
5F9 scFv-Fc
2427_01_B02
2427_01_A08
2436_02_F11
2433_01_G08
2436_02_F10
2433_01_H07
2427_01_B07
2436_02_G01
2426_01_A02
2437_02_G07
2437_02_H08
2437_02_G10
2432_01_D05
2437_02_H05
2427_01_C02

【FIG. 3a】

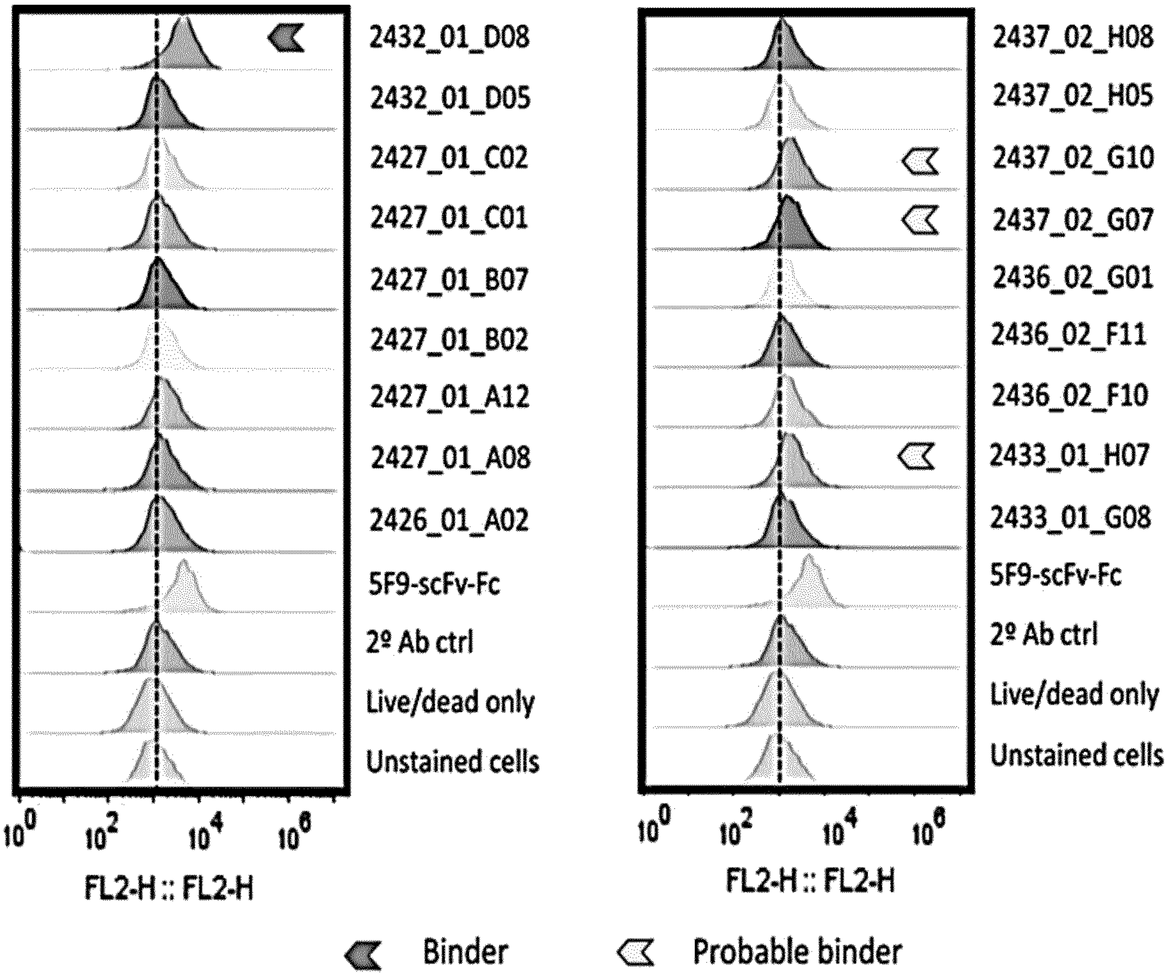
2432_01_D08
2432_01_D05
2427_01_C02
2427_01_C01
2427_01_B07
2427_01_B02
2427_01_A12
2427_01_A08
2426_01_A02
5F9-scFv-Fc
2º Ab ctrl
Live/dead only
Unstained cells
$10^0$
$10^2$
$10^4$
$10^6$
FL2-H :: FL2-H
2437_02_H08
2437_02_H05
2437_02_G10
2437_02_G07
2436_02_G01
2436_02_F11
2436_02_F10
2433_01_H07
2433_01_G08
5F9-scFv-Fc
2º Ab ctrl
Live/dead only
Unstained cells
$10^0$
$10^2$
$10^4$
$10^6$
FL2-H :: FL2-H
Binder
Probable binder

【FIG. 3b】

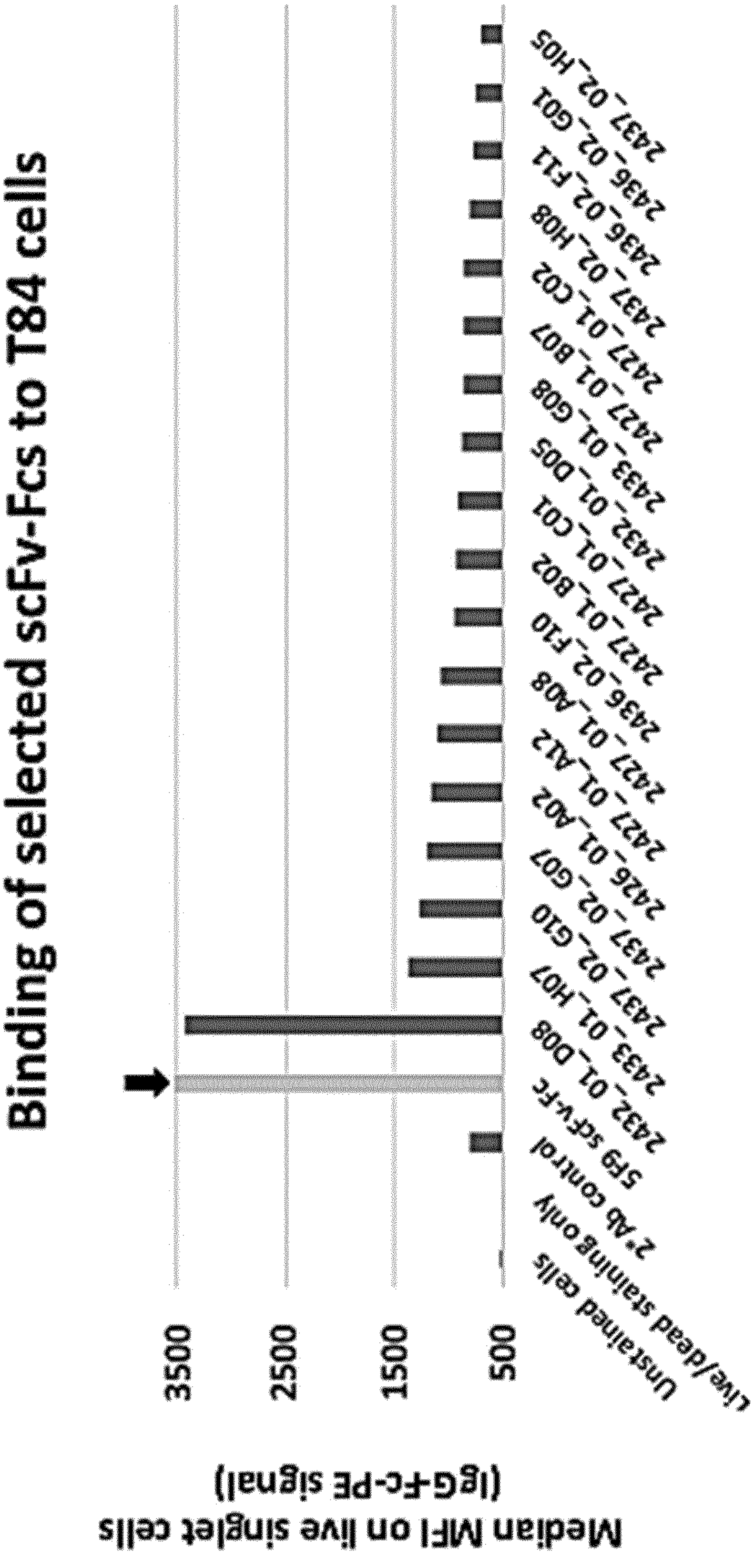

Binding of selected scFv-Fcs to T84 cells
Median MFI on live singlet cells (IgG-Fc-PE signal)
3500
2500
1500
500
Unstained cells
Live/dead staining only
2°Ab control
5F9 scFv-Fc
2432_01_D08
2433_01_H07
2437_02_G10
2437_02_G07
2426_01_A02
2427_01_A12
2427_01_A08
2436_02_F10
2427_01_B02
2427_01_C01
2432_01_D05
2433_01_G08
2427_01_B07
2427_01_C02
2437_02_H08
2436_02_F11
2436_02_G01
2437_02_H05

【FIG. 4a】

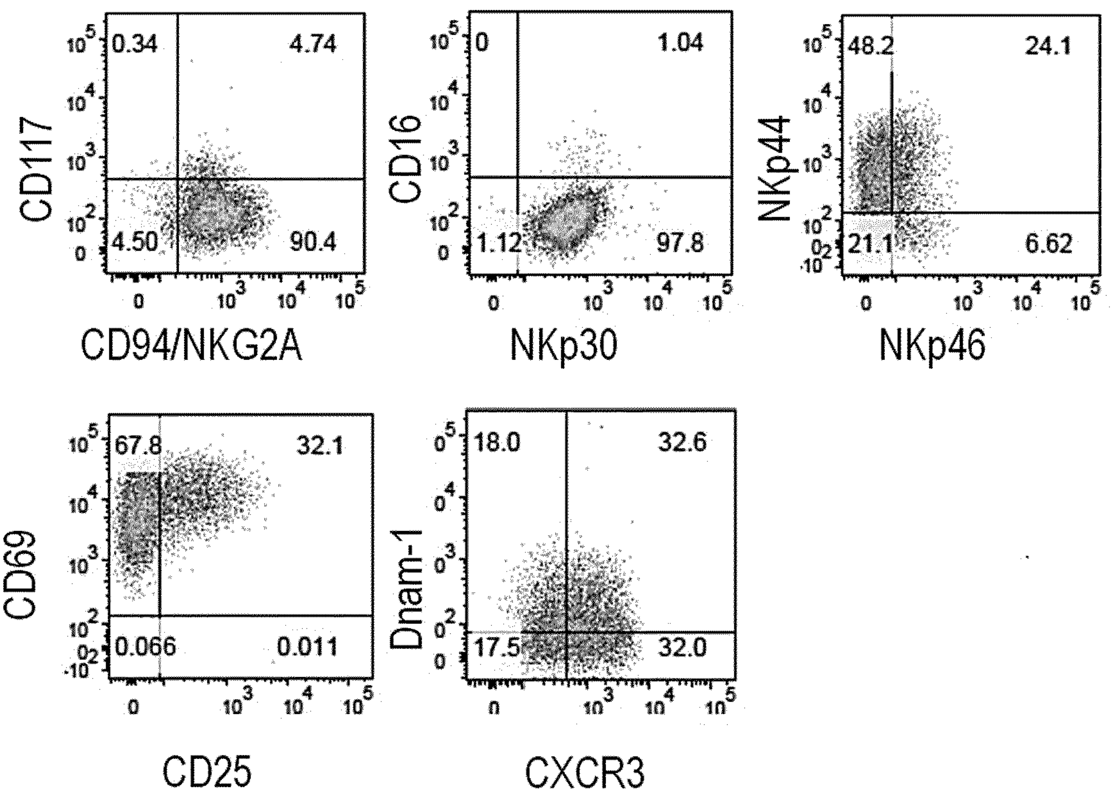

CD117
CD94/NKG2A
0.34
4.74
4.50
90.4
CD16
NKp30
0
1.04
1.12
97.8
NKp44
NKp46
48.2
24.1
21.1
6.62
CD69
CD25
67.8
32.1
0.066
0.011
Dnam-1
CXCR3
18.0
32.6
17.5
32.0

【FIG. 4b】

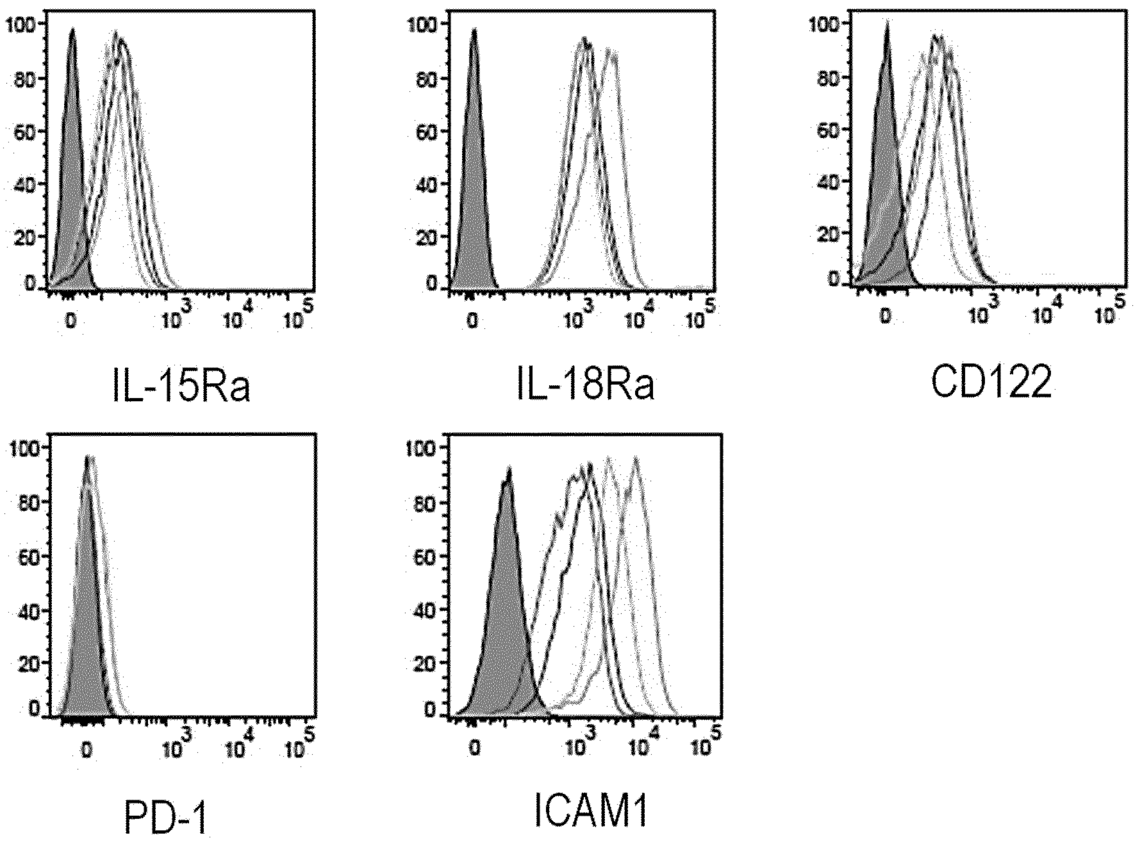
IL-15Ra
IL-18Ra
CD122
PD-1
ICAM1

【FIG. 4c】

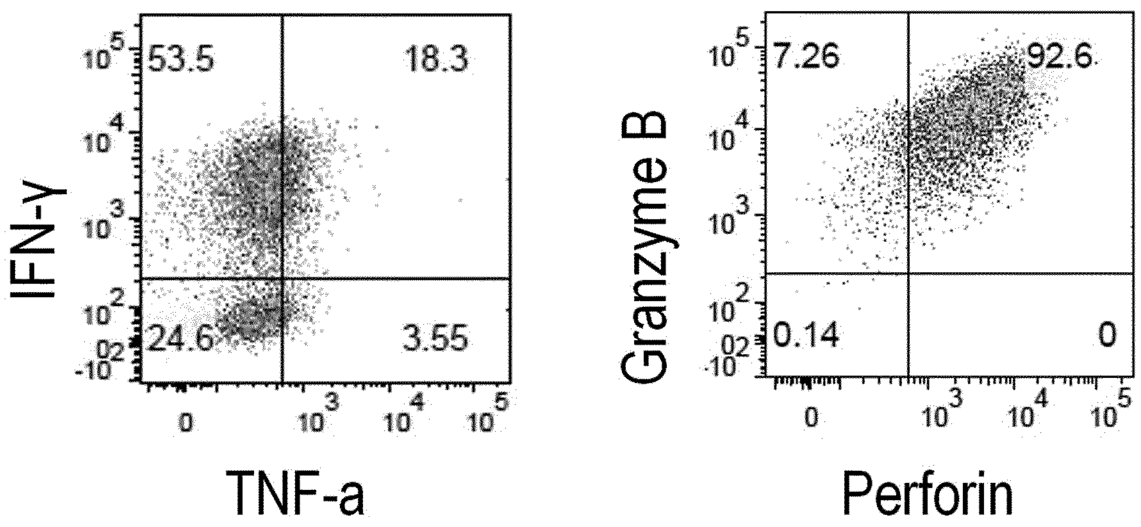
IFN-γ
TNF-a
53.5
18.3
24.6
3.55
Granzyme B
Perforin
7.26
92.6
0.14
0

【FIG. 5】

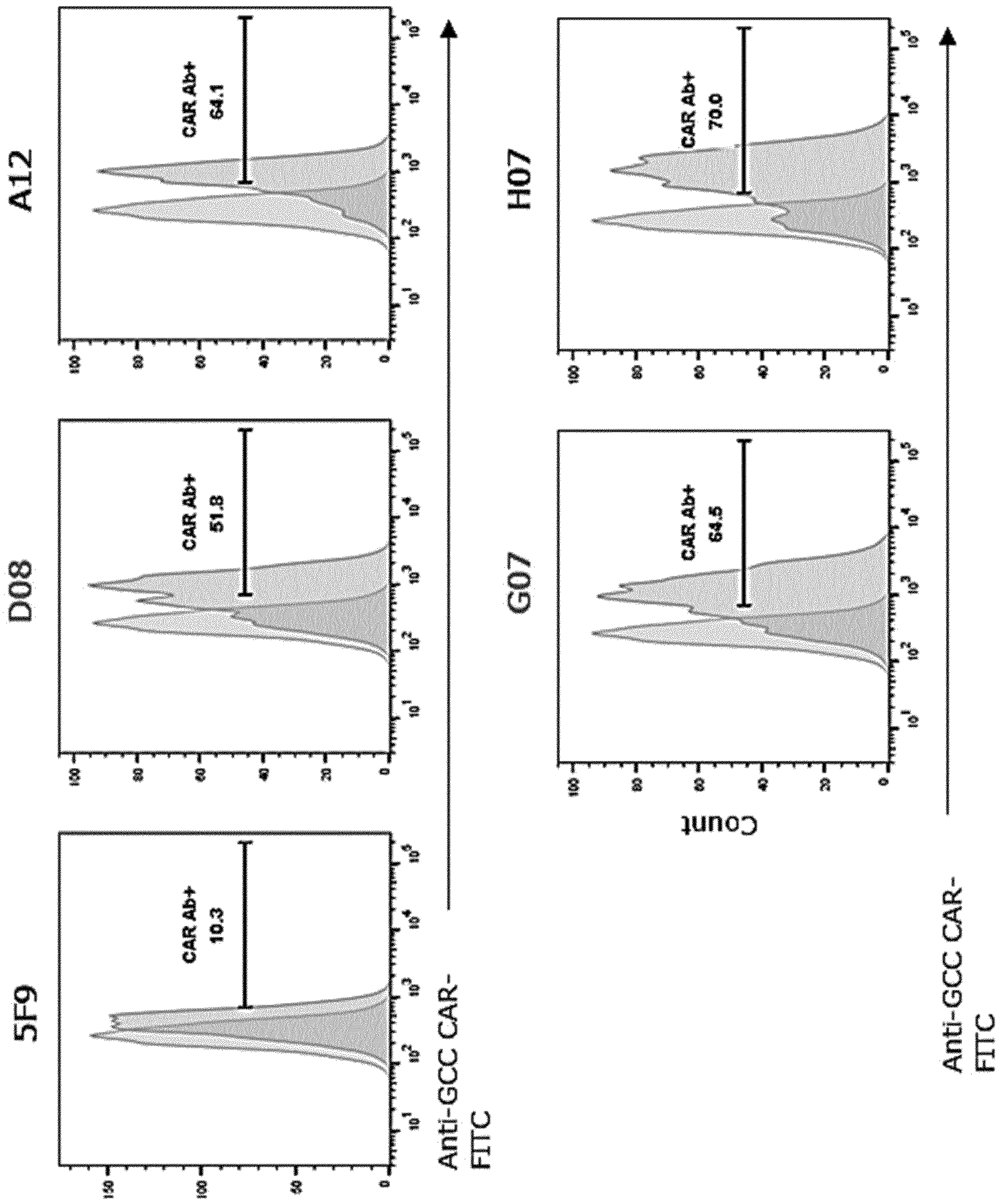
5F9
CAR Ab+
10.3
D08
CAR Ab+
51.8
A12
CAR Ab+
64.1
G07
CAR Ab+
64.5
H07
CAR Ab+
70.0
Count
Count
Anti-GCC CAR-FITC
Anti-GCC CAR-FITC

【FIG. 6a】

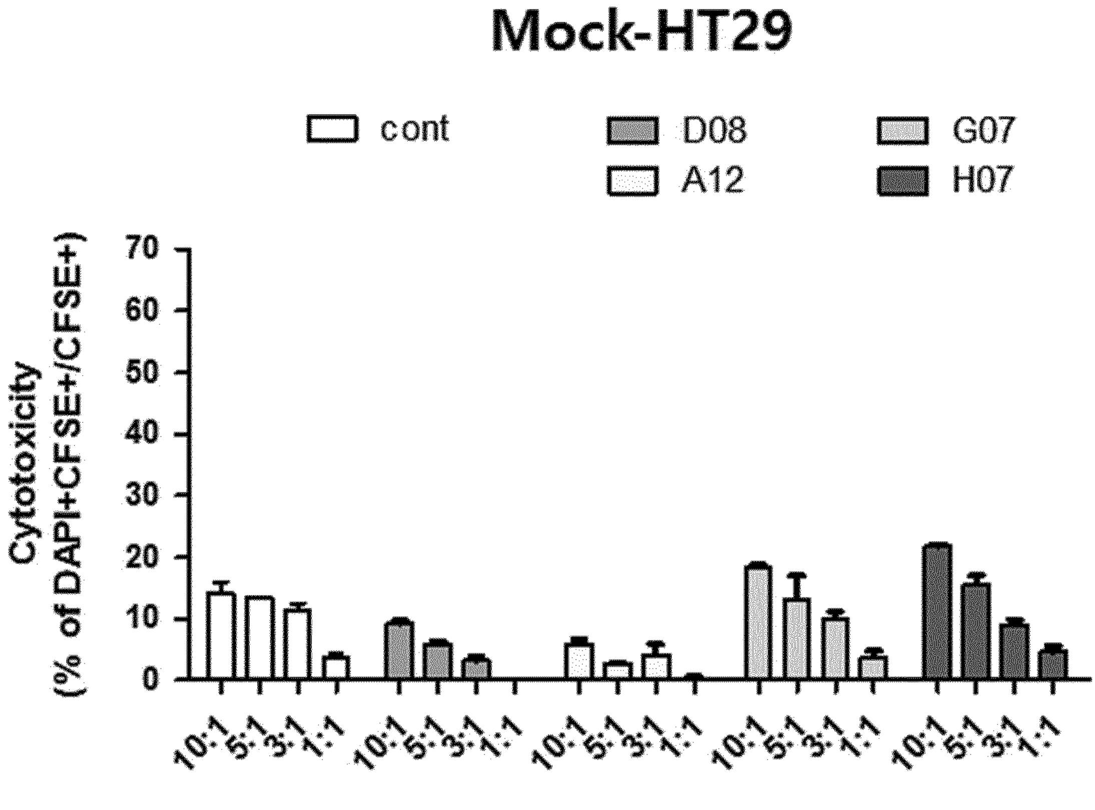
Mock-HT29
cont
D08
A12
G07
H07
Cytotoxicity
(% of DAPI+CFSE+/CFSE+)
70
60
50
40
30
20
10
0
10:1
5:1
3:1
1:1
10:1
5:1
3:1
1:1
10:1
5:1
3:1
1:1
10:1
5:1
3:1
1:1
10:1
5:1
3:1
1:1

【FIG. 6b】

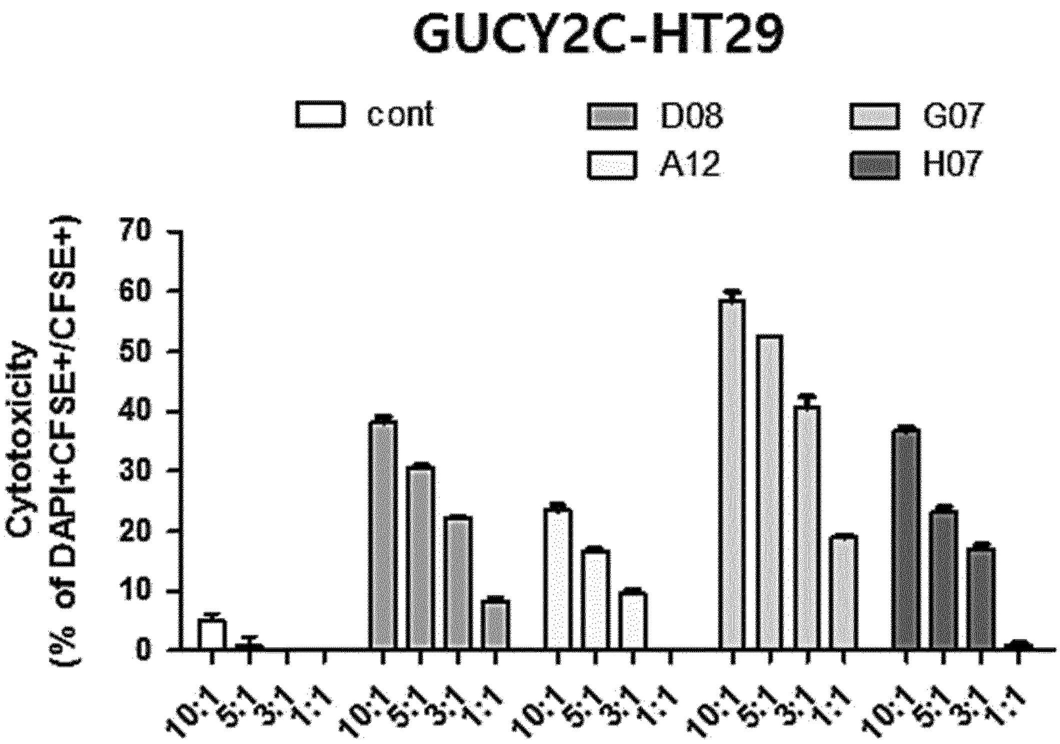
GUCY2C-HT29
cont
D08
A12
G07
H07
Cytotoxicity
(% of DAPI+CFSE+/CFSE+)
70
60
50
40
30
20
10
0
10:1
5:1
3:1
1:1

【FIG. 7】

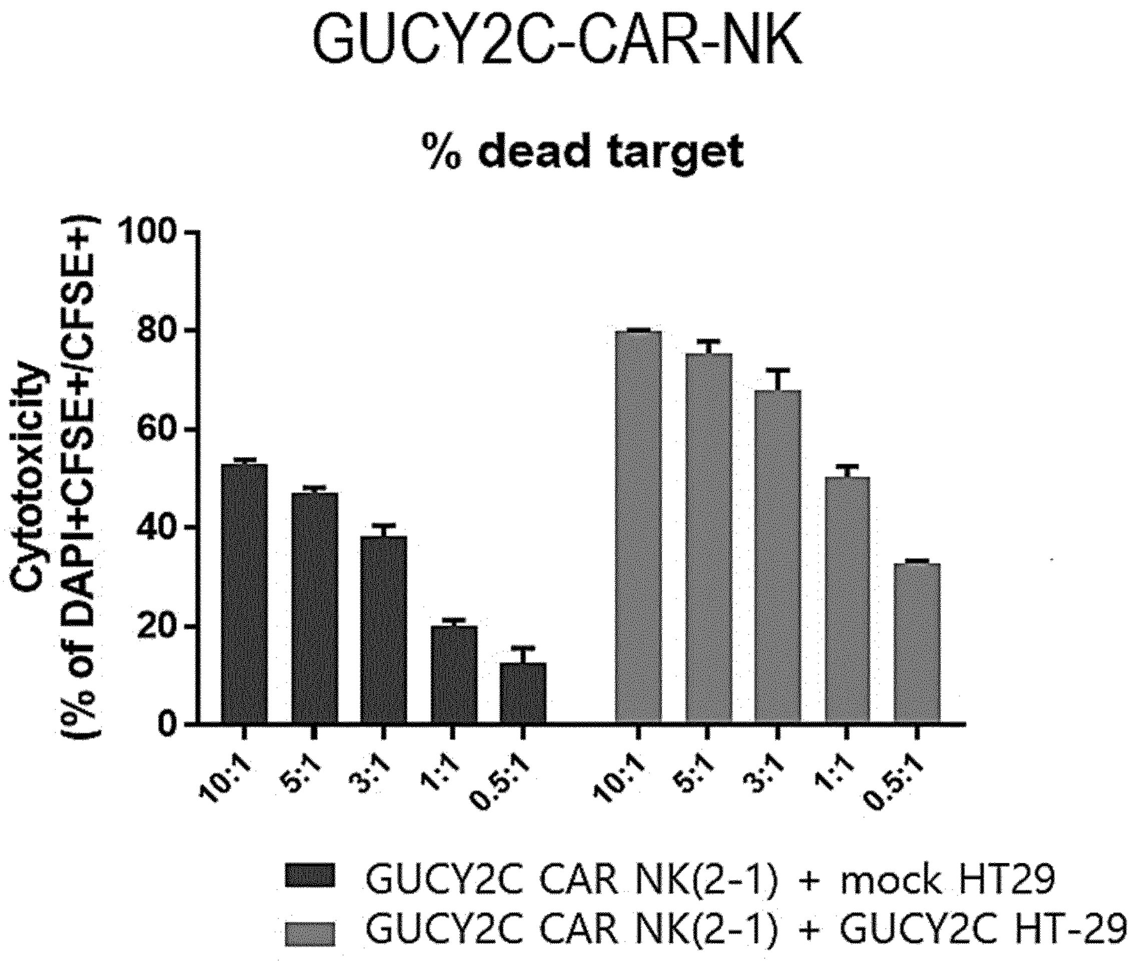
GUCY2C-CAR-NK
% dead target
Cytotoxicity
(% of DAPI+CFSE+/CFSE+)
100
80
60
40
20
0
10:1
5:1
3:1
1:1
0.5:1
10:1
5:1
3:1
1:1
0.5:1
GUCY2C CAR NK(2-1) + mock HT29
GUCY2C CAR NK(2-1) + GUCY2C HT-29

【FIG. 8】

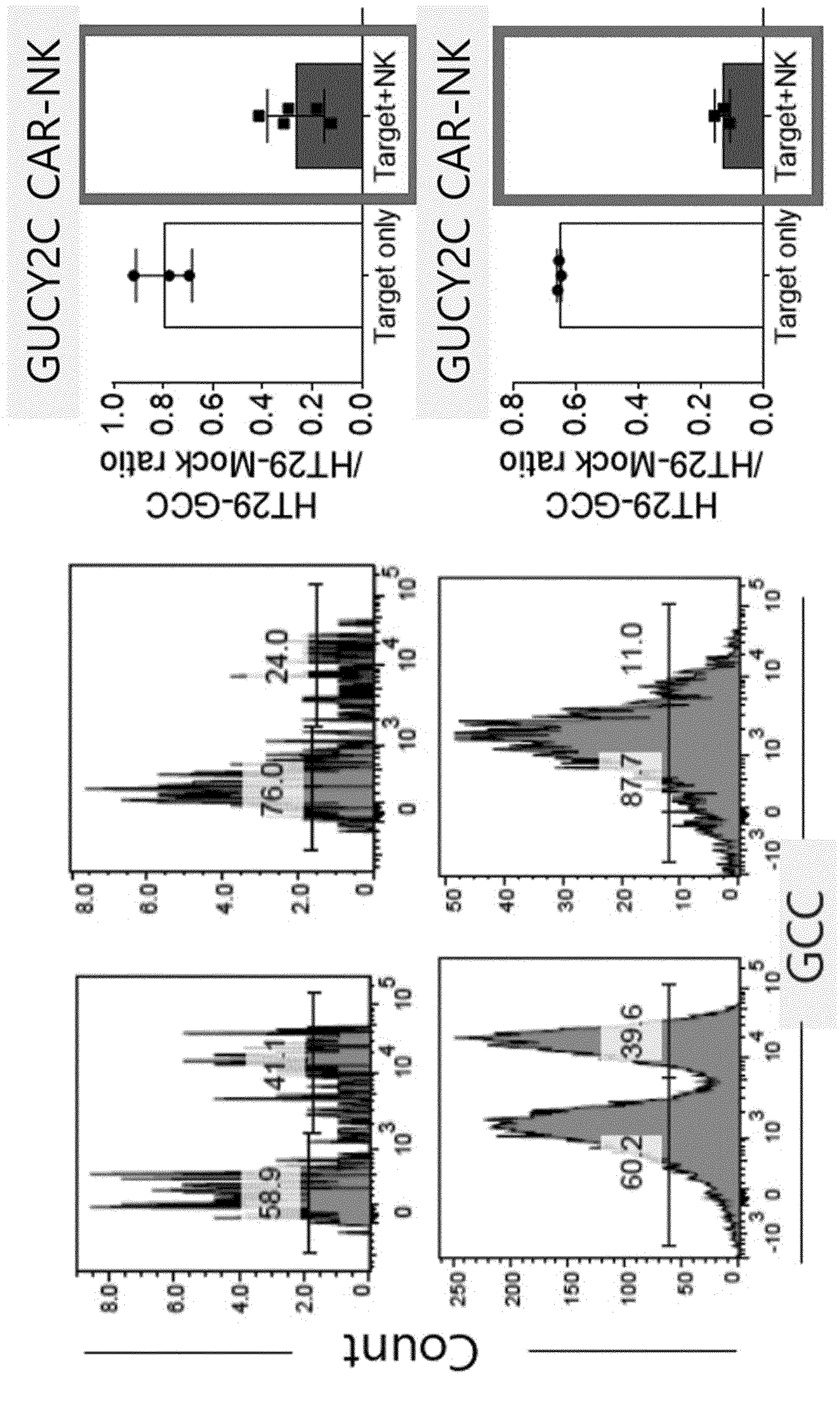
GUCY2C CAR-NK
HT29-GCC /HT29-Mock ratio
Target only
Target+NK
GUCY2C CAR-NK
HT29-GCC /HT29-Mock ratio
Target only
Target+NK
Count
GCC
58.9
41.1
76.0
24.0
60.2
39.6
87.7
11.0

【FIG. 9a】

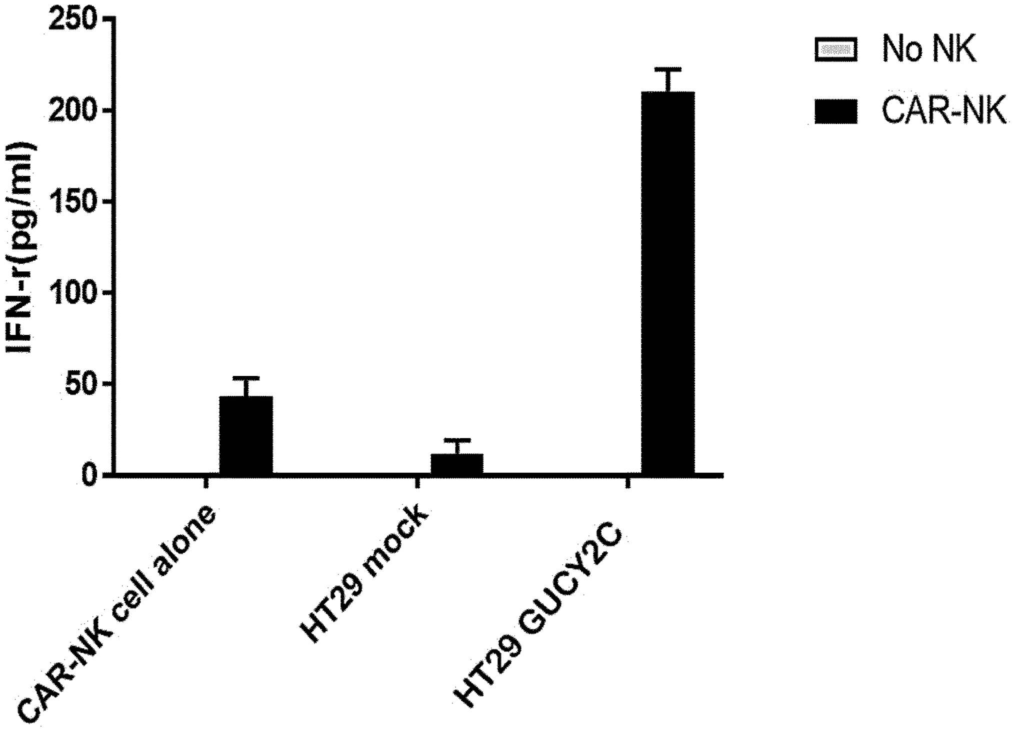
250
200
150
100
50
0
IFN-r(pg/ml)
No NK
CAR-NK
CAR-NK cell alone
HT29 mock
HT29 GUCY2C

【FIG. 9b】

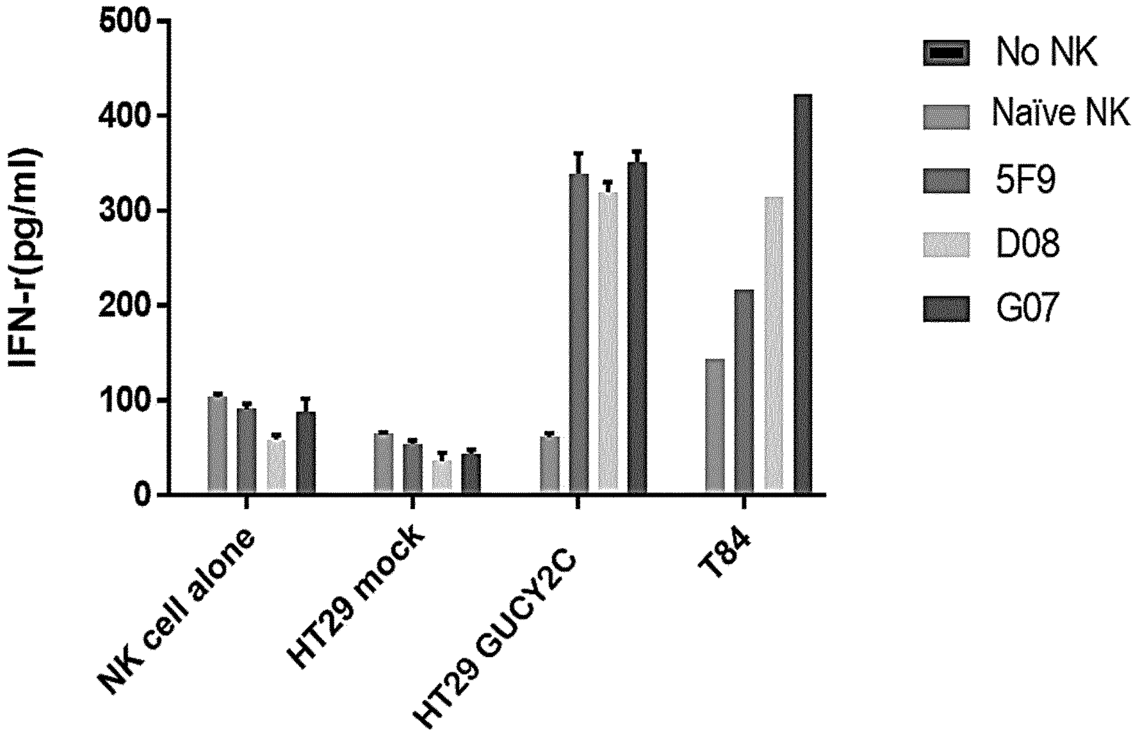
IFN-r(pg/ml)
500
400
300
200
100
0
NK cell alone
HT29 mock
HT29 GUCY2C
T84
No NK
Naïve NK
5F9
D08
G07

【FIG. 10】

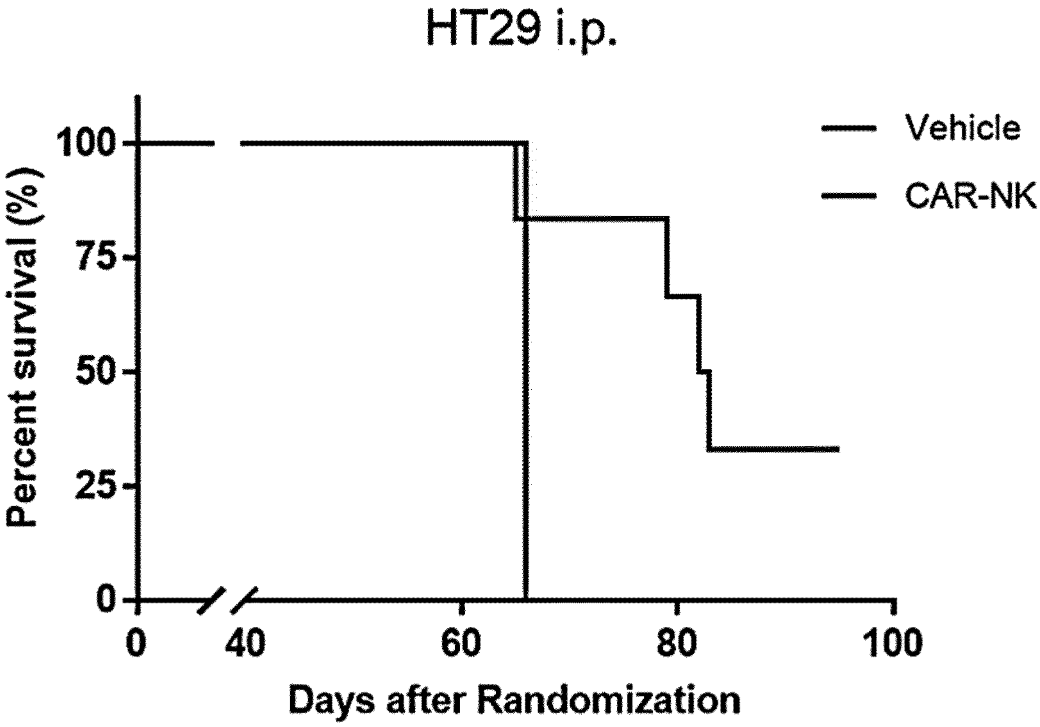
HT29 i.p.
Percent survival (%)
100
75
50
25
0
0
40
60
80
100
Days after Randomization
Vehicle
CAR-NK

**INTERNATIONAL SEARCH REPORT**

International application No.
**PCT/KR2022/005030**

**A. CLASSIFICATION OF SUBJECT MATTER**

**C07K 16/40**(2006.01)i; **C12N 15/85**(2006.01)i; **C12N 15/62**(2006.01)i; **G01N 33/68**(2006.01)i; **G01N 33/574**(2006.01)i; **A61P 35/00**(2006.01)i; **A61K 38/00**(2006.01)i

According to International Patent Classification (IPC) or to both national classification and IPC

**B. FIELDS SEARCHED**

Minimum documentation searched (classification system followed by classification symbols)

C07K 16/40(2006.01); A61K 39/00(2006.01); A61K 39/395(2006.01); A61K 47/68(2017.01); A61P 35/00(2006.01); C07K 14/725(2006.01); C07K 16/28(2006.01); G01N 33/574(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: GUCY2C, CDR, 중쇄(heavy chain), 경쇄(light chain), 키메라 항원 수용체 (chimeric antigen receptor), 면역 세포(immune cell), scFv

**C. DOCUMENTS CONSIDERED TO BE RELEVANT**

| Category* | Citation of document, with indication, where appropriate, of the relevant passages | Relevant to claim No. |
|---|---|---|
| A | KR 10-2021-0013165 A (PFIZER INC.) 03 February 2021 (2021-02-03)<br>See entire document. | 1-17 |
| A | WO 2019-178580 A1 (THOMAS JEFFERSON UNIVERSITY) 19 September 2019 (2019-09-19)<br>See entire document. | 1-17 |
| A | US 2013-0315923 A1 (THOMAS JEFFERSON UNIVERSITY) 28 November 2013 (2013-11-28)<br>See entire document. | 1-17 |
| A | WO 2011-050242 A1 (MILLENNIUM PHARMACEUTICALS, INC. et al.) 28 April 2011 (2011-04-28)<br>See entire document. | 1-17 |
| A | US 2019-0038762 A1 (MILLENNIUM PHARMACEUTICALS, INC. et al.) 07 February 2019 (2019-02-07)<br>See entire document. | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 July 2022** | **26 July 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2022/005030**

**C. DOCUMENTS CONSIDERED TO BE RELEVANT**

| Category* | Citation of document, with indication, where appropriate, of the relevant passages | Relevant to claim No. |
|---|---|---|
| A | US 2020-0270328 A1 (MEMORIAL SLOAN KETTERING CANCER CENTER et al.) 27 August 2020 (2020-08-27)<br>See entire document. | 1-17 |

Form PCT/ISA/210 (second sheet) (July 2019)

**INTERNATIONAL SEARCH REPORT**

International application No.
**PCT/KR2022/005030**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (July 2019)

**INTERNATIONAL SEARCH REPORT**
**Information on patent family members**

International application No.
**PCT/KR2022/005030**

| Patent document cited in search report | Publication date (day/month/year) | Patent family member(s) | Publication date (day/month/year) |
|---|---|---|---|
| KR 10-2021-0013165 A | 03 February 2021 | AU 2019-274655 A1 | 26 November 2020 |
| | | CA 3100829 A1 | 28 November 2019 |
| | | CN 113286634 A | 20 August 2021 |
| | | EP 3796983 A2 | 31 March 2021 |
| | | IL 278924 A | 31 January 2021 |
| | | JP 2021-525080 A | 24 September 2021 |
| | | JP 7057843 B2 | 20 April 2022 |
| | | TW 202003041 A | 16 January 2020 |
| | | US 2020-0010566 A1 | 09 January 2020 |
| | | WO 2019-224716 A2 | 28 November 2019 |
| | | WO 2019-224716 A3 | 05 March 2020 |
| | | WO 2019-224716 A8 | 28 November 2019 |
| WO 2019-178580 A1 | 19 September 2019 | AU 2019-236307 A1 | 22 October 2020 |
| | | CA 3093705 A1 | 19 September 2019 |
| | | CN 112004552 A | 27 November 2020 |
| | | EP 3765078 A1 | 20 January 2021 |
| | | JP 2021-520229 A | 19 August 2021 |
| | | KR 10-2021-0011909 A | 02 February 2021 |
| | | US 2021-0038648 A1 | 11 February 2021 |
| US 2013-0315923 A1 | 28 November 2013 | US 2016-0030595 A1 | 04 February 2016 |
| | | US 9156915 B2 | 13 October 2015 |
| | | US 9662405 B2 | 30 May 2017 |
| WO 2011-050242 A1 | 28 April 2011 | AU 2010-310545 A1 | 17 May 2012 |
| | | AU 2010-310545 A8 | 11 October 2012 |
| | | AU 2010-310545 B2 | 06 November 2014 |
| | | AU 2010-310545 C1 | 30 April 2015 |
| | | AU 2013-202036 A1 | 18 April 2013 |
| | | AU 2013-202036 B2 | 05 February 2015 |
| | | AU 2013-202036 C1 | 03 September 2015 |
| | | AU 2015-202283 A1 | 21 May 2015 |
| | | AU 2015-202283 B2 | 22 June 2017 |
| | | CN 102573908 A | 11 July 2012 |
| | | CN 102573908 B | 17 December 2014 |
| | | CN 104447996 A | 25 March 2015 |
| | | CN 110054692 A | 26 July 2019 |
| | | EP 2490720 A1 | 29 August 2012 |
| | | EP 2490720 B1 | 12 February 2020 |
| | | EP 3520816 A2 | 07 August 2019 |
| | | EP 3520816 A3 | 09 October 2019 |
| | | IL 218763 A | 28 June 2012 |
| | | IL 218763 B | 31 October 2016 |
| | | IL 260285 A | 31 July 2018 |
| | | IL 260285 B | 29 August 2019 |
| | | JP 2013-507968 A | 07 March 2013 |
| | | JP 2016-028603 A | 03 March 2016 |
| | | JP 5925684 B2 | 25 May 2016 |
| | | KR 10-1834890 B1 | 20 April 2018 |
| | | KR 10-2012-0128120 A | 26 November 2012 |
| | | KR 10-2017-0123723 A | 08 November 2017 |
| | | NZ 598791 A | 30 May 2014 |

Form PCT/ISA/210 (patent family annex) (July 2019)

**INTERNATIONAL SEARCH REPORT**
**Information on patent family members**

International application No.
**PCT/KR2022/005030**

| Patent document cited in search report | Publication date (day/month/year) | Patent family member(s) | Publication date (day/month/year) |
|---|---|---|---|
| | | NZ 623607 A | 29 July 2016 |
| | | US 10941211 B2 | 09 March 2021 |
| | | US 2011-0110936 A1 | 12 May 2011 |
| | | US 2014-0322233 A1 | 30 October 2014 |
| | | US 2018-0355062 A1 | 13 December 2018 |
| | | US 8785600 B2 | 22 July 2014 |
| | | ZA 201201944 B | 29 May 2013 |
| US 2019-0038762 A1 | 07 February 2019 | AR 108825 A1 | 03 October 2018 |
| | | AU 2017-214544 A1 | 02 August 2018 |
| | | EP 3411075 A1 | 12 December 2018 |
| | | IL 260832 A | 20 September 2018 |
| | | JP 2019-511462 A | 25 April 2019 |
| | | KR 10-2018-0115687 A | 23 October 2018 |
| | | TW 201813670 A | 16 April 2018 |
| | | WO 2017-136693 A1 | 10 August 2017 |
| US 2020-0270328 A1 | 27 August 2020 | AU 2015-357518 A1 | 29 June 2017 |
| | | AU 2015-357518 B2 | 07 October 2021 |
| | | CN 107428829 A | 01 December 2017 |
| | | CN 107428829 B | 15 October 2021 |
| | | CN 113683711 A | 23 November 2021 |
| | | EP 3227339 A1 | 11 October 2017 |
| | | EP 3227339 A4 | 08 August 2018 |
| | | EP 3227339 B1 | 10 November 2021 |
| | | JP 2017-537629 A | 21 December 2017 |
| | | JP 2021-040652 A | 18 March 2021 |
| | | JP 6919091 B2 | 18 August 2021 |
| | | KR 10-2017-0109537 A | 29 September 2017 |
| | | MX 2017007242 A | 25 January 2018 |
| | | US 10633426 B2 | 28 April 2020 |
| | | US 10906956 B2 | 02 February 2021 |
| | | US 2018-0118803 A1 | 03 May 2018 |
| | | US 2020-0270326 A1 | 27 August 2020 |
| | | US 2020-0270327 A1 | 27 August 2020 |
| | | WO 2016-090312 A1 | 09 June 2016 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210045510


### Non-patent literature cited in the description


- **KARLIN ; ALTSCHUL.** *Pro. Natl. Acad. Sci. USA,* 1993, vol. 90, 5873
- **PEARSON.** *Methods Enzymol.,* 1990, vol. 183, 63